# EUROPEAN PATENT SPECIFICATION

(11) **EP 1 945 217 B1**
(45) Date of publication and mention of the grant of the patent: **02.05.2012**
(21) Application number: 06791989.4
(22) Date of filing: 12.09.2006
(51) Int. Cl.: A61K 31/505, A61K 31/506, A61K 31/519, A61K 31/538, A61K 31/501, A61K 31/44, A61K 9/00, A61K 9/70, A61K 9/48, A61K 47/10, A61K 47/20, A61P 17/00, A61P 17/06, A61P 17/10

(54) **COMBINATIONS COMPRISING A VEGF RECEPTOR INHIBITOR AND A PENETRATION ENHANCER**
KOMBINATIONEN AUS EINEM VEGF-REZEPTOR-HEMMER UND EINEM PENETRATIONSVERSTÄRKER
COMBINAISONS COMPRENANT UN INHIBITEUR DES RÉCEPTEURS VEGF ET UN AGENT AMÉLIORANT LA PÉNÉTRATION

(30) Priority: 13.09.2005 GB 0518671; 13.09.2005 GB 0518672
(43) Date of publication of application: 23.07.2008
(73) Proprietor: Novartis AG, 4056 Basel (CH)
(72) Inventor: BILLICH, Andreas, A-1235 Vienna (AT); STÜTZ, Anton, A-1235 Wien (AT)
(74) Representative: Lettenbichler-Ager, Isolde Maria
(86) International application number: PCT/EP2006/008857
(87) International publication number: WO 2007/031265

(56) References cited:
- EP-A- 1 522 540
- EP-A- 1 568 368
- EP-A1- 1 415 987
- WO-A-2005/021553
- WO-A-2005/069906
- WO-A2-2005/070891
- WO-A2-2006/059234
- US-B1- 6 355 657
- T.C.K. CHAN: "Percutaneous penetration enhancers: an update" PROCEEDINGS OF THE 9TH BIENNAL INTERNATIONAL CONFERENCE OF PERSPECTIVES IN PERCUTANEOUS PENETRATION, January 2005 (2005-01), pages 18-22, XP002416625

## Description

The present invention relates to combinations comprising a VEGF receptor inhibitor of formula I, together with a penetration enhancer selected from oleyl alcohol for topical administration.

WO-A-2005/070891 discloses that compounds which fit in present formula (I) can have kinase inhibitory activity, such as VEGFR inhibitory activity. They can be used for the treatment of psoriasis; in that case they are preferably applied topically in a formulation which may desirably include a component which enhances the absorption or penetration of active ingredients through the skin, such as DMSO.

WO-A-2005/021553 describes compounds which fit in present formula (I) and their VEGFR kinase inhibitory activity. They can be used for the treatment of, inter alia, skin diseases, such as psoriasis, eczema or scleroderma. They can be administered topically, e.g. as an ointment or cream. For transmucosal administration, penetrants appropriate to the barrier to be permeated are used in the formulation.

In one aspect the present invention provides a composition comprising a VEGF receptor inhibitor and a penetration enhancer, for topical administration, wherein
the VEGF receptor inhibitor is a compound of formula 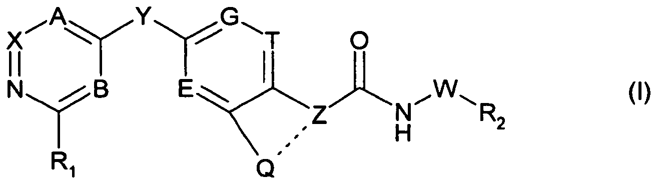 wherein
R₁ is H; halo; -(C₀₋₇)-R₃; -(C₀₋₇)-NR₄R₅; or -C(=O)-R₆;
R₂ is substituted (C₃₋₈)cycloalkyl; substituted aryl; or substituted heterocyclyl;
R₃ is H or unsubstituted or substituted (C₁₋₄)alkyl;
R₄ and R₅ are independently selected from the group consisting of H; unsubstituted or substituted (C₁₋₄)alkyl; (C₁₋₄)alkyl-carbonyl, wherein the (C₁₋₄)alkyl moiety is optionally substituted; and (C₁₋₄)alkoxy-carbonyl, wherein the (C₁₋₄)alkyl moiety is optionally substituted;
R₆ is H; unsubstituted or substituted (C₁₋₄)alkyl; (C₁₋₄)alkoxy, wherein the (C₁₋₄)alkyl moiety is optionally substituted; or unsubstituted, mono- or di-substituted amino;
A, B and X are independently selected from =C(R₇)- or N;
E, G and T are independently selected from =C(R₈)- or N;.
R₇ and R₈ are independently selected from the group consisting of H, halo and unsubstituted or substituted (C₁₋₄)alkyl;
Y is -O-, -S-, -S(O)-, -S(O)₂-, -CH₂- or -CH₂-CH₂- ;
Z is CH or N and Q is (C₁₋₄)alkylene or (C₂₋₄)alkenylene, wherein (C₁₋₄)alkylene or (C₂₋₄)alkenylene optionally may be substituted and wherein one or more of the carbon atoms of said (C₁₋₄)alkylene or (C₂₋₄)alkenylene chain optionally may be replaced by a heteroatom independently selected from nitrogen, oxygen and sulfur; and the bond between Q and Z characterized by a dotted line is a single bond; with the proviso that if Z is N, Q is not unsubstituted unbranched (C₁₋₄)alkylene; or
Z is C and Q is as defined above wherein the bond between Q and Z characterized by a dotted line is a double bond; and
W is either not present or (C₁₋₃)alkylene;
or a tautomer thereof, or in the form of a salt and wherein the penetration enhancer is selected from oleyl alcohol.
If not defined otherwise
   - Alkyl includes (C₁₋₇)alkyl, which may be branched or straight-chained, such as e.g. n-pentyl, n-hexyl or n-heptyl or preferably (C₁₋₄)alkyl, such as methyl, ethyl, n-propyl, sec-propyl, n-butyl, isobutyl, sec-butyl, tert-butyl.
   - The term "(C₀₋₇)" is as defined above for alkyl with the difference that in case of "C₀-" no carbon atom is present.
   - Substituted (C₁₋₇)alkyl or a substituted (C₁₋₇)alkyl moiety is a (C₁₋₇)alkyl radical/moiety substituted by one or more, preferably one, substituent, selected independently from e.g. amino, N-(C₁₋₇)alkylamino, N,N-di-(C₁₋₇)alkylamino, N-(C₁₋₇)alkanoylamino, N,N-di-(C₁₋₇)alkanoylamino, hydroxy, (C₁₋₇)alkoxy, (C₁₋₇)alkanoyl, (C₁₋₇)alkanoyloxy, cyano, nitro, carboxy, (C₁₋₇)alkoxycarbonyl, carbamoyl, N-(C₁₋₇)alkyl-carbamoyl, N,N-di-(C₁₋₇)alkyl-carbamoyl, amidino, guanidino, ureido, mercapto, (C₁₋₇)alkylthio, halo, or unsubstituted or substituted heterocyclyl.
   - (C₁₋₄)alkylene and (C₂₋₄)alkenylene may be branched or unbranched and are in particular (C₂₋₃)alkylene and (C₂₋₃)alkenylene, respectively. In optionally substituted (C₁₋₄)alkylene or (C₂₋₄)alkenylene, the substituents are e.g. selected from amino, N-(C₁₋₇)alkylamino, N,N-di-(C₁₋₇)alkylamino, N-(C₁₋₇)alkanoylamino, N,N-di-(C₁₋₇)alkanoylamino, hydroxy, (C₁₋₇)alkoxy, (C₁₋₇)alkanoyl, (C₁₋₇)alkanoyloxy, cyano, nitro, carboxy, (C₁₋₇)alkoxycarbonyl, carbamoyl, N-(C₁₋₇)alkylcarbamoyl, N,N-di-(C₁₋₇)alkyl-carbamoyl, amidino, guanidino, ureido, mercapto, (C₁₋₇)alkylthio and halo, or (C₁₋₇)alkyl substituted by one or more, preferably one, of said substituents.
   - Mono- or di-substituted amino is amino substituted by one or two radicals selected independently of one another from e.g. substituted and especially unsubstituted lower alkyl.
   - Substituted (C₃₋₈)cycloalkyl is especially cyclohexyl and is preferably substituted as described for substituted aryl.
   - Substituted aryl is preferably an aromatic radical with 4 to 8 carbon atoms, especially phenyl, wherein said radical is substituted by one or more, preferably by one or two, radicals such as e.g. unsubstituted or substituted (C₁₋₇)alkyl, amino, N-(C₁₋₇)alkylamino, N,N-di-(C₁₋₇)alkylamino, N-(C₁₋₇)alkanoylamino, N,N-di-(C₁₋₇)alkanoylamino, hydroxy, (C₁₋₇)alkoxy, (C₁₋₄)alkanoyl, (C₁₋₇)alkanoyloxy, cyano, nitro, carboxy, (C₁₋₇)alkoxycarbonyl, carbamoyl, N-(C₁₋₇)alkyl-carbamoyl, N,N-di-(C₁₋₇)alkyl-carbamoyl, amidino, guanidino, ureido, mercapto, (C₁₋₇)alkylthio, halo, or unsubstituted or substituted heterocyclyl.
   - Unsubstituted or substituted heterocyclyl is preferably a saturated, partially saturated or unsaturated mono- or bicyclic radical having from 4 to 8 ring members and from 1 to 3 heteroatoms which are preferably selected from N, O, S, said radical being unsubstituted or substituted preferably as described for substituted aryl.
   - Halo(geno) is preferably iodo, bromo, chloro or fluoro, especially fluoro, chloro or bromo.
R₁ is preferably amino; halo, such as especially chloro; alkyl-amino, such as especially methylamino; mono- or di-(C₁₋₇)alkyl-amino-lower alkyl, such as especially methylamino-methyl or dimethylamino-methyl; (C₁₋₇)alkoxy-carbonyl, such as especially methoxy-carbonyl; mono- or di-(C₁₋₇)alkyl-amino-carbonyl, such as especially methylamino-carbonyl or dimethylamino-carbonyl; (C₁₋₇)alkyl-carbonyl-amino such as especially methylcarbonyl-amino; or (C₁₋₇)alkoxy-carbonylamino, such as especially methoxycarbonyl-amino.
R₂ is preferably a cyclohexyl, phenyl or pyridyl, especially a phenyl, radical wherein said radical is substituted by one or more, especially 1 or 2, substituents independently selected from the group consisting of (C₁₋₇)alkyl; halo; halo(C₁₋₇)alkyl, such as especially trifluoromethyl; morpholinyl, such as especially morpholin-4-yl; morpholinyl-lower alkyl, such as especially morpholin-4-ylmethyl; and (C₁₋₇)alkyl-piperazinyl-(C₁₋₇)alkyl, such as especially 4-methylpiperazin-1-ylmethyl.

Preferably X is =C(R₇)- and one of A and B is N while the other is =C(R₇)-; most preferably X and A are both =CH- and B is N.

Preferably E, G and T are =C(R₇)-; most preferably E, G and T are all =CH-.

R₇ and R₈ are preferably H or halo.

Y is preferably -O-.

Z is preferably N or C, most preferably C.

Q is preferably (C₂₋₄)alkenylene, or (C₁₋₄)alkylene wherein one or more, especially one, of the carbon atoms of (C₁₋₄)alkylene is replaced by a heteroatom independently selected from N, O, S, especially from O. Q is especially selected from -O-CH₂-[Z], -O-CH₂-CH₂-[Z], -CH=CH- and - CH=CH-CH=, wherein "-[Z]" indicates where the bivalent radical is bound to Z in formula I if there are two possibilities; most preferably Q is -CH=CH-CH=.

W is preferably not present.

In another aspect the VEGF receptor inhibitor is a compound of formula I, wherein R₁ is halo; -(C₀₋₇)-NR₄R₅; or -C(=O)-R₆;

R₂ is a cyclohexyl, phenyl, pyridyl, 2H-indazolyl, 1,3-dihydro-2-benzofuranyl or pyrazole radical wherein said radical is substituted by one or more substituents independently selected from the group consisting of lower alkyl; (C₃₋₈)cycloalkyl; (C₁₋₄)alkoxy; halo; halo-(C₁₋₄)alkyl; halo-(C₁₋₄)alkoxy; SF₅; morpholinyl; morpholinyl-(C₁₋₄)alkyl; piperazinyl-(C₁₋₄)alkyl; (C₁₋₄)alkyl-piperazinyl-(C₁₋₄)alky/ and phenyl, wherein said phenyl is optionally substituted by (C₁₋₄)alkyl,
halo, di-(C₁₋₄)alkyl-amino-(C₁₋₄)alkyl, (C₁₋₄)alkyl-piperazinyl-(C₁₋₄)alkyl or morpholinyl-(C₁₋₄)alkyl; R₄ and R₅ are independently selected from the group consisting of H; (C₁₋₄)alkyl; (C₁₋₄)alkylcarbonyl; and (C₁₋₄)alkoxy-carbonyl;
R₆ is (C₁₋₄)alkoxy, (C₁₋₄)alkyl-amino or di-(C₁₋₄)alkyl-amino;
X is =C(R₇)- and one of A and B is N while the other is =C(R₇)-;
E, G and T are =C(R₈)-;
R₇ and R₈ are independently selected from the group consisting of H and halo;
Y is -0-;
Z is N and Q is (C₂₋₃)alkylene or (C₂₋₃)alkenylene, wherein one of the carbon atoms of said (C₁₋₄)alkylene chain optionally may be replaced by oxygen; and the bond between Q and Z characterized by a dotted line in formula I is a single bond; with the proviso that Q is not unsubstituted unbranched (C₁₋₄)alkylene; or
Z is C and Q is -CH=CH-CH=; and
W is not present.
In another aspect of the present invention the VEGF receptor inhibitor is a compound of formula I, wherein
Z is C and Q is -CH=CH-CH=.

In another aspect of the present invention the VEGF receptor inhibitor is a compound of formula (I), wherein
R₁ is halo; -(C₀₋₇)-NR₄R₅; or -C(=O)-R₆;
R₂ is substituted (C₃₋₈)cycloalkyl; substituted aryl; or substituted heterocyclyl;
R₄ and R₅ are independently selected from the group consisting of H; (C₁₋₄)alkyl; (C₁₋₄)alkyl - carbonyl; and (C₁₋₄)alkoxy-carbonyl;
R₆ is (C₁₋₄)alkyl, (C₁₋₄)alkoxy, (C₁₋₄)alkyl-amino, di-(C₁₋₄)alkyl-amino-(C₁₋₄)alkyl-amino or di-(C₁₋₄)alkyl-amino;
A, B and X are independently selected from =C(R₇)- or N;
E, G and T are =C(R₈)-;
R₇ and R₈ are independently selected from H and halo;
Y is -O-, -S- or -CH₂-, especially -O-;
Z is N and Q is (C₁₋₄)alkylene or (C₂₋₄)alkenylene, wherein one or more, especially one, of the carbon atoms of said (C₁₋₄)alkylene or (C₂₋₄)alkenylene chain optionally may be replaced by a heteroatom independently selected from N, O and S, especially from O, the N optionally substituted by (C₁₋₄)alkyl; and the bond between Q and Z characterized by a dotted line in formula I is a single bond; with the proviso that Q is not unsubstituted unbranched (C₁₋₄)alkylene; or Z is C and Q is as defined above wherein the bond between Q and Z characterized by a dotted line in formula I is a double bond; and
W is (C₁₋₃)alkylene or especially not present.

In another aspect of the present invention the VEGF inhibitor is a compound of formula (I), wherein
Z is N and Q is (C₂₋₄)alkenylene, or (C₁₋₄)alkylene wherein one or more, especially one, of the carbon atoms of (C₁₋₄)alkylene is replaced by a heteroatom independently selected from N, O and S, especially from O; and the bond between Q and Z characterized by a dotted line in
formula I is a single bond; or
Z is C and Q is as defined above wherein the bond between Q and Z characterized by a dotted line in formula I is a double bond.

In a further aspect of the present invention the VEGF receptor inhibitor is a compound of formula (I), wherein
A is N and B is =CH or B is N and A is =CH,
X is =CH,
Y is O,
E, G and T are =CH,
Z is C,
Q is -CH=CH-CH=,
W is not present,
R₁ is -(C₀₋₇)-NR₄R₅ and R₄ and R₅ are as defined above,
R₂ is substituted aryl.

In a further aspect of the present invention the VEGF receptor inhibitor is compound of formula 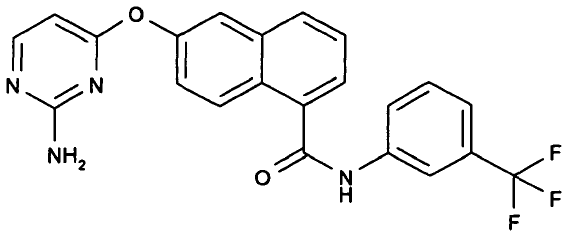 or a pharmaceutically acceptable salt thereof.

Compositions provided by the present invention are hereinafter designated as "composition(s) of (according to) the present invention". The VEGF receptor inhibitor of formula I in a composition of the present invention includes a VEGF receptor inhibitor of formula I in any form, e.g. in free form, in the form of a salt, in the form of a solvate and in the form of a salt and a solvate.

We now found that a combination of VEGF receptor inhibitor compounds of the present invention when combined with a substance selected from oleyl alcohol leads to enhanced skin penetration. This was surprising, especially in view of the fact that a combination of DMSO, which is known to be one of the standard penetration enhancers, when applied together with a VEGF receptor inhibitor compound of the present invention does not work, i.e. no penetration enhancing effect has been found.

"Penetration enhancer" as used herein means a substance that enhances, i.e. improves the penetration of a VEGF receptor inhibitor of formula I , e.g. when administered topically (epicutanously), into skin or mucosa, e.g. into skin, such as the lower epidermis and the dermis, compared with the penetration for a VEGF receptor inhibitor of formula I without that penetration enhancer. This enhanced penetration will lead to higher levels within the skin, in particular in the lower epidermis and the dermis. Higher penetration may also result in an increased permeation, e.g. increased permeation through the skin. Preferably the delivery of a VEGF receptor inhibitor of formula I to the systemic circulation is not or not significantly enhanced.

In a further aspect the present invention provides a composition of the present invention in a form for topical administration, as a cream, gel, spray.

A penetration enhancer preferably is present in an amount between 1 to 20 % w/w, more preferably between 1 to 10% w/w.

In another aspect the present invention provides a VEGF receptor inhibitor of formula I in a composition of the present invention in the form of a salt.

Such salts include preferably pharmaceutically acceptable salts, although pharmaceutically unacceptable salts are included, e.g. for preparation / isolation / purification purposes. A salt of a compound of the present invention includes a metal salt or an acid addition salt. Metal salts include for example alkali or earth alkali salts; acid addition salts include salts of a compound of formula I with an acid, e.g. hydrogen fumaric acid, fumaric acid, naphthalin-1,5-sulphonic acid, hydrochloric acid, deuterochloric acid; preferably hydrochloric acid.

A VEGF receptor inhibitor of formula I in a composition of the present invention in free form may be converted into a corresponding compound in the form of a salt; and vice versa. A VEGF receptor inhibitor of the present invention in free form or in the form of a salt and in the form of a solvate may be converted into a corresponding compound in free form or in the form of a salt in non-solvated form; and vice versa.

A VEGF receptor inhibitor of formula I in a composition of the present invention may exist in the form of pure isomers or mixtures thereof; e.g. optical isomers, diastereoisomers, cis/trans isomers. A VEGF receptor inhibitor of formula I in a composition of the present invention may e.g. contain asymmetric carbon atoms and may thus exist in the form of enantiomers or diastereoisomers and mixtures thereof, e.g. racemates. Any asymmetric carbon atom may be present in the (R)-, (S)- or (R,S)-configuration, preferably in the (R)- or (S)-configuration.

Isomeric mixtures may be separated as appropriate, e.g. according, e.g. analogously, to a method as conventional, to obtain pure isomers. The present invention includes a VEGF receptor inhibitor in any isomeric form and in any isomeric mixture.

The present invention also includes tautomers of a VEGF receptor inhibitor of formula I, where tautomers can exist.

VEGF receptor inhibitors of formula I, are prepared analogously to methods that, for other compounds, are in principle known in the art, and are especially prepared according to the methods described herein below under 'Examples'.

The starting materials used in the preparation of the VEGF receptor inhibitors of formula I are known, capable of being prepared according to known processes, or commercially obtainable. In particular, the anilines to be used as starting material in the preparation of the compounds of formula I can be prepared as described in WO 03/099771 or analogously thereto, are commercially available or can be prepared according to known processes.

The compositions of the present invention, including a compound of formula I, exhibit pharmacological activity and are therefore useful as pharmaceuticals.

In another aspect the present invention provides a composition for use as a pharmaceutical for topical administration, e.g. in the form of a cream.

A composition of the present invention shows therapeutic activity in dermatological diseases, e.g. proriasis, atopic dermatitis and acne.

In a further aspect the present invention provides a composition of the present invention for use in the treatment of a dermatological disease, e.g. selected from the group consisting of psoriasis, atopic dermatitis and acne.

For pharmaceutical use a composition of the present invention includes one or more, preferably one, VEGF receptor inhibitors of formula I, e.g. a combination of two or more VEGF receptor inhibitors of formula I.

In another aspect the present invention provides a composition of the present invention for use as a medicament, e.g. a pharmaceutical composition, for the treatment of a dermatological disease, e.g. selected from the group consisting of psoriasis, atopic dermatitis and acne.

In a further aspect the present invention provides a composition of the present invention for use in the method of treatment of a dermatological disease selected from the group consisting of psoriasis, atopic dermatitis and acne, which treatment comprises administering to a subject in need of such treatment an effective amount of a composition of the present invention.

Treatment includes treatment and prophylaxis.

For such treatment, the appropriate dosage will, of course, vary depending upon, for example, the chemical nature and the pharmacokinetic data of a compound of the present invention employed, the individual host, the mode of administration and the nature and severity of the conditions being treated. However, in general, for satisfactory results in larger mammals, for example humans, an indicated daily dosage is in the range from about 0.01 g to about 1.0 g, of a compound of the present invention; conveniently administered, for example, in divided doses up to four times a day.

A composition of the present invention may be administered topically; e.g. including epicutaneous, intranasal, intratracheal administration; e.g. in the form of creams, gels, pastes, inhaler powder, foams, tinctures, lip sticks, drops or sprays.

A composition of the present invention may be used for pharmaceutical treatment according to the present invention alone or in combination with one or more other pharmaceutically active agents. Combinations include fixed combinations, in which two or more pharmaceutically active agents are in the same formulation; kits, in which two or more pharmaceutically active agents in separate formulations are sold in the same package, e.g. with instruction for co-administration; and free combinations in which the pharmaceutically active agents are packaged separately, but instruction for simultaneous or sequential administration are given.

In another aspect the present invention provides a pharmaceutical composition comprising a compound of the present invention in association with at least one pharmaceutical excipient, e.g. appropriate carrier and/or diluent, e.g. including fillers, binders, disintegrators, flow conditioners, lubricants, sugars and sweeteners, fragrances, preservatives, stabilizers, wetting agents and/or emulsifiers, solubilizers, salts for regulating osmotic pressure and/or buffers.

In another aspect the present invention provides a pharmaceutical composition of the present invention, further comprising another pharmaceutically active agent.

Such compositions may be manufactured according, e.g. analogously to a method as conventional, e.g. by mixing, granulating, coating, dissolving or lyophilizing processes. Unit dosage forms may contain, for example, from about 0.5 mg to about 1000 mg, such as 1 mg to about 500 mg.

In a further aspect the present invention provides a kit of parts comprising
a) a VEGF receptor inhibitor of formula I as defined above, and
b) a penetration enhancer, as defined above.

The following Examples serve to illustrate the invention .

Temperatures are given in degrees Celsius (°). Unless otherwise indicated, the reactions take place at room temperature under N₂-atmosphere.

The R_{f} values which indicate the ratio of the distance moved by each substance to the distance moved by the eluent front are determined on silica gel thin-layer plates (Merck, Darmstadt, Germany) by thin-layer chromatography using the respective named solvent systems.

### Abbreviations:

- Anal.: elemental analysis (for indicated atoms, difference between calculated and measured value ≤ 0.4 %)
- aq.: aqueous
- brine: saturated solution of NaCl in H₂O
- BuOH: butanol
- conc.: concentrated
- DEPC: diethyl-cyanophosphonate
- DIPE: diisopropyl-ether
- DMAP: dimethylaminopyridine
- DMF: dimethyl formamide
- DMSO: dimethyl sulfoxide
- ether: diethylether
- Et₃N: triethylamine
- EtOAc: ethyl acetate
- EtOH: ethanol
- eq.: equivalent
- Ex.: Example
- h: hour(s)
- HATU: O-(7-azabenzotriazol-1-yl)-N,N,N',N'-tetramethyluronium-hexafluorophosphate
- HPLC: high pressure liquid chromatography
- I: litre(s)
- Me: methyl
- MeOH: methanol
- min: minute(s)
- m.p.: melting point
- MPLC: medium pressure liquid chromatography

- Combi Flash system: normal phase SiO₂
- Gilson system: reversed phase Nucleosil C18 (H₂O/CH₃CN + TFA), generally product obtained as free base after neutralization with NaHCO₃

- MS: mass spectrum
- NMM: N-methyl-morpholine
- NMP: N-methyl-pyrrolidone
- prep-HPLC: preparative high pressure liquid chromatography ; Waters system ; column: reversed phase Atlantis^{™} (100 x 19 mm), dC18 OBD (H₂O/CH₃CN + 0.1% TFA), 5 µM, generally product obtained as a TFA salt after lyophilization. propylphosphonic anhydride N-propylphosphonic acid anhydride, cyclic trimer [68957-94-8]; 50 % in DMF
- R_{f}: ratio of fronts (TLC)
- rt: room temperature
- sat.: saturated
- THF: tetrahydrofuran (distilled from Na/benzophenone)
- TFA: trifluoroacetic acid
- TLC: thin layer chromatography
- t*_{Ret}*: retention time (HPLC)
- triphosgene: bis(trichloromethyl) carbonate

### HPLC Conditions:

- ^{A}t*_{Ret}*:: retention time [min] for *System A:* Linear gradient 20-100% CH₃CN (0.1 % TFA) and H₂O (0.1% TFA) in 13 min + 5 min 100% CH₃CN (0.1% TFA); detection at 215 nm, flow rate 1 ml/min at 25 or 30 °C. Column: Nucleosil 120-3 C18 (125 x 3.0 mm).
- ^{B}t*_{Ret}:*: retention time [min] for *System B:* Linear gradient 5-40% CH₃CN (0.1 % TFA) and H₂O (0.1 % TFA) in 9 min + 7 min 40% CH₃CN (0.1% TFA); detection at 215 nm, flow rate 1 ml/min at 25 or 30 °C. Column: Nucleosil 120-3 C18 (125 x 3.0 mm).
- ^{C}t*_{Ret}*:: retention time [min] for *System C.* Linear gradient 15-100% CH₃CN (0.1% TFA) and H₂O (0.1% TFA) in 2.25 min + 1.25 min 100% CH₃CN (0.1% TFA); detection at 215 nm, flow rate 2 ml/min at 25 or 30 °C. Column: CC (50 x 4.6 mm) Uptisphere UP3ODB-5QS.
- ^{D}t*_{Ret}*:: retention time [min] for *System D.* Linear gradient 20-100% CH₃CN (0.1% TFA) and H₂O (0.1% TFA) in 5 min + 1 min 100% CH₃CN (0.1% TFA); detection at 215 nm, flow rate 1 ml/min at 25 or 30 °C. Column: CC (250 x 4.6 mm) Nucleosil 100-5 C18.
- ^{E}t*_{Ret}*:: retention time [min] for *System E*: Linear gradient 20-100% CH3CN (0.1 % TFA) and H₂O (0.1% TFA) in 14 min + 5 min 100% CH₃CN (0.1 % TFA); detection at 215 nm, flow rate 1 ml/min at 25 or 30 °C. Column: CC 70/4 (70 x 4.0 mm) Nucleosil 100-3 C18.
- ^{F}t*_{Ret}*:: retention time [min] for *System F:* Linear gradient 5-100% CH₃CN and H₂O (0.1% TFA) in 4 min + 0.5 min 100% CH₃CN; PDA MaxPlot detection (210.0 nm to 400.0 nm), flow rate 3 ml/min at 35 °C. Column: Sunfire^{™} (4.6 x 20 mm) C18, 3.5 µm.
- ^{G}t*_{Ret}*:: retention time [min] for *System G*: Linear gradient 5-100% CH₃CN (0.07% formic acid) and H₂O (0.1% formic acid) in 4 min + 0.5 min 100% CH₃CN (0.07% formic acid); PDA MaxPlot detection (210.0 nm to 400.0 nm), flow rate 1.8 ml/min at 40°C. Column: X-Terra^{™} (4.6 x 50 mm) MSC18, 5 µm.

### Example 1: rac-5-(2-Amino-6-chloro-pyrimidin-4-yloxy)-4-fluoro-2-methyl-2,3-dihydro-indole-1-carboxylic acid (3-trifluoromethyl-phenyl)-amide

0.68 mMol *rac*-5-(2-Amino-6-chloro-pyrimidin-4-yloxy)-4-fluoro-2-methyl-2,3-dihydro-indole (Step 1.2) are dissolved in 5 ml THF. Then 106 µl (0.77 mMol) 3-trifluoromethyl-phenylisocyanat are added and the solution is stirred for 1 h at rt. Concentration and chromatography (Combi Flash; EtOAc/hexane 1:9 → 1:1) gives the title compound: MS: [M+1]⁺ = 482/484; HPLC: ^{A}t*_{Ret}* = 16.4; Anal.: C,H,N,Cl,F.

The starting material is prepared as follows:

### Step 1.1: 5-(2-Amino-6-chloro-pyrimidin-4-yloxy)-4-fluoro-2-methyl-1H-indole

1.00 g (6.05 mMol) 2-amino-4,6-dichloro-pyrimidine and 993 mg (6.05 mMol) 4-fluoro-5-hydroxy-2-methylindole [preparation see: WO 00/47212 Ex. 237] are suspended in 25 ml acetone. Then 12 ml 1 N NaOH in H₂O are added and the mixture is stirred at an oilbath temperature of 70 °C for 5 h. Then additional 210 mg 2-amino-4,6-dichloro-pyrimidine are added portionwise and stirring continued for another 4.5 h. The reaction mixture is partially concentrated and the crystallized title compound filtered off and washed with H₂O: m.p.: 255-258 °C; MS: [M+1]⁺ = 293/295; HPLC: ^{A}t*_{Ret}* = 13.7.

### Step 1.2: rac-5-(2-Amino-6-chloro-pyrimidin-4-yloxy)-4-fluoro-2-methyl-2,3-dihydro-1H-indole

A solution of 200 mg (0.68 mMol) of 5-(2-amino-6-chloro-pyrimidin-4-yloxy)-4-fluoro-2-methyl-1H-indole in 4 ml acetic acid is cooled to 10-15 °C. Then 214 mg (3.4 mMol) NaBH₃CN are added. After 3 h stirring at rt, 8 g of ice are added, then the mixture is made basic by addition of 1 N NaOH and extracted three times with EtOAc. The organic layers are washed with H₂O and brine, dried (Na₂SO₄) and concentrated at rt *in vacuo:* HPLC: *^{A}t_{Ret}* = 9.9.

### Example 2: rac-5-(2-Amino-pyrimidin-4-yloxy)-4-fluoro-2-methyl-2,3-dihydro-indole-1-carboxylic acid (3-trifluoromethyl-phenyl)-amide

A solution of 230 mg (0.48 mMol) *rac*-5-(2-amino-6-chloro-pyrimidin-4-yloxy)-4-fluoro-2-methyl-2,3-dihydro-indole-1-carboxylic acid (3-trifluoromethyl-phenyl)-amide and 75 µl (0.54 mMol) Et₃N in 12 ml THF is hydrogenated in the presence of 63 mg Pd/C (10 %; Engelhard 4505). The catalyst is filtered off, the filtrate concentrated and the residue dissolved in EtOAc and H₂O. The aqueous layer is extracted twice with EtOAc. The organic layers are washed with H₂O and brine, dried (Na₂SO₄) and concentrated. Crystallization from EtOAc/DIPE gives the title compound: MS: [M+1]⁺ = 448; HPLC: ^{A}t*_{Ret}* = 12.7.

### Example 3: 5-(6-Chloro-pyrimidin-4-yloxy)-indole-1-carboxylic acid (3-trifluoromethyl-phenyl)-amide

A solution of 492 mg (2.00 mMol) of 5-(6-chloro-pyrimidin-4-yloxy)-1H-indole (WO 03/099771; Stage 163.1), 344 mg (2.12 mMol) of 1,1'-carbonyl-diimidazol and 6 mg DMAP in 7 ml CH₃CN is stirred at 80 °C for 8 h. After cooling to rt, 356 µl (2.86 mMol) 3-trifluoromethyl-aniline are added to the suspension, which then is stirred again for 9 h at 80 °C. The reaction mixture is filtered and the filtrate diluted with EtOAc and H₂O. The aqueous layer is extracted twice with EtOAc. The organic layers are washed with H₂O and brine, dried (Na₂SO₄) and concentrated. Reversed phase MPLC (Gilson system) gives the title compound: MS: [M+1]⁺ = 433/435; TLC(EtOAc/CH₂Cl₂ 3:97): R_{f} = 0.15; HPLC: ^{A}t*_{Ret}* = 16.8.

### Example 4: 5-(6-Amino-pyrimidin-4-yloxyl-indole-1-carboxylic acid (3-trifluoromethyl-phenyl)-amide

To a solution of 150 mg (0.35 mMol) 5-(6-chloro-pyrimidin-4-yloxy)-indole-1-carboxylic acid (3-trifluoromethyl-phenyl)-amide in 2 ml DMF, 45 mg (0.69 mMol) NaN₃ are added at rt. After 2 h at 65 °C, the solution containing 5-(6-azido-pyrimidin-4-yloxy)-indole-1-carboxylic acid (3-trifluoromethyl-phenyl)-amide is cooled to rt. Then 12 mg Pd/C (10 %; Engelhard 4505) are added and the mixture is hydrogenated for 1 h. The catalyst is filtered off and the filtrate concentrated *in vacuo.* Reversed phase MPLC (Gilson system) gives the title compound: MS: [M+1]⁺ = 414; HPLC: ^{A}t*_{Ret}* = 12.6.

### Example 5: 7-(6-Amino-pyrimidin-4-yloxy)-2,3-dihydro-benzo[1,4]oxazine-4-carboxylic acid (4-methyl-3-trifluoromethyl-phenyl)-amide

104 mg (0.35 mMol) triphosgene are disolved in 13 ml ice cooled CH₂Cl₂. Then a solution of 183 mg (1.05 mMol) 5-amino-2-methylbenzotrifluoride and 209 µl (1.5 mMol) Et₃N in 6 ml CH₂Cl₂ is added during 8 min. After 3 minutes, the mixture is warmed up to rt by a H₂O bath and then a solution of 244 mg (1.00 mMol) 6-(3,4-dihydro-2H-benzo[1,4]oxazin-7-yloxy)-pyrimidin-4-ylamine (Step 5.6) and 139 µl (1.0 mMol) Et₃N in 6 ml CH₂Cl₂ is added during 10 min. After 2.5 h at rt, the mixture is diluted with sat. Na₂CO₃/H₂O 1:1 and EtOAc, the aqueous phase separated off and extracted twice with EtOAc. The organic layers are washed with H₂O and brine, dried (Na₂SO₄) and concentrated partially. Addition of ether and hexane gives the crystalline title compound: MS: [M+1]⁺ = 446; TLC(EtOAc): R_{f} = 0.30; HPLC: ^{A}t*_{Ret}* = 12.3.

The starting material is prepared as follows:

### Step 5.1: 2-(2,4-Dimethoxy-phenylamino)-ethanol

30.6 g (0.20 Mol) 2,4-dimethoxy-aniline are dissolved in 200 ml EtOAc. Then 14.8 ml (95 %; 0.20 Mol) of 2-bromethanol and 33.6 g (0.40 Mol) NaHCO₃ are added and the suspension is heated to 77 °C for 20 h. The mixture is cooled to rt and filtered. Concentration of the filtrate and column chromatography (SiO₂; hexane/EtOAc 3:1 → 2:1 → 1:1) gives the title compound as an oil: MS: [M+1]⁺ = 198; TLC(EtOAc): R_{f} = 0.50; HPLC: ^{B}t*_{Ret}* = 9.2.

### Step 5.2: (2-Bromo-ethyl)-(2,4-dihydroxy-phenyl)-carbamic acid benzyl ester

22 g (0.11 Mol) 2-(2,4-dimethoxy-phenylamino)-ethanol and 125 ml HBr (62 % in H₂O) are heated to 140 °C for 15 h. The resulting dark solution is concentrated *in vacuo.* The residue is diluted with 60 ml H₂O and 120 ml EtOAc and cooled in an ice bath. Then 30 ml (95 %; 0.20 Mol) benzyl chloroformate are added. Under vigorous stirring, 90 ml Na₂CO₃ 2 M are added dropwise during 20 min. After 90 min stirring at rt, the reaction mixture is filtered, the aqueous phase of the filtrate separated off and extracted twice with 30 ml EtOAc. The organic layers are washed with 30 ml brine, dried (Na₂SO₄) and concentrated. Column chromatography (SiO₂; hexane/EtOAc 4:1 → 7:3 → 3:2) gives the title compound as an oil: MS: [M+1]⁺ = 366/368; TLC(EtOAc/hexane 1:1): R_{f} = 0.34; HPLC: ^{A}t*_{Ret}* = 12.6.

### Step 5.3: 7-Hydroxy-2,3-dihydro-benzo[1,4]oxazine-4-carboxylic acid benzyl ester

To a solution of 32 g (87.4 mMol) (2-bromo-ethyl)-(2,4-dihydroxy-phenyl)-carbamic acid benzyl ester in 100 ml DMF, 16 g (116 mMol) K₂CO₃ are added. After 1 h stirring at rt, the reaction mixture is filtered and the filtrate concentrated *in vacuo.* The resulting brown oil is diluted with 120 ml EtOAc and 50 ml citric acid (5 % in H₂O). The aqueous phase is separated off and extracted twice with EtOAc. The organic layers are washed with H₂O and brine, dried (Na₂SO₄) and concentrated, giving the oily title compound: MS: [M+1]⁺ = 286;
TLC(EtOAclhexane 1:1): R_{f} = 0.61; HPLC: ^{A}t*_{Ret}* = 13.0.

### Step 5.4: 7-(6-Chloro-pyrimidin-4-yloxy)-2,3-dihydro-benzo[1,4]oxazine-4-carboxylic acid benzyl ester

To a solution of 9.73 g (95 %; 32.5 mMol) 7-hydroxy-2,3-dihydro-benzo[1,4]oxazine-4-carboxylic acid benzyl ester in 96 ml of acetone, 4.84 g (32.5 mMol) 4,6-dichloropyrimidine are added. Then a solution of 1.3 g (32.5 mMol) NaOH in 48 ml of H₂O is added and the mixture stirred for 2 h at rt. Addition of some seeding crystals leads to the crystallization of the title compound, which is filtered off and washed with acetone/H₂O 1:1: m.p.: 110 °C; MS: [M+1]⁺ = 398/400; HPLC: ^{A}t*_{Ret}* = 16.4. More product can be isolated from the filtrate by extraction (EtOAc; NaHCO₃, H₂O and brine) and column chromatography (SiO₂; hexane/EtOAc 9:1 → 4:1 → 3:1).

### Step 5.5: 7-(6-Azido-pyrimidin-4-yloxy)-2,3-dihydro-benzo[1,4]oxazine-4-carboxylic acid benzyl ester

To a solution of 8.0 g (20.1 mMol) 7-(6-chloro-pyrimidin-4-yloxy)-2,3-dihydro-benzo[1,4]oxazine-4-carboxylic acid benzyl ester in 35 ml DMF, 2.61 g (40.2 mMol) NaN₃ are added. After heating at 70 °C for 90 min, the resulting mixture is diluted with EtOAc and H₂O, the aqueous phase separated off and extracted twice with EtOAc. The organic layers are washed with H₂O and brine, dried (Na₂SO₄) and concentrated, giving the title compound: MS: [M+1]⁺ = 405; HPLC: ^{A}t*_{Ret}* = 16.6.

### Step 5.6: 6-(3,4-Dihydro-2H-benzo[1,4]oxazin-7-yloxy)-pyrimidin-4-ylamine

A solution of 20.1 mMol 7-(6-azido-pyrimidin-4-yloxy)-2,3-dihydro-benzo[1,4]oxazine-4-carboxylic acid benzyl ester in 500 ml THF is hydrogenated in the presence of 3.5 g Pd/C (10 %; Engelhard 4505). The catalyst is filtered off, the filtrate concentrated and crystallized from DIPE, giving the title compound: m.p.: 140-141 °C; MS: [M+1]⁺ = 245; TLC(EtOAc): R, = 0.11.

### Example 6: 7-(6-Amino-pyrimidin-4-yloxy)-2,3-dihydro-benzo[1,4]oxazine-4-carboxylic acid (4-fluoro-3-trifluoromethyl-phenyl)-amide

Prepared analogously to Ex. 5: MS: [M+1]⁺ = 450; TLC(EtOAc): R_{f} = 0.23; HPLC: ^{A}t*_{Ret}* = 11.9.

### Example 7: 7-(6-Amino-pyrimidin-4-yloxy)-2,3-dihydro-benzo[1,4]oxazine-4-carboxylic acrid (3-trifluoromethyl-phenyl)-amide

A solution of 120 µl (0.87 mMol) 3-trifluoromethyl-phenyl-isocyanate in 9 ml ether is added dropwise to 0.20 g (0.82 mMol) 6-(3,4-dihydro-2H-benzo[1,4]oxazin-7-yloxy)-pyrimidin-4-ylamine in 4.5 ml THF. After 45 min at rt, 10 ml hexane are added. Then the crystallized title compound is filtered off and washed with hexane: MS: [M+1]⁺ = 432; TLC(EtOAc): R_{f} = 0.32; HPLC: ^{A}t*_{Ret}*= 11.9; Anal.: C,H,N,F.

### Example 8: 7-(6-Methylamino-pyrimidin-4-yloxy)-2,3-dihydro-benzo[1,4]oxazine-4-carboxylic acid (4-methyl-3-trifluoromethyl-phenyl)-amide

73 mg (0.25 mMol) triphosgene are disolved in 9 ml ice cooled CH₂Cl₂. Then a solution of 134 mg (97 %; 0.74 mMol) 5-amino-2-methylbenzotrifluoride and 146 µl (1.05 mMol) Et₃N in 4 ml CH₂Cl₂ is added during 5 min. After 3 minutes, the mixture is warmed up to rt by a H₂O bath and then a solution of 180 mg (0.70 mMol) [6-(3,4-dihydro-2H-benzo[1,4]oxazin-7-yloxy)-pyrimidin-4-yl]-methyl-amine (Step 8.2) and 98 µl (0.70 mMol) Et₃N in 4 ml CH₂Cl₂ is added during 5 min. After 2.5 h at rt, the mixture is diluted with sat. Na₂CO₃/H₂O 1:1 and EtOAc, the aqueous phase separated off and extracted twice with EtOAc. The organic layers are washed with H₂O and brine, dried (Na₂SO₄) and concentrated. Crystallization from THF and hexane gives the title compound: m.p.: 180-182 °C; MS: [M+1]⁺ = 460; TLC(EtOAc): R_{f} = 0.38; HPLC: ^{B}t*_{Ret}* = 12.7.

The starting material is prepared as follows:

### Step 8.1: 7-(6-Methylamino-pyrimidin-4-yloxy)-2,3-dihydro-benzo[1,4]oxazine-4-carboxylic acid benzyl ester

To a suspension of 1.39 g (3.49 mMol) 7-(6-chloro-pyrimidin-4-yloxy)-2,3-dihydro-benzo[1,4]oxazine-4-carboxylic acid benzyl ester (Step 5.4) in 8 ml THF, 8.7 ml (2 M in THF; 17.5 mMol) methylamine are added. After stirring in a sealed vessel for 16 h at rt, the resulting mixture is dissolved with EtOAc and MeOH. Then 6 g of SiO₂ are added and the mixture is concentrated *in vacuo.* The resulting powder is put on top of a SiO₂ column (EtOAc/hexane 1:3) and the title compound eluted with EtOAc/hexane 1:3 → 1:1 → 2:1: MS: [M+1]⁺ = 393; TLC(EtOAc/hexane 1:1): Rₜ = 0.08; HPLC: ^{A}t*_{Ret}* = 12.1.

### Step 8.2: [6-(3,4-Dihydro-2H-benzo[1,4]oxazin-7-yloxy)-pyrimidin-4-yl]-methyl-amine

A solution of 0.79 g (2.0 mMol) 7-(6-methylamino-pyrimidin-4-yloxy)-2,3-dihydro-benzo[1,4]oxazine-4-carboxylic acid benzyl ester in 50 ml THF is hydrogenated in the presence of 0.35 g Pd/C (10 %; Engelhard 4505). The catalyst is filtered off, the filtrate concentrated partially and the title compound crystallized by addition of hexane: m.p.: 153 °C; MS: [M+1]⁺ = 259; TLC(EtOAc): R_{f} = 0.16; HPLC: ^{B}t*_{Ret}* = 10.6.

### Example 9: 7-(6-Methylamino-pyrimidin-4-yloxy)-2,3-dihydro-benzo[1,4]oxazine-4-carboxylic acid (3-trifluoromethyl-phenyl)-amide

Prepared analogously to Ex. 7: m.p.: 180 °C; MS: [M+1]⁺ = 446; TLC(EtOAc): R_{f} = 0.47; HPLC: ^{A}t*_{Ret}* = 12.3; Anal.: C,H,N,F.

### Example 10: 7-(2-Amino-pyrimidin-4-yloxy)-2,3-dihydro-benzo[1,4]oxazine-4-carboxylic acid (3-trifluoromethyl-phenyl)-amide

A solution of 0.183 g (0.75 mMol) 4-(3,4-dihydro-2H-benzo[1,4]oxazin-7-yloxy)-pyrimidin-2-ylamine (Step 10.2) in 3 ml of acetonitrile is treated at rt with 0.114 ml (0.825 mMol) 1-isocyanato-3-trifluoromethyl-benzene and stirred for 2 h at rt. The solvent is evaporated and the residue chromatographed on a 40 g silica gel column on a Combi-Flash Companion™ (Isco Inc.) apparatus using EtOAc as solvent. The title compound is obtained as a colorless foam: MS: [M+1]⁺ = 432; TLC(EtOAc): R_{f} = 0.31; HPLC: ^{C}t*_{Ret}* = 1.88 min.

The starting material is prepared as follows:

### Step 10.1: 7-(2-amino-6-chloro-pyrimidin-4-yloxy)-2,3-dihydro-benzo[1,4]oxazine-4-carboxylic acid benzyl ester

To a solution of 5.8 g (0.0203 Mol) crude 7-hydroxy-2,3-dihydro-benzo[1,4]oxazine-4-carboxylic acid benzyl ester (Step 5.3) in 30 ml of DMF are added 2.0 g anhydrous potassium carbonate and 3.3 g (0.0201 Mol) 2-amino-4,6-dichloropyrimidine and the resulting mixture is heated to 80°C for 16 h. After cooling to rt the mixture is filtered and the DMF evaporated to leave a dark brown oil. This material is purified by flash-chromatography on a 240 g silica gel column using CH₂Cl₂/MeOH 100:0.5 → 100:2.5 as eluent. Pure fractions are evaporated to leave a yellow oil. On addition of 15 ml of EtOAc the product crystallizes. It is filtered off, washed with EtOAc/hexane 1:1 and dried. The title compound is obtained as colorless crystals: m.p. 161-163 °C; MS: [M+1]⁺ = 412.9; TLC (CH₂Cl₂/MeOH/NH₃^{aq} 350:50:1): Rₜ = 0.76; HPLC: ^{C}t*_{Ret}* = 2.57 min.

### Step 10.2: 4-(3,4-dihydro-2H-benzo[1,4]oxazin-7-yloxy)-pyrimidin-2-ylamine

2.0 g (4.8 mMol) 7-(2-amino-6-chloro-pyrimidin-4-yloxy)-2,3-dihydro-benzo[1,4]oxazine-4-carboxylic acid benzyl ester are dissolved in 100 ml of a 1:1 mixture of THF and methanol and hydrogenated at rt in the presence of 0.5 g Pd/C (10 %; Engelhard 4505). The catalyst is filtered off, the filtrate concentrated and partitioned between 40 ml conc. sodium bicarbonate and 50 ml of EtOAc. The organic layer is washed with brine, dried with sodium sulfate and evaporated. The crude title compound is obtained as an amorphous material: MS: [M+1]⁺ = 245; TLC(CH₂Cl₂/MeOH/NH₃^{aq} 350:50:1): Rₜ = 0.47; HPLC: ^{C}t_{Ret} = 0.86 min.

### Example 11: 7-(2-Amino-pyrimidin-4-yloxy)-2,3-dihydro-benzo[1,4]oxazine-4-carboxylic acid (4-morpholin-4-ylmethyl-3-trifluoromethyl-phenyl)-amide

A solution of 0.86 ml (7.13 mMol) trichloromethyl chloroformate in 4 ml of dry THF is treated at 40 °C with a solution of 0.51 g (2.09 mmol) 4-(3,4-dihydro-2H-benzo[1,4]oxazin-7-yloxy)-pyrimidin-2-ylamine. The mixture is stirred under reflux for 2 h, cooled to rt and evaporated to leave a tan foam. This is added to a solution of 4-morpholin-4-ylmethyl-3-trifluoromethyl-phenylamine (0.362 g, 1.4 mMol) in 4 ml of ethanol. The mixture is heated to 80 °C and stirred at that temperature for 3 h. After cooling to rt the solvent is evaporated and the residue partitioned between CH₂Cl₂ and sat. sodium bicarbonate solution. The organic phase is dried with sodium sulfate and evaporated. The residue is purified on a 40 g silica gel column on a Combi-Flash Companion™ (Isco Inc.) apparatus using EtOAc for 10 min, then a gradient of 1 to 30 % acetone in EtOAc. The title compound is obtained as a slightly reddish foam: MS: [M+1]⁺ = 531; TLC (EtOAc): R_{f} = 0.10; HPLC: ^{C}t*_{Ret}* = 1.32 min.

### Example 12: The following compounds can be obtained analogously to Ex. 10 or 11.

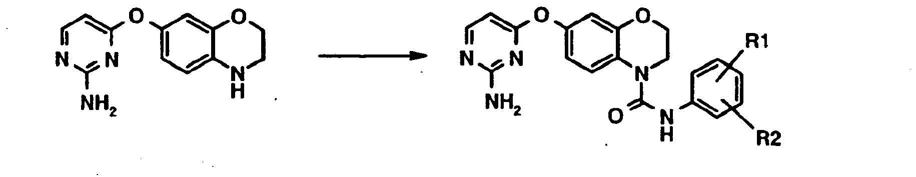

| | 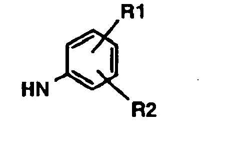 | TLC R_{f} | HPLC *t_{Ret}* [min] | m.p. [°C] | MS [M+1]⁺ |
|---|---|---|---|---|---|
| a) | 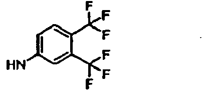 | 0.23¹⁾ | 12.7^{A)} | | 500 |
| b) | 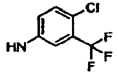 | 0.35²⁾ | 2.03^{C)} | 127-130 | 465.8 |
| c) | 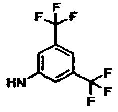 | | 2.13^{C)} | 146-149 | 499.9 |
| d) | 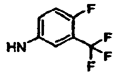 | | 1.92^{C)} | 119-124 | 449.9 |
| e) | 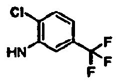 | | 2.01^{C)} | 191-195 | 465.9 |
| f) | 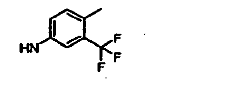 | | 1.97^{C)} | 133-136 | 445.9 |

| | | | | | |
|---|---|---|---|---|---|
| ¹⁾ TLC(EtOAc/hexane 2:1); ²⁾ TLC(EtOAc) | | | | | |

| | |
|---|---|
| 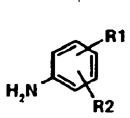 | Most respective anilines are either commercially available or described in WO 03/099771 or can be prepared analogously to the therein exemplified derivatives. |

### Example 13: 6-(2-Amino-6-chloro-pyrimidin-4-yloxy)-naphthalene-1-carboxylic acid (3-trifluoromethyl-phenyl)-amide

To an ice cooled solution of 3.5 g (11 mMol) 6-(2-amino-6-chloro-pyrimidin-4-yloxy)-naphthalene-1-carboxylic acid in 60 ml DMF, 1.8 ml (16 mMol) NMM and 3.1 ml (20 mMol) DEPC are added, followed by 1.5 ml (12 mMol) of 3-amino-benzotrifluoride. After 2 h stirring at 0 °C and 16 h at rt, the solution is concentrated *in vacuo.* The residue is re-dissolved in H₂O and EtOAc, the aqueous phase separated off and extracted twice with EtOAc. The organic layers are washed twice with H₂O and brine, dried (Na₂SO₄) and after addition of SiO₂ concentrated. The resulting powder is put on top of a SiO₂ column (EtOAc/hexane 1:9) and eluted with EtOAc/hexane 1:9 → 1:1. Partial concentration of the product containing fractions leads to crystallization. Filtration and washing with hexane gives the title compound: m.p.: 234-236 °C; MS: [M+1]⁺= 459; TLC(EtOAc/hexane 1:2): R_{f} = 0.24; HPLC: ^{A}t_{Ret} = 16.2.

The starting material is prepared as follows:

### Step 13.1: 6-(2-Amino-6-chloro-pyrimidin-4-yloxy)-naphthalene-1-carboxylic acid

A suspension of 6.56 g (40 mMol) 2-amino-4,6-dichloropyrimidine and 7.52 g (40 mMol) 6-hydroxy-1-naphthoic acid in 160 ml acetone and 80 ml 1 N NaOH^{aq.} is heated to 62°C for 36 h. The mixture is cooled to rt, partially concentrated *in vacuo* and the residue poured into 1.6 l ice H₂O. Under vigorous stirring, 20 ml 2 N HCl are added dropwise (pH ≈ 4). After stirring the suspension for 30 min, the title compound is filtered off and washed with H₂O: MS: [M+1]⁺ = 316/318; HPLC: ^{A}t*_{Ret}* = 12.8.

### Example 14: 6-(2-Amino-pyrimidin-4-yloxy)-naphthalene-1-carboxylic acid (3-trifluoromethyl-phenyl)-amide

A solution of 660 mg (1.44 mMol) 6-(2-amino-6-chloro-pyrimidin-4-yloxy)-naphthalene-1-carboxylic acid (3-trifluoromethyl-phenyl)-amide in 70 ml THF and 0.22 ml (1.58 mMol) Et₃N is hydrogenated in the presence of 0.4 g Pd/C (10 %; Engelhard 4505). The catalyst is filtered off, the filtrate concentrated and the residue diluted with EtOAc and H₂O. The aqueous layer is extracted twice with EtOAc. The organic layers are washed with H₂O and brine, dried (Na₂SO₄) and concentrated. Chromatography (Combi Flash; hexane/EtOAc 1:1 → EtOAc) gives the title compound: m.p.: 218°C; MS: [M+1]⁺ = 425; TLC(EtOAc/hexane 1:1): R_{f} = 0.07.

### Example 15: 6-(2-Amino-pyrimidin-4-yloxy)-naphthalene-1-carboxylic acid (4-methyl-3-trifluoromethyl-phenyl)-amide

To an ice cooled solution of 140 mg (0.50 mMol) 6-(2-amino-pyrimidin-4-yloxy)-naphthalene-1-carboxylic acid in 3 ml DMF, 78 µl (97 %; 0.53 mMol) 4-methyl-3-trifluoromethyl-aniline, 74 µl (0.67 mMol) NMM and 124 µl (0.83 mMol) DEPC are added. After overnight stirring, the mixture is poured into H₂O and EtOAc, the aqueous phase separated off and extracted twice with EtOAc. The organic layers are washed with H₂O and brine, dried (Na₂SO₄) and concentrated. Chromatography (Combi Flash; hexane/EtOAc 4:1 → 1:9) and crystallization from hexane gives the title compound: MS: [M+1]⁺= 439; TLC(EtOAc): R_{f} = 0.49; HPLC: ^{A}t*_{Ret}* = 12.7.

### The starting material is prepared as follows:

### Step 15.1: 6-(2-Amino-pyrimidin-4-yloxyl-naphthalene-1-carboxylic acid

A solution of 230 mg (0.73 mMol) 6-(2-amino-6-chloro-pyrimidin-4-yloxy)-naphthalene-1-carboxylic acid (Step 13.1) in 41 ml THF and 1 ml Et₃N is hydrogenated in the presence of 0.17 g Pd/C (10 %; Engelhard 4505). The catalyst is filtered off and the filtrate concentrated. The residue is dissolved in EtOAc and H₂O and the aqueous layer extracted twice with EtOAc. Acidification of the aqueous phase with 2 N HCl (→ pH 3-4) leads to the crystallization of the title compound, which is filtered off and washed with H₂O: MS: [M+1]⁺= 282; HPLC: ^{B}t_{Ret} = 13.6.

### Example 16: 6-(2-Amino-pyrimidin-4-yloxy)-naphthalene-1-carboxylic acid (2-trifluoromethyl-pyridin-4-yl)-amide

0.24 mMol 6-(2-Amino-pyrimidin-4-yloxy)-naphthalene-1-carboxylic acid, 43 mg (0.26 mMol) 4-amino-2-(trifluoromethyl)pyridine [J. Med. Chem. 36 (1993), 733-746], 20 mg (0.16 mMol) DMAP and 0.8 ml (5.7 mmol) Et₃N are dissolved in 4 ml DMF. Then 0.6 ml (1 mMol) propylphosphonic anhydride are added and the mixture is stirred for 3 days at rt. The mixture is poured into H₂O and EtOAc, the aqueous phase separated off and extracted twice with EtOAc. The organic layers are washed with H₂O and brine, dried (Na₂SO₄) and concentrated. Reversed phase chromatography gives the title compound: MS: [M+1]⁺ = 426; HPLC: ^{A}t*_{Ret}* = 11.9.

### Example 17: The following compounds can be obtained analogously to Ex. 15.

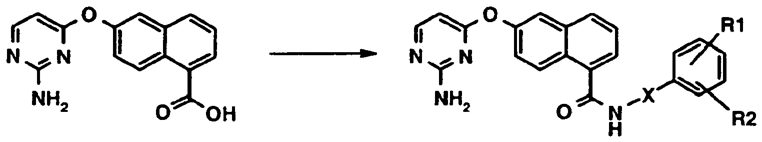

| | 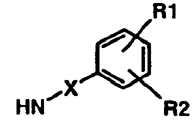 | TLC R_{f} | HPLC ^{A}t*_{Ret}* [min] | m.p. [°C] | MS [M+1]⁺ | Anal. |
|---|---|---|---|---|---|---|
| a) | 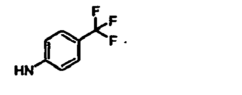 | 0.19¹⁾ | 12.4 | | 425 | |
| b) | 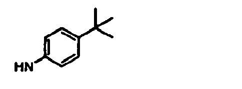 | 0.30¹⁾ | 13.1 | 228-229 | 413 | C,H,N |
| c) | 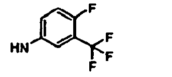 | 0.21¹⁾ | 12.6 | 205-206 | 443 | C,H,N |
| d) | 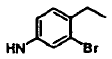 | 0.29¹⁾ | 13.5 | | 463/465 | |
| e) | 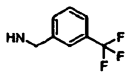 | | 12.3 | | 493 | |

| | | | | | | |
|---|---|---|---|---|---|---|
| ¹⁾ TLC(EtOAc/hexane 2:1) | | | | | | |

### Example 18: 6-(2-Acetylamino-pyrimidin-4-yloxy)-naphthalene-1-carboxylic acid (3-trifluoromethyl-phenyl)-amide

A solution of 170 mg (0.40 mMol) 6-(2-amino-pyrimidin-4-yloxy)-naphthalene-1-carboxylic acid (3-trifluoromethyl-phenyl)-amide (Ex. 14) in 3 ml pyridine is diluted with 2 ml CH₂Cl₂. Then 0.20 ml of an acetylchloride solution (2.2 M in CH₂Cl₂; 0.44 mMol) are added dropwise, followed by another 0.10 ml after 40 min. After totally 75 min, the reaction mixture is diluted with EtOAc and H₂O. The aqueous layer is separated off and extracted twice with EtOAc. The organic layers are washed with H₂O and brine, dried (Na₂SO₄) and concentrated. Chromatography (Combi Flash; hexane/EtOAc 1:1 → EtOAc), partial concentration and crystallization by addition of DIPE gives the title compound: m.p.: 216-217 °C; MS: [M+1]⁺ = 467; TLC(EtOAc): R_{f} = 0.36; HPLC: ^{A}t*_{Ret}* = 13.1; Anal.: C,H,N,F.

### Example 19: 6-(2-Methoxycarbonylamino-pyrimidin-4-yloxy)-naphthalene-1-carboxylic acid (3-trifluoromethyl-phenyl)-amide

A solution of 200 mg (0.47 mMo!) 6-(2-amino-pyrimidin-4-yloxy)-naphthalene-1-carboxylic acid (3-trifluoromethyl-phenyl)-amide (Ex. 14) in 5 ml pyridine is diluted with 3 ml CH₂Cl₂. Then 7.1 ml of a methyl chloroformate solution (2.8 M in CH₂Cl₂; 20 mMol) are added portionwise during 7 h. The reaction mixture is diluted with EtOAc and H₂O. The aqueous layer is separeted off and extracted twice with EtOAc. The organic layers are washed with H₂O and brine, dried (Na₂SO₄) and concentrated. Stirring in DIPE gives the title compound: m.p.: 199-200 °C; MS: [M+1]⁺= 483; HPLC: ^{A}t*_{Ret}* = 13.6; Anal.: C,H,N,F.

### Example 20: 7-(2-Acetylamino-pyrimidin-4-yloxy)-2,3-dihydro-benzo[1,4]oxazine-4-carboxylic acid (4-methyl-3-trifluoromethyl-phenyl)-amide

Prepared at 0 °C as described in Ex. 18 from 161 mg (0.36 mMol) 7-(2-amino-pyrimidin-4-yloxy)-2,3-dihydro-benzo[1,4]oxazine-4-carboxylic acid (4-methyl-3-trifluoromethyl-phenyl)-amide (Ex. 12f): MS: [M+1]⁺ = 488; TLC(EtOAc): R_{f} = 0.47; HPLC: ^{A}t*_{Ret}* = 12.9; Anal.: C,H,N,F.

### Example 21: 7-(2-Methoxycarbonylamino-pyrimidin-4-yloxy)-2,3-dihydro-benzo[1,4]oxazine-4-carboxylic acid (4-methyl-3-trifluoromethyl-phenyl)-amide

Prepared as described in Ex. 19 from 150 mg (0.34 mMol) 7-(2-amino-pyrimidin-4-yloxy)-2,3-dihydro-benzo[1,4]oxazine-4-carboxylic acid (4-methyl-3-trifluoromethyl-phenyl)-amide (Ex. 12f): MS: [M+1]⁺ = 504; TLC(EtOAc): R_{f} = 0.52; HPLC: ^{A}t*_{Ret}* = 13.3; Anal.: C,H,N,F.

### Example 22: 7-(2-Amino-pyrimidin-4-yloxy)-2,3-dihydro-benzo[1,4]oxazine-4-carboxylic acid [4-(4-methyl-piperazin-1-yl methyl)-3-trifluoromethyl-phenyl]-amide

A solution of 0.174 g (0.71 mMol) 4-(3,4-dihydro-2H-benzo[1,4]oxazin-7-yloxy)-pyrimidin-2-ylamine (Step 10.2) and 0.291 g (0.68 mMol) [4-(4-methyl-piperazin-1-ylmethyl)-3-trifluoromethyl-phenyl]-carbamic acid phenyl ester hydrochloride in 0.75 ml of DMSO is warmed to 60 °C. After the addition of 0.133 ml (0.77 mMol) of N,N-diisopropyl-ethylamine the mixture is stirred at 60°C for 1.5 h. A solution of 0.055 g KOH in 0.1 ml of H₂O is added at 50°C and the mixture stirred rapidly for about 10 min. After cooling to rt the mixture is partitioned between EtOAc and H₂O and the organic layer washed with brine. 2 g of silica gel are added and the solvent evaporated. The resulting powder is applied to a 40 g silica gel column on a Combi-Flash Companion™ (Isco Inc.) apparatus and eluted with EtOAc (A) and MeOH/NH₃^{aq} 10:3 (B) with a gradient of 0% B → 10% B in 30 min then 10% B for 20 min. The title compound is obtained as a yellowish foam: MS: [M+1]⁺ = 543.9; TLC (CH₂Cl₂/MeOH/NH₃^{aq} 350:50:1): R_{f} = 0.36; HPLC: ^{C}t*_{Ret}* = 1.30 min.

The starting material is prepared as follows:

### Step 22.1: [4-(4-Methyl-piperazin-1-ylmethyl)-3-trifluoromethyl-phenyl]-carbamic acid phenyl ester hydrochloride

As described in Synth. Commun. 30 (2000), 1937 the title compound can be prepared by dropwise addition of a solution of [4-(4-methyl-piperazin-1-ylmethyl)-3-trifluoromethyl]-aniline (1.0 eq.) in THF to a solution of phenyl chloroformate (1.1 eq.) in THF at -25°C and warming the mixture up to rt.

### Example 23: 6-(2-Amino-pyrimidin-4-yloxy)-naphthalene-1-carboxylic acid (4-morpholin-4-yl-3-trifluoromethyl-phenyl)-amide

To a solution of 184 mg (0.65 mMol) 6-(2-amino-pyrimidin-4-yloxy)-naphthalene-1-carboxylic acid (Step 15.1) in 5 ml NMP, 214 µl (1.95 mMol) NMM and 247 mg (0.65 mMol) HATU are added. After 15 min stirring, 242 mg (0.98 mMol) 4-morpholin-4-yl-3-trifluoromethyl-aniline are added, followed by some DMAP. After 16 h, the mixture is poured into H₂O and EtOAc, the aqueous phase separated off and extracted twice with EtOAc. The organic layers are washed with sat. Na₂CO₃/H₂O 1:1, H₂O and brine, dried (Na₂SO₄) and concentrated. Chromatography (Combi Flash; hexane/EtOAc 4:1 → 1:9) and crystallization from EtOAc/hexane gives the title compound: MS: [M+1]⁺= 510; TLC(EtOAc/hexane 2:1): R_{f} = 0.17; HPLC: ^{A}t*_{Ret}* = 12.5.

### Example 24: 6-(2-Amino-pyrimidin-4-yloxy)-naphthalene-1-carboxylic acid cis/trans-(4-isopropyl-cyclohexyl)-amide

To an ice cooled solution of 180 mg (0.47 mMol) 6-(2-amino-pyrimidin-4-yloxy)-naphthalene-1-carboxylic acid (Step 15.1) in 5 ml DMF, 69 µl (0.63 mMol) NMM and 118 µl (0.79 mMol) DEPC are added, followed by 133 mg (0.94 mMol) cis/*trans*-(4-isopropyl-cyclohexyl)-amine [Arzneim. Forsch. 19 (1969), 140]. After 16 h stirring, the mixture is poured into H₂O and EtOAc, the aqueous phase separated off and extracted twice with EtOAc. The organic layers are washed with H₂O and brine, dried (Na₂SO₄) and concentrated. Chromatography (Combi Flash; CH₂Cl₂/EtOAc 9:1 → 3:2) gives the title compound as a *cis*/*trans* mixture: MS: [M+1]⁺ = 405; TLC(CH₂Cl₂/EtOAc 1:1): R_{f} = 0.18; HPLC: ^{A}t*_{Ret}* = 13.6.

### Example 25: 6-(6-Amino-pyrimidin-4-yloxy)-naphthalene-1-carboxylic acid (4-fluoro-3-trifluoromethyl-phenyl)-amide

To an ice cooled solution of 120 mg (0.43 mMol) 6-(6-amino-pyrimidin-4-yloxy)-naphthalene-1-carboxylic acid (Step 25.3) in 3 ml DMF, 63 µl (0.57 mMol) NMM and 107 µl (0.72 mMol) DEPC are added, followed by 111 µl (0.86 mMol) 4-fluoro-3-trifluoromethyl-aniline. The solution is stirred for 1 h at 0 °C and 5 h at rt. Then it is poured into H₂O and EtOAc, the aqueous phase separated off and extracted twice with EtOAc. The organic layers are washed with H₂O and brine, dried (Na₂SO₄) and concentrated. Chromatography (Combi Flash; hexane/EtOAc 4:1 → 1:9) and crystallization from hexane gives the title compound: m.p.: 224-225 °C; MS: [M+1]⁺= 443; TLC(EtOAc): R₁ = 0.38; HPLC: ^{A}t*_{Ret}* = 12.5.

The starting material is prepared as follows:

### Step 25.1: 6-(6-Chloro-pyrimidin-4-yloxy)-naphthalene-1-carboxylic acid

23.6 g (125 mMol) 6-hydroxy-1-naphthoic acid are dissolved in a solution of 10.7 g (265 mMol) NaOH in 125 ml H₂O. Then 19.8 g (133 mMol) 4,6-dichloro-pyrimidine dissolved in 125 ml acetone are added dropwise during 30 min. The suspension is stirred for 20 h at rt and then partially concentrated *in vacuo.* The resulting residue is diluted with 600 ml EtOAc and 300 ml H₂O and acidified to pH 3 with 4 N HCl. The aqueous layer is separated off and extracted 3 times with EtOAc. The organic phases are washed 3 times with H₂O and brine, dried (Na₂SO₄), treeted with char coal and partially concentrated. The resulting suspension is diluted with 400 ml ether, the crystals filtered off and washed with hexane, yielding the title compound: m.p.: 194-195 °C; MS: [M+1]⁺ = 301.

### Step 25.2: 6-(6-Azido-pyrimidin-4-yloxy)-naphthalene-1-carboxylic acid

To 1.00 g (3.3 mMol) 6-(6-chloro-pyrimidin-4-yloxy)-naphthalene-1-carboxylic acid in 11 ml DMF, 0.43 g (6.6 mMol) NaN₃ are added. After stirring for 2.5 h at 60 °C, the reaction mixture is concentrated *in vacuo* (40°C). The residue is dissolved in H₂O and EtOAc, the aqueous phase separated off and extracted twice with EtOAc. The organic layers are washed with brine, dried (Na₂SO₄) and concentrated, giving the title compound: MS: [M+1]⁺ = 308; HPLC: ^{A}t*_{Ret}* = 13.4. More product can be precipitated from the combined aqueous phases by acidifying them with citric acid to pH ≈ 2.

### Step 25.3: 6-(6-Amino-pyrimidin-4-yloxy)-naphthalene-1-carboxylic acid

0.49 g (1.6 mMol) 6-(6-azido-pyrimidin-4-yloxy)-naphthalene-1-carboxylic acid in 25 ml THF are hydrogenated in the presence of 0.2 g Pd/C (10 %; Engelhard 4505). The partially crystallized product can be isolated by dissolving it in a mixture of MeOH/EtOAc/THF at 40 °C, filtration, extensively washing with MeOH/CH₂Cl₂, and concentration of the filtrate: m.p.: 288-290 °C; MS: [M+1]⁺ = 282; HPLC: ^{A}t*_{Ret}* = 8.6.

Alternative method for synthesis of 6-(6-amino-pyrimidin-4-yloxy)-naphthalene-1-carboxylic acid 1.00 g (3.3 mMol) 6-(6-Chloro-pyrimidin-4-yloxy)-naphthalene-1-carboxylic acid and 0.43 g (6.6 mMol) NaN₃ are stirred for 2 h at 65 °C in 11 ml DMF. The suspension is cooled to rt, 200 mg Pd/C (10 %; Engelhard 4505) are added and the mixture is hydrogenated for 16 h. The catalyst is filtered off and the filtrate concentrated *in vacuo.* The residue is re-dissolved in 5 ml DMF and poured into 150 ml H₂O and 3 ml 10 % citric acid. Filtration and washing with H₂O gives the title compound.

### Example 26: 6-(6-Amino-pyrimidin-4-yloxy)-naphthalene-1-carboxylic acid (3-trifluoromethyl-phenyl)-amide

To a solution of 11.0 g (39.1 mMol) 6-(6-amino-pyrimidin-4-yloxy)-naphthalene-1-carboxylic acid, 5.9 ml (47 mMol) 3-trifluoromethyl-aniline, 54 ml (390 mMol) Et₃N and 2.4 g (19.6 mMol) DMAP in 200 ml DMF, 46 ml (78 mMol) propylphosphonic anhydride are added dropwise. After 2 h, the mixture is concentrated *in vacuo* and the residue diluted with H₂O and EtOAc. The aqueous phase is separated off and extracted twice with EtOAc. The organic layers are washed with H₂O and brine, dried (Na₂SO4) and concentrated. Column chromatography (SiO₂; CH₂Cl₂/EtOAc 2:1 → 1:1 → 1:3) and crystallization from EtOAc gives the title compound: m.p.: 243-244 °C;. MS: [M+1]⁺ = 425; HPLC: ^{A}t*_{Ret}* = 12.6; Anal.: C,H,N,F.

### Example 27: 6-(6-Chloro-pyrimidin-4-yloxy)-benzooxazole-3-carboxylic acid (3-trifluoromethyl-phenyl)-amide

To a solution of 300 mg (0.92 mMol) 6-hydroxy-benzooxazole-3-carboxylic acid (3-trifluoromethyl-phenyl)-amide (Step 27.3) and 138 mg (0.92 mMol) 4,6-dichlorpyrimidine in 9 ml acetone, 0.92 ml NaOH^{aq.} 1 N are added. Then the mixture is stirred for 120 min at 50 °C and concentrated *in vacuo.* The residue is dissolved in EtOAc and H₂O, the aqueous phase separated off and extracted twice with EtOAc. The organic layers are washed with H₂O and brine, dried (Na₂SO₄) and concentrated. Chromatography (Combi Flash; hexane/EtOAc 85:15 → 4:1) gives the title compound: MS: [M+1]⁺ = 437; TLC(hexane/EtOAc 1:1): R_{f} = 0.42; HPLC: ^{A}t*_{Ret}* = 15.9.

The starting material is prepared as follows:

### Step 27.1: 1-(4-Benzyloxy-2-hydroxy-phenyl)-3-(3-trifluoromethyl-phenyl)-urea

To a solution of 4.44 g (20.6 mMol) 2-amino-5-benzyloxy-phenol [preparation see: WO 03/045925; page 146] in 90 ml THF, a solution of 3.01 ml (21.9 mMol) 3-trifluoromethyl-phenylisocyanate in 90 ml THF is added dropwise. After 15 h at rt, the reaction mixture is concentrated partially *in vacuo,* the residue is re-dissolved in H₂O and EtOAc, the aqueous phase separated off and extracted twice with EtOAc. The organic layers are washed with H₂O and brine, dried (Na₂SO₄) and concentrated. The crude product is dissolved in 50 ml boiling EtOAc. Addition of 40 ml hexane and cooling to rt gives the crystalline title compound: m.p.: 184-185 °C; MS: [M+1]⁺ = 403; HPLC: ^{A}t*_{Ret}* = 15.3.

### Step 27.2: 6-Benzyloxy-benzooxazole-3-carboxylic acid (3-trifluoromethyl-phenyl)-amide

To a solution of 6.80 g (16.9 mMol) of 1-(4-benzyloxy-2-hydroxy-phenyl)-3-(3-trifluoromethyl-phenyl)-urea in 100 ml DMF, 11.9 ml (0.17 Mol) dibromo-methane are added, followed by small portions of 19.3 g (59 mMol) of Cs₂CO₃. After 10 h at rt, the reaction mixture is concentrated *in vacuo* and the residue dissolved in EtOAc and a 10 % citric acid solution. The separated aqueous phase is extracted twice with EtOAc. The organic layers are washed with H₂O and brine, dried (Na₂SO₄) and concentrated. The dark brown oil is dissolved in CH₂Cl₂/MeOH, then 28 g of SiO₂ are added and the mixture is evaporated. The resulting powder is put on top of a chromatography column (SiO₂; hexane/EtOAc 3:1) and the title compound eluated with hexane/EtOAc 3:1 as an oil: MS: [M+1]⁺ = 415; TLC(hexane/EtOAc 1:1): R_{f} = 0.59; HPLC: ^{A}t*_{Ret}* = 17.2.

### Step 27.3: 6-Hydroxy-benzooxazole-3-carboxylic acid (3-trifluoromethyl-phenyl)-amide

As a solution in 60 ml THF, 1.67 g (4.0 mMol) 6-benzyloxy-benzooxazole-3-carboxylic acid (3-trifluoromethyl-phenyl)-amide are hydrogenated in the presence of 0.9 g Pd/C (10 %; Engelhard 5125). The catalyst is filtered off, washed with THF and the filtrate diluted with hexane. Partial concentration leads to the crystalline title compound, which is filtered off and washed with hexane: m.p.: 173-174 °C; MS: [M+1]⁺ = 325; HPLC: ^{A}t*_{Ret}* = 13.3.

### Example 28: 6-(6-Amino-pyrimidin-4-yloxy)-benzooxazole-3-carboxylic acid (3-trifluoromethyl-phenyl)-amide

150 mg (0.34 mMol) 6-(6-Chloro-pyrimidin-4-yloxy)-benzooxazole-3-carboxylic acid (3-trifluoromethyl-phenyl)-amide and 45 mg (0.69 mMol) NaN₃ in 2 ml DMF are stirred for 5 h at 60 °C giving 6-(6-azido-pyrimidin-4-yloxy)-benzooxazole-3-carboxylic acid (3-trifluoromethyl-phenyl)-amide (MS: [M+1]⁺ = 444). After cooling to rt, 35 mg Pd/C (10 %; Engelhard 4505) are added and the mixture is hydrogenated for 30 min. The catalyst is filtered off, washed with DMF and the filtrate concentrated *in vacuo.* Chromatography (Combi Flash; hexane/EtOAc 1:1 → EtOAc) gives the title compound: MS: [M+1]⁺ = 418; TLC(EtOAc: R_{f} = 0.25; HPLC: ^{A}t*_{Ret}* = 12.0.

### Example 29: 6-(6-Methylamino-pyrimidin-4-yloxy)-benzooxazole-3-carboxylic acid (3-trifluoromethyl-phenyl)-amide

In a sealed tube, 0.43 mMol of 6-(6-chloro-pyrimidin-4-yloxy)-benzooxazole-3-carboxylic acid (3-trifluoromethyl-phenyl)-amide in 2 ml THF and 2 ml methylamine (2 N in THF) are stirred for 16 h at rt. Concentration, chromatography (Combi Flash; CH₂Cl₂/MeOH 99:1 → 95:5) and crystallizatione from hexane gives the title compound: MS: [M+1]⁺ = 432; TLC(CH₂Cl₂/MeOH 9:1: R_{f} = 0.34; HPLC: ^{A}t*_{Ret}* = 12.5.

### Example 30: 6-(2-Amino-6-chloro-pyrimidin-4-yloxy)-benzooxazole-3-carboxylic acid (3-trifluoromethyl-phenyl)-amide

To an ice cooled solution of 300 mg (0.92 mMol) 6-hydroxy-benzooxazole-3-carboxylic acid (3-trifluoromethyl-phenyl)-amide (Step 27.3) and 151 mg (0.92 mMol) 2-amino-4,6-dichlorpyrimidine in 8 ml DMF, 600 mg (1.84 mMol) Cs₂CO₃ are added. Then the mixture is stirred for 4 h at rt and 1.5 h at 40°C and finally concentrated *in vacuo.* The residue is dissolved in EtOAc and H₂O /brine 1:1. The separated aqueous phase is extracted twice with EtOAc. The organic layers are washed with H₂O /brine 1:1 and brine, dried (Na₂SO₄) and concentrated. Chromatography (Combi Flash; CH₂Cl₂/MeOH 99:1 → 95:5) gives the title compound: MS: [M+1]⁺ = 452/454; TLC(CH₂Cl₂/MeOH 9:1): R_{f} = 0.51; HPLC: ^{A}t*_{Ret}* = 15.3.

### Example 31: 6-(2-Amino-pyrimidin-4-yloxy)-benzooxazole-3-carboxylic acid (3-trifluoromethyl-phenyl)-amide

Hydrogenation of 38 mg 6-(2-amino-6-chloro-pyrimidin-4-yloxy)-benzooxazole-3-carboxylic acid (3-trifluoromethyl-phenyl)-amide as described in Ex. 14 gives the title compound: MS: [M+1]⁺ = 418; HPLC: ^{A}t*_{Ret}* = 12.5.

### Example 31A: 6-(6-Chloro-pyrimidin-4-yloxy)-benzooxazole-3-carboxylic acid (4-fluoro-3-trifluoromethyl-phenyl)-amide

To a solution of 650 mg (1.90 mMol) 6-hydroxy-benzooxazole-3-carboxylic acid (4-fluoro-3-trifluoromethyl-phenyl)-amide in 15 ml acetone, 2.5 ml 1 N NaOH^{aq.} are dropped in followed by a solution of 325 mg (2.18 mMol) 4,6-dichlorpyrimidine in 5 ml acetone. After 75 min the reaction mixture is concentrated *in vacuo* and the residue dissolved in EtOAc, H₂O and brine. The aqueous phase is separated off and extracted twice with EtOAc. The organic layers are washed with 2 portions of H₂O/brine 1:1 and brine, dried (Na₂SO₄) and concentrated. Chromatography (Combi Flash; CH₂Cl₂/EtOAc 97:3 → 9:1) gives the title compound: MS: [M+1]⁺ = 455/457; TLC(CH₂Cl₂/EtOAc 9:1): R_{f} = 0.26; HPLC: ^{A}t*_{Ret}* = 16.3.

The starting material is prepared as follows:

### Step 31A.1: 1-(4-Benzyloxy-2-hydroxy-phenyl)-3-(4-fluoro-3-trifluoromethyl-phenyl)-urea

To a solution of 3.01 g (14 mMol) 2-amino-5-benzyloxy-phenol [preparation see: WO 03/045925; page 146] in 60 ml THF, a solution of 2.11 ml (14.8 mMol) 4-fluoro-3-trifluoromethyl-phenylisocyanate in 50 ml THF is added dropwise. After 1.5 h at rt, the reaction mixture is concentrated partially *in vacuo,* the residue is re-dissolved in H₂O and EtOAc. The organic layers are separated off and washed with H₂O and brine, dried (Na₂SO₄) and concentrated to a volume of = 80 ml. Addition of 50 ml hexane gives the crystalline title compound: m.p.: 188-191 °C; MS: [M+1]⁺ = 421; TLC(hexane/EtOAc 1:1): R_{f} = 0.31.

### Step 31A.2: 6-Benzyloxy-benzooxazole-3-carboxylic acid (4-fluoro-3-trifluoromethyl-phenyl)-amide

12.5 g (38.3 mMol) Cs₂CO₃ are added to a solution of 4.6 g (10.9 mMol) 1-(4-benzyloxy-2-hydroxyphenyl)-3-(4-fluoro-3-trifluoromethyl-phenyl)-urea in 100 ml DMF, followed by 7.6 ml (109 mMol) CH₂Br₂. This dark green mixture is stirred for 13 h at rt and 70 min at 50 °C. Then it is concentrated *in vacuo* and the residue re-dissolved in EtOAc and a 10 % citric acid solution. The separated aqueous phase is extracted twice with EtOAc. The organic layers are washed with 2 portions of H₂O and brine, dried (Na₂SO₄) and after addition of SiO₂ concentrated. The resulting powder is put on top of a chromatography column (SiO₂) and the title compound eluated with hexane/EtOAc 4:1: m.p.: 144-146 °C; MS: [M-1]= 431; TLC(hexane/EtOAc 1:1): R_{f} = 0.57.

### Step 31A.3: 6-Hydroxy-benzooxazole-3-carboxylic acid (4-fluoro-3-trifluoromethyl-phenyl)-amide

Hydrogenation of 1.69 g (3.91 mMol) 6-benzyloxy-benzooxazole-3-carboxylic acid (4-fluoro-3-trifluoromethyl-phenyl)-amide in 60 ml THF in the presence of 0.85 g Pd/C (10 %; Engelhard 5125), filtration, partial concentration of the filtrate, dilution with ≈ 15 ml hexane and cooling to 0 °C gives the crystalline title compound: m.p.: 171-172 °C; MS: [M-1]= 341; HPLC: ^{A}t*_{Ret}* = 13.7.

### Example 31B: 6-(6-Amino-pyrimidin-4-yloxy)-benzooxazole-3-carboxylic acid (4-fluoro-3-trifluoromethyl-phenyl)-amide

50 mg (0.11 mMol) 6-(6-Chloro-pyrimidin-4-yloxy)-benzooxazole-3-carboxylic acid (4-fluoro-3-trifluoromethyl-phenyl)-amide and 14.3 mg (0.22 mMol) NaN₃ in 1.5 ml DMF are stirred for 5 h at 60°C giving 6-(6-azido-pyrimidin-4-yloxy)-benzooxazole-3-carboxylic acid (4-fluoro3-trifluoromethyl-phenyl)-amide (MS: [M-1] = 460). After cooling to rt, 13 mg Pd/C (10 %; Engelhard 4505) are added and the mixture is hydrogenated over night. The catalyst is filtered off, washed with DMF and the filtrate concentrated *in vacuo.* Chromatography (Combi Flash; CH₂Cl₂/EtOAc 3:2 → EtOAc) gives the title compound: MS: [M+1]⁺= 436; TLC(EtOAc: R_{f} = 0.22; HPLC: ^{A}t*_{Ret}* = 12.5.

### Example 32: 6-(2-Amino-6-chloro-pyrimidin-4-yloxy)-naphthalene-1-carboxylic acid (3-trifluoromethyl-4-chloro-phenyl)-amide

To an ice cooled solution of 161 mg (0.51 mMol) 6-(2-amino-6-chloro-pyrimidin-4-yloxy)-naphthalene-1-carboxylic acid (Step 13.1) in 5 ml DMF, 75 µl (0.68 mMol) NMM and 127 µl (0.85 mMol) DEPC are added, followed by 0.30 g (1.5 mMol) of 2-chloro-5-amino-benzotrifluoride and a catalytic amount of DMAP. After 20 h stirring at rt, the solution is poured into H₂O and EtOAc, the aqueous phase separated off and extracted twice with EtOAc. The organic layers are washed with H₂O and brine, dried (Na₂SO₄) and concentrated. Column chromatography (SiO₂; EtOAc/hexane 2:8 → 3:7) and partial concentration of the product containing fractions leads to crystallization. Filtration and washing with hexane gives the title compound: MS: [M+1]⁺ = 493/495; HPLC: ^{A}t_{Ret} = 16.7.

### Example 32A: 6-(2-Amino-6-chloro-pyrimidin-4-yloxy)-benzooxazole-3-carboxylic acid (4-fluoro-3-trifluoromethyl-phenyl)-amide

can be obtained analogous to the route described in Example 32.

### Example 32B: 6-(2-Amino-pyrimidin-4-yloxy)-benzooxazole-3-carboxylic acid (4-fluoro-3-trifluoromethyl-phenyl)-amide

can be obtained analogous to the route described in Example 31.

### Example 33: 6-(2-Amino-pyrimidin-4-yloxy)-naphthalene-1-carboxylic acid (3-trifluoromethyl-4-chloro-phenyl)-amide

A solution of 100 mg (0.20 mmol) 6-(2-amino-6-chloro-pyrimidin-4-yloxy)-naphthalene-1-carboxylic acid (3-trifluoromethyl-4-chloro-phenyl)-amide in 10 ml THF and 31 µl (0.22 mMol) Et₃N is hydrogenated in the presence of 40 mg Pd/C (10 %; Engelhard 4505). The catalyst is filtered off, the filtrate concentrated and the residue diluted with EtOAc and H₂O. The aqueous layer is extracted twice with EtOAc. The organic layers are washed with H₂O and brine, dried (Na₂SO₄) and concentrated. Reversed phase MPLC gives the title compound: MS: [M+1]⁺= 459; HPLC: ^{A}**t***_{Ref}* = 13.3.

**Examples 34** (a)-(b): Via analogous routes the following derivatives can be obtained:

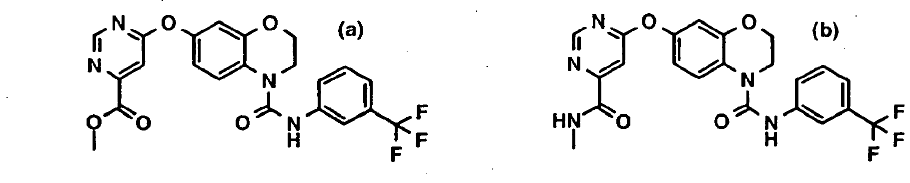

### Example 35: 6-(2-Amino-pyrimidin-4-yloxy)-naphthalene-1-carboxylic acid [4-(4-methyl-piperazin-1-yl-methyl)-3-trifluoromethyl-phenyl]-amide

To a solution of 50 mg (0.178 mMol) 6-(2-amino-pyrimidin-4-yloxy)-naphthalene-1-carboxylic acid (Step 15.1) and 53 mg (0.19 mmol) 4-(4-methyl-piperazin-1-yl-methyl)-3-trifluoromethyl-aniline in 2 ml DMF, 198 µl (1.42 mMol) Et₃N, 156 µl (0.267 mmol) propylphosphonic anhydride and 10 mg DMAP are added. After 3 days, the mixture is poured into H₂O and EtOAc, the aqueous phase separated off and extracted twice with EtOAc. The organic layers are washed with H₂O and brine, dried (Na₂SO₄) and concentrated. Reversed phase chromatography and crystallization from DIPE/hexane gives the title compound: MS: [M+1]⁺ = 537; TLC(EtOA_{c}/EtOH/NH₃^{aq} 80:20:1): R_{f} = 0.38; HPLC: ^{A}t*_{Ref}* = 9.2; Anal.: C,H,N,F.

### Example 36: 6-(2-Amino-pyrimidin-4-yloxy)-naphthalene-1-carboxylic acid [3,4-bis(trifluoromethyl)-phenyl]-amide

To a solution of 100 mg (0.356 mmol) 6-(2-amino-pyrimidin-4-yloxy)-naphthalene-1-carboxylic acid (Step 15.1) and 89.6 mg (0.39 mmol) 3,4-bis(trifluoromethyl)-aniline in 4 ml DMF, 396 µl (2.75 mmol) Et₃N, 312 µl (0.53 mmol) propylphosphonic anhydride and 10 mg DMAP are added. After 16 h, the mixture is poured into H₂O and EtOAc, the aqueous phase separated off and extracted twice with EtOAc. The organic layers are washed with H₂O and brine, dried (Na₂SO₄) and concentrated. Chromatography (Combi Flash; EtOAc/hexane 1:1 → EtOAc) gives the title compound: MS: [M+1 ]⁺ = 493; TLC(EtOAc): R_{f} = 0.54; HPLC: ^{A}t*_{Ret}* = 12.6.

### Example 37: The following compounds can be obtained analogously to Ex. 35 and 36.

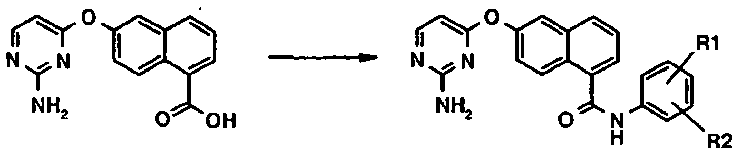

| | 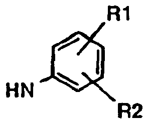 | TLC R_{f} | HPLC At*_{Ret}* [min] | m.p. [°C] | MS [M+1]⁺ | Anal. |
|---|---|---|---|---|---|---|
| a) | 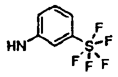 | 0.37¹⁾ | 13.4 | | 483 | |
| b) | 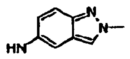 | | 10.2 | | 411 | |
| C) | 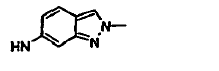 | | 10.1 | | 411 | |
| d) | 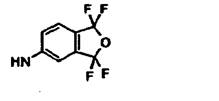 | 0.35¹⁾ | 13.6 | | 471 | |
| e) | 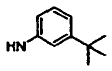 | 0.20²⁾ | 13.8 | 182-184 | 413 | C,H,N |
| f) | 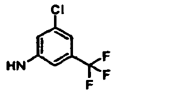 | 0.43³⁾ | 14.2 | 210 | 459/461 | |
| g) | 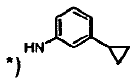 | 0.44⁴⁾ | 12.9 | 217 | 397 | C,H,N |
| h) | 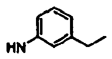 | 0.23⁵⁾ | 11.2 | 228 | 385 | C,H,N |
| i) | 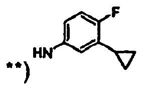 | 0.13⁵⁾ | 12.9 | 236-237 | 415 | |
| j) | 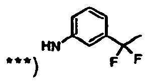 | 0.28⁵⁾ | 12.3 | 212 | 421 | C,H,N,F |
| k) | 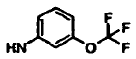 | 0.19⁵⁾ | 12.9 | 212-213 | 441 | |

| | | | | | | |
|---|---|---|---|---|---|---|
| ^{f)} TLC(EtOAc); ²⁾ TLC(hexane/EtOAc 1:3); ³⁾ TLC(EtOAc/CH₂Cl₂ 9:1); ⁴⁾ TLC(EtOAc/CH₂Cl₂ 4:1); ⁵⁾ TLC(CH₂Cl₂/EtOH 19:1) *) 3-cyclopropyl-aniline see: Tet. Lett. 43 (2002), 6987; **) 3-cyclopropyl-4-fluor-aniline prepared from 3-brom-4-fluor-aniline analogously to the procedure described in Tet. Lett. 43 (2002), 6987: TLC(hexane/EtOAc 4:1): R_{f} = 0.15; ***) 3-(α,α-difluorethyl)-aniline see: DE2130452 Ex. 12b | | | | | | |

### Example 38: 6-(6-Amino-pyrimidin-4-yloxy)-naphthalene-1-carboxylic acid (3-cyclopropyl-4-fluorophenyl)-amide

To a solution of 267 mg (0.95 mMol) 6-(6-amino-pyrimidin-4-yloxy)-naphthalene-1-carboxylic acid (Step 25.3) and 172 mg (1.14 mmol) 3-cyclopropyl-4-fluor-aniline in 5 ml DMF, 1.32 ml (9.5 mMol) Et₃N, 1.11 ml (1.9 mMol) propylphosphonic anhydride and 51 mg DMAP are added. After 1 h, the mixture is poured into H₂O and EtOAc, the aqueous phase separated off and extracted twice with EtOAc. The organic layers are washed with H₂O and brine, dried (Na₂SO₄) and concentrated. Chromatography (Combi Flash; CH₂Cl₂/ EtOH 99:1 → 19:1) gives the title compound: mp.: 256-257 °C; Anal.: C,H,N,F.

### Example 39: The following compounds can be obtained analogously to Ex. 38 (eventually longer reaction times necessary).

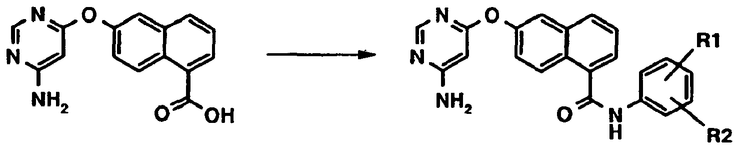

| | 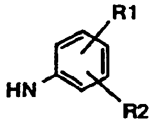 | TLC R_{f} | HPLC ^{A}t*_{Ret}* [min] | m.p. [°C] | MS [M+1]⁺ | Anal. |
|---|---|---|---|---|---|---|
| a) | 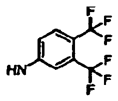 | 0.23^{f)} | 14.1 | | 493 | |
| b) | 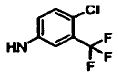 | 0.37²⁾ | 14.0 | 222-223 | 459/461 | |
| c) | 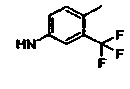 | 0.28²⁾ | 13.8 | 272 | 439 | |
| d) | 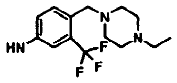 | 0.40³⁾ | 9.7 | | 551 | |
| e) | 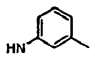 | 0.35²⁾ | 12.5 | 267 | 371 | C,H,N |
| f) | 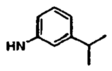 | 0.29²⁾ | 13.7 | 218-219 | 399 | C,H,N |
| g) | 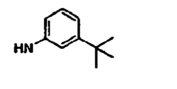 | 0.45²⁾ | 14.3 | 186-187 | 413 | C,H,N |
| h) | 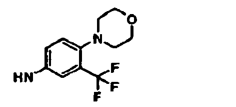 | 0.22³⁾ | 13.6 | | 510 | |
| i) | 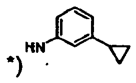 | 0.28⁴⁾ | 12.9 | 246 | 397 | C,H,N |
| j) | 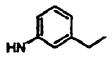 | 0.09⁵⁾ | 11.3 | 251-252 | 385 | C,H,N |
| k) | 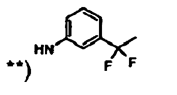 | 0.27⁴⁾ | 12.4 | 250-251 | 421 | C,H,N,F |
| l) | 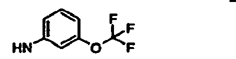 | 0.15⁵⁾ | 13.0 | 239-240 | 441 | C,H,N,F |

| | | | | | | |
|---|---|---|---|---|---|---|
| ¹⁾ TLC(EtOAclCH₂Cl₂ 1:1); ²⁾ TLC(EtOAc/CH₂Cl₂ 9:1); ³⁾ TLC(EtOAc/EtOH/NH₃^{aq} 80:20:1); ⁴⁾ TLC(EtOAc/CH₂Cl₂ 4:1); ⁵⁾ TLC(CH₂Cl₂/EtOH 19:1) *) 3-cyclopropyl-aniline see: Tet. Lett. 43 (2002), 6987; **) 3-(α,α-difluorethyl)-aniline see: DE2130452 Ex. 12b | | | | | | |

### Example 40: 6-(6-Methylamino-pyrimidin-4-yloxy)-naphthalene-1-carboxylic acid (4-morpholin-4-yl-3-trifluoromethyl-phenyl)-amide

To a solution of 295.3 mg (1.00 mmol) 6-(6-methylamino-pyrimidin-4-yloxy)-naphthalene-1-carboxylic acid and 295 mg (1.2 mMol) 4-morpholin-4-yl-3-trifluoromethyl-aniline in 5 ml DMF, 1.39 ml (10 mMol) Et₃N, 1.17 ml (2.0 mmol) propylphosphonic anhydride and 50 mg (0.4 mmol) DMAP are added. After 30 min, the mixture is poured into H₂O and EtOAc, the aqueous phase separated off and extracted 3 times with EtOAc. The organic layers are washed 3 times with H₂O and brine, dried (Na₂SO₄) and concentrated. Column chromatography (SiO₂; CH₂Cl₂/ EtOH 95:5) gives the title compound: mp.: 156-157 °C; MS: [M+1]⁺ = 524.

The starting material is prepared as follows:

### Step 40.1: 6-(6-Methylamino-pyrimidin-4-yloxy)-naphthalene-1-carboxylic acid

To a solution of 8.1 g (27 mmol) 6-(6-chloro-pyrimidin-4-yloxy)-naphthalene-1-carboxylic acid (Step 25.1) in 100 ml THF, 200 ml of a 2 M solution of methylamin in THF are added. After 3 days at rt, the suspension is concentrated partially *in vacuo*, the solid filtered off and washed with ether. The crude product dissolved in 300 ml H₂O is treated with char coal and filtered. The filtrate is acidified to pH 1 with 1 N HCl and the formed precipitate filtered off, washed with H₂O and dried. Repeated stirring in ether followed by filtration gives the title compound: m.p.: 267-268 °C; MS: [M+1]⁺= 296.

### Example 41: The following compounds can be obtained analogously to Ex. 40.

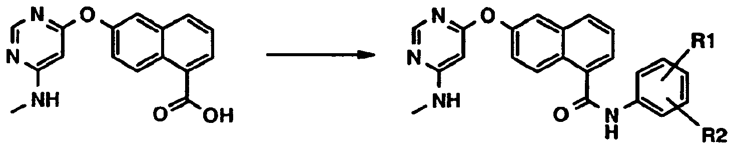

| | 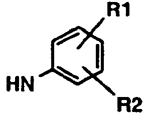 | TLC R_{f} | HPLC ^{A}t*_{Ret}* [min] | m.p. [°C] | MS [M+1]⁺ | Anal. |
|---|---|---|---|---|---|---|
| a) | 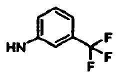 | 0.29¹⁾ | 11.7 | 152-153 | 439 | |
| b) | 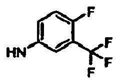 | 0.26¹⁾ | 11.9 | 154-155 | 457 | |
| c) | 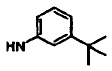 | 0.22¹⁾ | 12.5 | 102-104 | 427 | |

| | 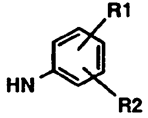 | TLC R*ₜ* | HPLC ^{A}t*_{Ret}* [min] | m.p. [°C] | MS [M+1]⁺ | Anal. |
|---|---|---|---|---|---|---|
| d) | 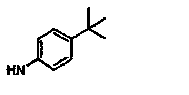 | 0.30¹⁾ | 12.6 | 141-142 | 427 | |
| e) | 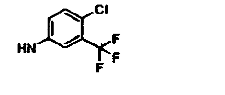 | 0.22¹⁾ | 12.5 | 148-149 | 473/475 | |
| f) | 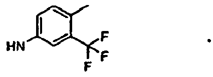 | 0.22¹⁾ | 12.2 | 238-239 | 453 | |
| g) | 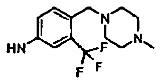 | 0.23²⁾ | 8.5 | | 551 | |
| h) | 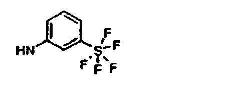 | 0.22¹⁾ | 12.3 | 211-212 | 497 | |
| i) | 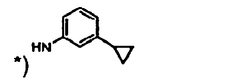 | 0.51³⁾ | 12.7 | 144-145 | 411 | C,H,N |
| j) | 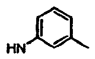 | 0.35⁴⁾ | 12.6 | 164 | 385 | |
| k) | 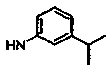 | 0.29⁴⁾ | 12.0 | 128-129 | 413 | C,H,N |
| l) | 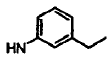 | 0.25⁴⁾ | 11.6 | 160-161 | 399 | C,H,N |

| | | | | | | |
|---|---|---|---|---|---|---|
| ¹⁾ TLC(CH₂Cl₂/MeOH 19:1); ²⁾ TLC(CH₂Cl₂/MₑOH/NH₃^{aq} 90:10:1); ³⁾ TLC(CH₂Cl₂/acetone 2:1); ⁴⁾ TLC(CH₂Cl₂/EtOH 19:1) *) 3-cyclopropyl-aniline see: Tet. Lett. 43 (2002), 6987 | | | | | | |

### Example 42: 6-(6-Chloro-pyrimidin-4-yloxy)-naphthalene-1-carboxylic acid (3-trifluoromethyl-phenyl)-amide

To 8.38 g (27.9 mMol) 6-(6-chloro-pyrimidin-4-yloxy)-naphthalene-1-carboxylic acid (Step 25.1), 31 ml (223 mMol) Et₃N and 1 g (8 mmol) DMAP in 100 ml ice-cooled DMF, 24.4 ml (41.8 mMol) propylphosphonic anhydride in 25 ml DMF are added dropwise during 20 min, followed by a solution of 3.83 ml (30.6 mmol) 3-trifluormethyl-aniline in 25 ml DMF. After 90 min at rt, the mixture is concentrated partially *in vacuo* at 40 °C. The resulting residue is diluted with H₂O and EtOAc, the aqueous phase separated off and extracted twice with EtOAc. The organic layers are washed with H₂O and brine, dried (Na₂SO₄) and concentrated. Column chromatography (SiO₂; CH₂Cl₂/ THF 99:1 → CH₂Cl₂/ THF/Et₃N 98:1:1) gives the title compound: MS: [M+1]⁺ = 444; TLC(CH₂Cl₂/ THF 99:1): R_{f} = 0.17; HPLC: ^{A}t*_{Ret}* = 16.8.

### Example 43: 6-(6-Cyano-pyrimidin-4-yloxy)-naphthalene-1-carboxylic acid (3-trifluoromethyl-phenyl)-amide

To a solution of 3.3 g (7.4 mMol) 6-(6-chloro-pyrimidin-4-yloxy)-naphthalene-1-carboxylic acid (3-trifluoromethyl-phenyl)-amide in 80 ml DMSO and 20 ml H₂O, 0.42 g (3.7 mmol) 1,4-diazobicyclo[2,2,2]octan and 1.0 g (15 mmol) KCN are added. The mixture is stirred for 30 min at 55 °C and then diluted with H₂O, brine and EtOAc. The aqueous phase is separated off and extracted twice with EtOAc. The organic layers are washed with H₂O and brine, dried (MgSO₄) and concentrated. The residue is re-dissolved in CH₂Cl₂ and after addition of SiO₂ concentrated again. The resulting powder is put on top of a SiO₂-column. Eluation with CH₂Cl₂/ EtOAc 98:2 → 93:7, partial concentration and crystallization by adding hexane gives the title compound: m.p.: 167 °C; MS: [M+1]⁺= 435; Anal.: C,H,N,F.

### Example 44: 6-(6-Aminomethyl-pyrimidin-4-yloxy)-naphthalene-1-carboxylic acid (3-trifluoromethyl-phenyl)-amide

830 mg (1.91 mmol) 6-(6-cyano-pyrimidin-4-yloxy)-naphthalene-1-carboxylic acid (3-trifluoromethyl-phenyl)-amide in 40 ml THF and 7.3 ml NH₃^{aq} 25 % are hydrogenated in presence of 0.7 g Raney-Nickel (Degussa B 113W). The aqueous layer is separeted off from the reaction mixture and extracted with EtOAc. The organic phases are dried (Na₂SO₄) and concentrated. This residue is re-dissolved in EtOAc/acetone and after addition of SiO₂ concentrated again. The resulting powder is put on top of a SiO₂-column. Eluation with EtOAc/acetone/Et₃N 80:20:0 → 80:20:1 → 60:40:1, partial concentration and crystallization by adding hexane gives the title compound: MS: [M+1]⁺ = 439; TLC(EtOActacetone 2:1 + NH₃^{aq}): R_{f} = 0.35; HPLC: ^{A}t*_{Ret}* = 12.5.

### Example 45: {6-[5-(3-Trifluoromethyl-phenylcarbamoyl)-naphthalen-2-yloxy]-pyrimidin-4-ylmethyl}-carbamic acid methylester

A mixture of 100 mg (0.23 mmol) 6-(6-aminomethyl-pyrimidin-4-yloxy)-naphthalene-1-carboxylic acid (3-trifluoromethyl-phenyl)-amide, 175 µl (1.2 mmol) Et₃N, 60 µl (0.77 mmol) methyl chloroformate and a trace of DMAP in 3 ml THF is stirred over night at rt. The mixture is poured into H₂O and EtOAc, the aqueous phase separated off and extracted twice with EtOAc. The organic layers are washed with H₂O and brine, dried (Na₂SO₄) and concentrated. Chromatography (Combi Flash; hexane/ EtOAc 7:3 → 1:1) gives the title compound: MS: [M+1]⁺ = 497; TLC(EtOAc/hexane 2:1): R*ₜ* = 0.35; HPLC: ^{A}t*_{Ret}* = 14.9.

### Example 46: 6[5-(3-Trifluoromethyl-phenylcarbamoyl)-naphthalen-2-yloxy]-pyrimidine-4-carboxylic acid ethyl ester

A mixture of 5.36 g (12.07 mMol) 6-(6-chloro-pyrimidin-4-yloxy)-naphthalene-1-carboxylic acid (3-trifluoromethyl-phenyl)-amide (Ex. 43), 3.4 ml (24 mmol) Et₃N and 1.35 g PdCl₂[P(C₆H₅)₃]₂ in 80 ml ethanol is prepared under a CO-atmosphere of 120 bar in an autoclave. Then it is heated for 30 h at 110 °C. After cooling to rt, the mixture is diluted with EtOH and filtered. The residue is washed vigorously with EtOH and the filtrate concentrated. Column chromatography (SiO₂; CH₂Cl₂/EtOAc 19:1 → 9:1) and partial concentration leads to the crystalline title compound: m.p.: 194 °C; Anal.: C,H,N,F.

### Example 47: 6-[5-(3-Trifluoromethyl-phenylcarbamoyl)-naphthalen-2-yloxy]-pyrimidine-4-carboxylic acid

To 0.36 g (0.75 mmol) 6-[5-(3-trifluoromethyl-phenylcarbamoyl)-naphthalen-2-yloxy]-pyrimidine-4-carboxylic acid ethyl ester dissolved in 9 ml THF, 1.15 ml 1 M LiOH in H₂O are added. After 2 h at rt, the mixture is concentrated *in vacuo.* The residue is dissolved in a mixture of EtOAc, H₂O and 1 N NaOH-solution. The aqueous layer is separated off and extracted with EtOAc. The organic phases are washed with a diluted NaOH-solution and discarded. The combined aqueous layers are acidified with 2 N HCl and extracted twice with EtOAc. These organic phases are washed with brine, dried (Na₂SO₄) and concentrated. Crystallization from DIPE gives the title compound: MS: [M+1]⁺= 454.

### Example 48: 6-[5-(3-Trifluoromethyl-phenylcarbamoyl)-naphthalen-2-yloxy]-pyrimidine-4-carboxylic acid dimethylamide

To 200 mg (0.435 mmol) of the lithium-salt of 6-[5-(3-trifluoromethyl-phenylcarbamoyl)-naphthalen-2-yloxyl-pyrimidine-4-carboxylic acid, 612 µl (4.4 mmol) Et₃N, 24 mg (0.2 mmol) DMAP and 24 mg (0.53 mmol) Me₂NH in 7 ml DMF, 516 µl (0.88 mmol) propylphosphonic anhydride are added. After 2.5 h at rt, the mixture is diluted with H₂O and EtOAc, the aqueous phase separated off and extracted twice with EtOAc. The organic layers are washed with H₂O and brine, dried (Na₂SO₄) and concentrated. Chromatography (Combi Flash; hexane/EtOAc 3:2 → 2:3) gives the title compound: MS: [M+1]⁺ = 481; TLC(CH₂Cl₂/EtOAc 1:1): R*_{f}* = 0.18; HPLC: ^{A}t*_{Ret}* = 15.8.

### Example 49: 6-[5-(3-Trifluoromethyl-phenylcarbamoyl)-naphthalen-2-yloxy]-pyrimidine-4-carboxylic acid amide

To a solution of 207 mg (0.43 mMol) 6-[5-(3-trifluoromethyl-phenylcarbamoyl)-naphthalen-2-yloxy]-pyrimidine-4-carboxylic acid ethyl ester (Ex. 47) in 12 ml CH₃CN and 5 ml THF, 5 ml of NH₃ (25 % in H₂O) are added. This mixture is stirred in a sealed vessel for 7 h at rt, while a precipitation is formed. Filtration and washing with CH₃CN yields the title compound: MS: [M-1]=451; HPLC:^{A}t*_{Ret} =* 15.2; Anal.: C,H,N,F.

### Example 50: 6-[5-(3-Trifluoromethyl-phenylcarbamoyl)-naphthalen-2-yloxy]-pyrimidine-4-carboxylic acid methylamide

To 200 mg (0.435 mmol) of the lithium-salt of 6-[5-(3-trifluoromethyl-phenylcarbamoyl)-naphthalen-2-yloxy]-pyrimidine-4-carboxylic acid, 609 µl (4.35 mmol) Et₃N, 24 mg (0.2 mMol) DMAP and 260 µl (2 M in THF; 0.52 mMol) MeNH₂ in 7 ml DMF, 510 µl (0.87 mMol) propylphosphonic anhydride are added. After 1 h at rt, the mixture is diluted with H₂O and EtOAc, the aqueous phase separated off and extracted twice with EtOAc. The organic layers are washed twice with H₂O and brine, dried (Na₂SO₄) and concentrated. Chromatography (Combi Flash; CH₂Cl₂/EtOAc 4:1) gives the title compound: MS: [M+1]⁺= 467; TLC(EtOAc): R_{f} = 0.55; HPLC: ^{A}t*_{Ret}* = 15.7.

### Example 51: 6-[5-(3-Trifluoromethyl-phenylcarbamoyl)-naphthalen-2-yloxy]-pyrimidine-4-carboxylic acid isopropylamide

To a solution of 197 mg (0.41 mMol) 6-[5-(3-trifluoromethyl-phenylcarbamoyl)-naphthalen-2-yloxy]-pyrimidine-4-carboxylic acid ethyl ester (Ex. 47) in 10 ml THF, 7 µl (0.4 mMol) H₂O and 0.7 ml (8 mMol) isopropylamine are added. This mixture is stirred in a sealed vessel for 5 days at 45 °C. Reversed phase chromatography gives the title compound: m.p.: 205-208 °C; MS: [M+1]⁺= 495; Anal.: C,H,N,F.

### Example 52: 6-(6-Hydroxymethyl-pyrimidin-4-yloxy)-naphthalene-1-carboxylic acid (3-trifluoromethyl-phenyl)-amide

To 1.16 g (2.41 mMol) 6-[5-(3-trifluoromethyl-phenylcarbamoyl)-naphthalen-2-yloxy]-pyrimidine-4-carboxylic acid ethyl ester (Ex. 47) in 40 ml tert-butanol, 218 mg (5.76 mMol) NaBH₄ are added and the mixture is stirred for 1 h at 70 °C. Then additional 109 mg NaBH₄ are added and stirring is continued for another 1 h at 80 °C. The reaction mixture is concentrated *in vacuo* and the residue re-dissolved in EtOAc and sat. NaHCO₃. The separated aqueous phase is extracted twice with EtOAc. The organic layers are washed with sat. NaHCO₃ and brine, dried (Na₂SO₄) and concentrated after addition of SiO₂. This powder is put on top of a SiO₂-column (CH₂Cl₂/EtOAc 2:1 → 1:1 → EtOAc): At first the side product 6-(6-methyl-pyrimidin-4-yloxy)-naphthalene-1-carboxylic acid (3-trifluoromethyl-phenyl)-amide is eluated {MS: [M+1]⁺ = 424; TLC(CH₂Cl₂/EtOAc 1:1): R*_{f}* = 0.33; HPLC: ^{A}t*_{Ret}* = 15.1}, followed by the title compound: m.p.: 183-184 °C; MS: [M+1]⁺ = 440; TLC(CH₂Cl₂/EtOAc 1:1): R*_{f}* = 0.13; HPLC: ^{A}t*_{Ret}* = 14.3; Anal.: C,H,N,F.

### Example 53: 6-(6-Chloromethyl-pyrimidin-4-yloxy)-naphthalene-1-carboxylic acid (3-trifluoromethyl-phenyl)-amide

1.0 ml (13.9 mmol) SOCl₂ is added via syringe to a solution of 610 mg (1.39 mMol) 6-(6-hydroxymethyl-pyrimidin-4-yloxy)-naphthalene-1-carboxylic acid (3-trifluoromethyl-phenyl)-amide in 50 ml CH₃CN and 10 ml THF. After 15 min at rt, the solution is poured into 75 ml sat. NaHCO₃ and 75 ml H₂O and then concentrated partially *in vacuo.* The formed precipitate is filtered off and washed with H₂O, yielding the title compound: m.p.: 178-181 °C; MS: [M+1]⁺= 458/460; Anal.: C,H,N,F.

### Example 54: 6-(6-Methylaminomethyl-pyrimidin-4-yloxy)-naphthalene-1-carboxylic acid (3-trifluoromethyl-phenyl)-amide

In a sealed tube a mixture of 150 mg (0.328 mMol) 6-(6-chloromethyl-pyrimidin-4-yloxy)-naphhalene-1-carboxylic acid (3-trifluoromethyl-phenyl)-amide, 10 mg (0.067 mMol) Nal and 0.8 ml methylamine (2 M in THF) in 8 ml THF is stirred for 2.5 h at 80 °C. The reaction mixture is concentrated *in vacuo* and the residue re-dissolved in EtOAc and sat. NaHCO₃/H₂O 1:1. The separated aqueous phase is extracted twice with EtOAc. The organic layers are washed with sat. NaHCO₃/H₂O 1:1 and brine, dried (Na₂SO₄) and concentrated. Chromatography (Combi Flash; EtOAc/(THF + 2 % Et₃N) 19:1 → 1:1) gives the title **compound: m.p.: 141-143 °C; MS: [M+1]⁺= 453; Anal.: C,H,N.**

### Example 55: 6-(6-Dimethylaminomethyl-pyrimidin-4-yloxy)-naphthalene-1-carboxylic acid (3-trifluoromethyl-phenyl)-amide

In a sealed tube a mixture of 150 mg (0.328 mMol) 6-(6-chloromethyl-pyrimidin-4-yloxy)-naphthalene-1-carboxylic acid (3-trifluoromethyl-phenyl)-amide, 134 mg (1.64 mMol) dimethylamine hydrochloride, 10 mg (0.067 mMol) Nal, 687 µl (4.9 mMol) Et₃N and 8 ml THF is stirred for 4.25 h at 80 °C. Work up analogously as described for Ex. 54 gives the title compound: m.p.: 180-181 °C; MS: [M+1]⁺= 467; Anal.: C,H,N,F.

### Example 56: {6-[5-(4-Fluoro-3-trifluoromethyl-phenylcarbamoyl)-naphthalen-2-ylsulfanyl]-pyrimidin-4-yl}-carbamic acid tert-butyl ester

To an ice-cooled solution of 127 mg (0.32 mMol) 6-(6-tert-butoxycarbonylamino-pyrimidin-4-ylsulfanyl)-naphthalene-1-carboxylic acid (Step 57.2) and 86 mg (0.48 mMol) 4-fluoro-3-trifluoromethyl-aniline in 3 ml DMF, 446 µl (3.2 mMol) Et₃N, 0.37 ml (0.63 mMol) propylphosphonic anhydride and 4 mg DMAP are added. After 2 h at rt, the mixture is poured into H₂O and EtOAc, the aqueous phase separated off and extracted twice with EtOAc. The organic layers are washed twice with H₂O and brine, dried (Na₂SO₄) and concentrated. Recrystallization from boiling CH₃CN gives the title compound: MS: [M+1]⁺= 559; TLC(hexane/EtOAc 1:1): R_{f} = 0.47; Anal.: C,H,N,F.

The starting material is prepared as follows:

### Step 56.1: 6-(6-Chloro-pyrimidin-4-ylsulfanyl)-narphthalene-1-carboxylic acid

894 mg (6.0 mMol) 4,6-Dichloro-pyrimidine and 1021 mg (5.0 mMol) 6-mercapto-naphthalene-1-carboxylic acid [preparation described in J. Med. Chem. 32 (1989), 2493; purification by chromatography (Combi Flash; hexane/EtOAc 7:3 → 3:2): m.p.: 212-213 °C] are suspended in 16 ml acetone. Then 16 ml of 1 N NaOH in H₂O are added. After 5 min at rt, the resulting solution is poured into 200 ml of 1 N HCl in H₂O and extracted 3 times with EtOAc. The organic layers are washed with H₂O and brine, dried (Na₂SO₄) and concentrated. Column chromatography (SiO₂; CH₂Cl₂/EtOAc 1:2) and crystallization from EtOAc/hexane yields the title compound: m.p.: 209 °C; MS: [M-1]= 317.

### Step 56.2: 6-(6-tert-Butoxycarbonylamino-pyrimidin-4-ylsulfanyl)-naphthalene-1-carboxylic acid

A suspension of 195 mg (0.61 mMol) 6-(6-chloro-pyrimidin-4-ylsulfanyl)-naphthalene-1-carboxylic acid and 495 mg (1.52 mMol) Cs₂CO₃ in 5 ml dioxane is degassed repeatedly by evaporation and flushing with N₂. Then 10.9 mg (0.019 mMol) 4,5-bis(diphenylphosphino)-9,9-dimethylxanthene, 5.6 mg (0.006 mMol) tris(dibenzylidenaceton)dipalladium (0) CHCl₃ adduct and 85.8 mg (0.73 mMol) carbamic acid tert-butyl ester are added successively. After 4 h stirring at 110 °C, another 10.9 mg 4,5-bis(diphenylphosphino)-9,9-dimethylxanthene and 5.6 mg tris(dibenzylidenaceton)dipalladium (0) CHCl₃ adduct are added and stirring is continued for 5 h. Then the cooled mixture is poured into EtOAc and 5 % citric acid in H₂O, the aqueous layer separated off and extracted twice with EtOAc. The organic layers are washed with H₂O and brine, dried (Na₂SO₄) and concentrated. Chromatography (Combi Flash; CH₂Cl₂/EtOAc 99:1 → CH₂Cl₂/(EtOAc + 1 % HOAc) 4:1) gives the title compound: m.p.: 208-209 °C; MS: [M-1] = 396.

### Example 57: 6-(6-Amino-pyrimidin-4-ylsulfanyl)-naphthalene-1-carboxylic acid (4-fluoro-3-trifluoromethyl-phenyl)-amide

To a solution of 80 mg (0.14 mMol) {6-[5-(4-fluoro-3-trifluoromethyl-phenylcarbamoyl)-naphthalen-2-ylsulfanyl]-pyrimidin-4-yl}-carbamic acid tert-butyl ester in 2 ml dioxane are added 2 ml 2 M HCl in dioxane. After 9 h at rt, the solution is diluted with EtOAc and sat. NaHCO₃/H₂O 1:1. The aqueous phase is separated off and extracted twice with EtOAc. The organic layers are washed with H₂O and brine, dried (Na₂SO₄) and concentrated. Chromatography (Combi Flash; CH₂Cl₂/EtOAc 4:1 → 1:4) and crystallization from EtOAc/hexane gives the title compound: m.p.: 221 °C; MS: [M+1]⁺= 459; TLC(CH₂Cl₂/EtOAc 1:2): R_{f} = 0.25.

### Example 58: 6-(6-Amino-pyrimidin-4-ylsulfanyl)-naphthalene-1-carboxylic acid (3-trifluoromethyl-phenyl)-amide

To 1.65 g (5.55 mMol) 6-(amino-pyrimidin-4-ylsulfanyl)-naphthalene-1-carboxylic acid, 832 µl (6.66 mmol) 3-trifluoromethyl-aniline, 7.87 ml (56.5 mmol) Et₃N and 291 mg (2.38 mmol) DMAP in 30 ml DMF, 6.8 ml (11.6 mmol) propylphosphonic anhydride are added dropwise. After 1 h at rt, the mixture is poured into H₂O and EtOAc, the aqueous phase separated off and extracted twice with EtOAc. The organic layers are washed with H₂O and brine, dried (Na₂SO₄) and concentrated after addition of SiO₂. The resulting powder is put on top of a SiO₂ column (CH₂Cl₂/EtOAc 9:1) and the title compound eluted with CH₂Cl₂/EtOAc 4:1 → 3:7: m.p.: 205 °C; MS: [M+1]⁺ = 441; TLC(CH₂Cl₂/EtOAc 1:1): R_{f} =0.16; HPLC: ^{A}t*_{Ret}* = 13.0.

### The starting material is prepared as follows:

### Step 58.1: 6-(6-Amino-pyrimidin-4-ylsulfanyl)-naphthalene-1-carboxylic acid

A mixture of 1.00 g (7.72 mmol) 4-amino-6-chloro-pyrimidine, 1.74 g (8.5 mmol) 6-mercapto-naphthalene-1-carboxylic acid, 10.8 g K₃PO₄ and 35 mg (0.23 mmol) Nal in 50 ml NMP is stirred for 2.5 h at 110 °C. Then the mixture is poured into 230 ml of a 5 % solution of citric acid in H₂O, the crude product filtered off and washed with H₂O. Stirring in boiling isopropanol and filtration gives the title compound: m.p.: 264-266 °C; MS: [M+1]⁺ = 298.

### Example 59: 6-(6-Amino-pyrimidine-4-sulfinyl)-naphthalene-1-carboxylic acid (3-trifluoromethyl-phenyl)-amide

### Example 60: 6-(6-Amino-pyrimidine-4-sulfonyl)-naphthalene-1-carboxylic acid (3-trifluoromethyl-phenyl)-amide

### Example 61: 6-(6-Amino-pyrimidin-4-ylsulfanyl)-naphthalene-1-carboxylic acid (3-trifluoromethoxyphenyl)-amide

To 95 mg (0.32 mMol)6-(amino-pyrimidin-4-ylsulfanyl)-naphthalene-1-carboxylic acid, 85 mg (0.48 mMol) 3-trifluoromethoxy-aniline, 446 µl (3.2 mmol) Et₃N and 4 mg (0.03 mMol) DMAP in 3 ml DMF, 0.37 ml (0.63 mmol) propylphosphonic anhydride are added. After 3 h at rt, the reaction mixture is worked up as described for Ex. 58, yielding the title compound: m.p.: 197-199 °C; MS: [M+1]⁺ = 457.

### Example 62: 6-(Pyridin-4-yl-methyl)-naphthalene-1-carboxylic acid (4-fluoro-3-trifluoromethyl-phenyl)-amide

A mixture of 22.4 mg (0.10 mmol) Pd(O₂CCH₃)₂, 52.6 mg (0.20 mmol) triphenylphosphine, 151 mg (0.33 mmol) 6-(4,4,5,5-tetramethyl-[1,3,2]dioxaborolan-2-yl)-naphthalene-1-carboxylic acid (4-fluoro-3-trifluoromethyl-phenyl)-amide and 229.3 mg (1.08 mmol) K₃PO₄ in 3 ml toluene is degassed repeatedly by evaporation and flushing with N₂. Then 59 mg (0.36 mmol) 4-chloromethyl-pyridine hydrochloride are added and the mixture is stirred at 80 °C for 20 h, when additional 18.5 mg (0.082 mmol) Pd(O₂CCH₃)₂ and 43.1 mg (0.164 mmol) triphenylphosphine are added. After 3 h at 110 °C the reaction mixture is poured into H₂O and EtOAc. The aqueous phase is separated off and extracted twice with EtOAc. The organic layers are washed with H₂O and brine, dried (Na₂SO₄) and concentrated. Chromatography [Combi Flash; CH₂Cl₂ → CH₂Cl₂/(MeOH + 10 % NH₃^{aq}) 9:1] gives the title compound: MS: [M+1]⁺ = 425; HPLC: ^{A}t*_{Ret}* = 12.7.

The starting material is prepared as follows:

### Step 62.1: Trifluoro-methanesulfonic acid 5-(4-fluoro-3-trifluoromethyl-phenylcarbamoyl)-naphthalen-2-yl ester

0.94 g (5.0 mmol) 6-hydroxy-naphthalene-1-carboxylic acid in a mixture of 50 ml CH₂Cl₂, 25 ml dioxane and 2.4 ml (30 mMol) pyridine is cooled to -78 °C. Then a solution of 1.98 ml (12 mMol) trifluoro-methanesulfonic acid anhydride in 5 ml CH₂Cl₂ is added dropwise and the mixture is warmed up to rt. After 5 h, 1.43 g (8.0 mMol) 4-fluoro-3-trifluoro-aniline dissolved in 5 ml CH₂Cl₂ are added and stirring is continued over night. A formed precipitate is filtered off and discarded and the filtrate diluted with EtOAc and sat. NaHCO₃/H₂O 1:1. The aqueous phase is separated and extracted twice with EtOAc. The organic layers are washed with H₂O and brine, dried (Na₂SO₄) and concentrated. Column chromatography (SiO₂; toluene/EtOAc 199:1 → 9:1) and partial concentration leads to the crystalline title compound: m.p.: 171-172 °C; MS: [M-1] = 480.

### Step 62.2: 6-(4,4,5,5-Tetramethyl-[1,3,2]dioxaborolan-2-yl)-naphthalene-1-carboxylic acid (4-fluoro-3-trifluoromethyl-phenyl)-amide

A mixture of 1.203 g (2.5 mMol) trifluoro-methanesulfonic acid 5-(4-fluoro-3-trifluoromethyl-phenylcarbamoyl)-naphthalen-2-yl ester, 762 mg (3.0 mMol) 4,4,5,5,4',4',5',5'-octamethyl-[2,2']bi[[1,3,2]dioxaborolanyl] and 736 mg (7.5 mMol) potassium acetate in 12 ml DMF is degassed repeatedly by evaporation and flushing with N₂. Then 100 mg (0.12 mMol) of the dichloromethane complex of [1,1'-bis(diphenylphosphino)ferrocene]dichloropalladium(II) are added and the mixture is stirred for 3 h at 80 °C. The mixture is diluted with EtOAc and H₂O, the aqueous phase separated off and extracted twice with EtOAc. The organic layers are washed twice with H₂O and brine, dried (Na₂SO₄) and concentrated. Chromatography (Combi Flash: CH₂Cl₂/hexane 1:1; crude product added as solution in CH₂Cl₂ onto preconditioned column and rapidly eluated with CH₂Cl₂/hexane 1:1 → CH₂Cl₂) gives the title compound: MS: [M+1]⁺ = 460; TLC(CH₂Cl₂): R_{f} = 0.30; HPLC: ^{A}t*_{Ret}* = 18.3.

### Example 63: 6-(2-Methyl-pyridin-4-yloxy)-naphthalene-1-carboxylic acid (3-trifluoromethyl-phenyl)-amide

To a solution of 331 mg (1.0 mmol) 6-hydroxy-naphthalene-1-carboxylic acid (3-trifluoromethyl-phenyl)-amide in 0.375 mL DMF and 1.37 mL DMPU (dimethyl-propylene urea) is added 4-chloro-2-methyl-pyridine (180 mg, 1.1 mmol) followed by potassium t-butoxide (336 mg, 3 mmol). The dark viscous mixture is stirred 3 days at 100 °C. After cooling to rt the mixture is diluted with ethyl acetate, washed with brine, dried over sodium sulfate and evaporated. The remaining DMPU and DMF is distilled off in a Kugelrohr apparatus (100 °C, under vacuum). The residue is purified by flash-chromatography on a silica gel column and eluting with ethyl acetate. Concentration of the pure samples leads to crystalline title compound: MS: [M+1]⁺ = 423; TLC(ethyl acetate): R_{f} = 0.5; HPLC: ^{E}t*_{Ret}* = 3.42.

### The starting material is prepared as follows:

### Step 63.1: 6-hydroxy-naphthalene-1-carboxylic acid (3-trifluoromethyl-phenyl)-amide

To an ice-cooled solution of 17.2 g (65 mmol) 6-hydroxy-naphthalene-1-carboxylic and 9.9 g (65 mmol) HOBT in 260 mL of THF is added dropwise a solution of 14.4 g (70.2 mmol) DCC in 45 mL of THF over 20 minutes. The reaction mixture is stirred 15 minutes at 0 °C and then 1 hour at rt. The solid formed is removed by filtration and washed with a small amount of cold THF. The filtrate is evaporated and the residue triturated with ethyl acetate/hexanes 4:6. The crystalline activated ester was filtered off and dried. 9.15 g of this active ester (30 mmol) are dissolved in 80 mL of THF and treated with 3.5 mL (30 mmol) 3-trifluoromethyl-anilin. After refluxing for 24 h another 0.35 mL (3 mmol) 3-trifluoromethyl-anilin is added and the mixture refluxed for another 24 h. The solvent is removed and the residue subjected to a flash-chromatography on silica gel using ethyl acetate/hexanes 4:6. Pure fractions are evaporated and the residue is triturated with petrol ether. The crystalline title compound is filtered and dried: MS: [M+1]⁺ = 332; TLC(ethyl acetate/hexanes 4:6): R_{f} = 0.53; HPLC: ^{E}t*_{Ret}* = 3.8.

### Example 64: 4-[5-(3-Trifluoromethyl-phenylcarbamoyl)-naphthalen-2-yloxy]-pyridine-2-carboxylic acid butyl ester

A mixture of 993 mg (3.0 mmol) 6-hydroxy-naphthalene-1-carboxylic acid (3-trifluoromethyl-phenyl)-amide, 10 mL *n*-BuOH, 1.286 g (7.5 mmol) 4-chloro-pyridine-2-carboxylic acid methyl ester, 0.5 mL (3.6 mmol) triethylamin and 10 mg 4-edimethylamino-pyridine is heated under reflux for 5 days. After cooling the mixture is evaporated, diluted with ethyl acetate and washed with diluted hydrochloric acid. The ethyl acetate phase is dried with sodium sulfate, concentrated and the residue is purified by flash-chromatography on a silica gel column eluting with ethyl acetate/hexanes 4:6. Evaporation of the pure samples leads to crystalline title compound: m.p. 128-130 °C; MS: [M+1]⁺ = 509; TLC(ethyl acetate): R_{f} = 0.27; HPLC: ^{E}t*_{Ret}* = 4.57.

### Example 65: 4-[5-(3-Trifluoromethyl-phenylcarbamoyl)-naphthalen-2-yloxyl-pyridine-2-carboxylic acid methylamide

4-[5-(3-Trifluoromethyl-phenylcarbamoyl)-naphthalen-2-yloxy]-pyridine-2-carboxylic acid butyl ester (101 mg, 0.2 mmol) and 1.0 mL of a 33% methylamine solution in ethanol are heated for 1 h under reflux. The mixture is cooled, concentrated and the residue is purified by flash-chromatography on a silica gel column eluting with ethyl acetate/hexanes 6:4. Evaporation of the pure samples leads to crystalline title compound: m.p. 175-177 °C; MS: [M+1]⁺= 466; HPLC: ^{E}t*_{Ret}* = 4.18.

Using the same procedure as for the previous example the following compounds were synthesized:

### Example 66: 4-[5-(3-Trifluoromethyl-phenylcarbamoyl)-naphthalen-2-yloxy]-pyridine-2-carboxylic acid amide

The title compound is obtained as a solid: m.p. 117-120 °C; MS: [M+1]⁺ = 452; TLC(ethyl acetate/hexanes 6:4): R_{f} = 0.3; HPLC: ^{E}t*_{Ret}* = 3.91.

### Example 67: 4-[5-(3-Trifluoromethyl-phenylcarbamoyl)-naphthalen-2-yloxy]-pyridine-2-carboxylic acid (2-dimethylamino-ethyl)-amide

The title compound is obtained as a solid: m.p. 80-82 °C; MS: [M+1]⁺ = 523; TLC (dichloromelhane/ethanol 9:1 and 1% conc. ammonia): R_{f} = 0.3; HPLC: ^{E}t*_{Ret}* = 3.46.

### Example 68: 6-(2-Amino-pyridin-4-yloxy)-naphthalene-1-carboxylic acid (3-trifluoromethyl-phenyl)-amide

A solution of 60 mg (0.11 mmol) {4-[5-(3-trifluoromethyl-phenylcarbamoyl)-naphthalen-2-yloxy]-pyridin-2-yl}-carbamic acid tert-butyl ester in 1 mL dioxane is added a 4N hydrochloric acid solution in dioxane (30 µL, 0.13 mmol) and the resulting solution heated under reflux for 8 h. The dioxane is evaporated and the residue partitioned between ethyl acetate and saturated sodium bicarbonate solution. The aqueous phase was extracted twice wit ethyl acetate. The combined ethyl acetate pahses are washed with brine, dried with sodium sulfate and evaporated. The residue is purified by flash-chromatography on a silica gel column eluting with dichloromethane/ethanol 95:5 containing 1 % conc. ammonia. Evaporation of the pure samples leads to crystalline title compound: m.p. 222-224 °C; MS: [M+1]⁺ = 424; TLC (dichloromethane/ethanol 95:5 and 1% conc. ammonia): R_{f} = 0.3; HPLC: ^{E}t*_{Ret}* = 3.46.

The starting material is prepared as follows:

### Step 68.1: {4-[5-(3-Trifluoromethyl-phenylcarbamoyl)-naphthalen-2-yloxyl-pyridin-2-yl}-carbamic acid tert-butyl ester

A mixture of 113 mg (0.25 mmol) 4-[5-(3-trifluoromethyl-phenylcarbamoyl)-naphthalen-2-yloxy]-pyridine-2-carboxylic acid, 1.5 mL *tert*.-butanol 0.07 mL (0.3 mmol) phosphorazidic acid diphenyl ester and 0.04 mL (0.03 mmol) triethylamine is heated under reflux for 4 h. The solvent is evaporated, the residue taken up in ethyl acetate and washed with saturated sodium bicarbonate solution and brine, dried with sodium sulfate and concentrated again. The residue is purified by flash-chromatography on a silica gel column eluting with ethyl acetate/hexanes 4:6. Evaporation of the pure samples leads to the title compound: MS: [M+1]⁺ = 524; TLC (ethyl acetate/hexanes 4:6): R_{f} = 0.35; HPLC: ^{E}t*_{Ret}* = 4.07.

### Step 68.2: 4-[5-(3-Trifluoromethyl-phenylcarbamoyl)-naphthalen-2-yloxyl-pyridine-2-carboxylic acid

4-[5-(3-Trifluoromethyl-phenylcarbamoyl)-naphthalen-2-yloxy]-pyridine-2-carboxylic acid butyl ester (165 mg, 0.32 mmol) is dissolved in 6 mL of ethanol and treated with 0.34 mL 1 N sodium hydroxide solution. The suspension is heated for 2 h under reflux, cooled and the solvent evaporated. The residue is triturated with ethyl acetate and filtered. The solid is taken up in a small amaont of H₂O and acidified with 2N hydrochloric acid (pH -5). Extraction with ethyl acetate followed by drying of the ethyl acetate extracts with sodium sulfate and evaporation of the solvent gives pure title compound: MS: [M+1]⁺= 453; TLC (ethyl acetate/hexanes 4:6): R_{f} = 0.35; HPLC: ^{E}t*_{Ret}* = 3.29.

### Example 69: {4-[5-(3-Trifluoromethyl-phenylcarbamoyl)-naphthalen-2-yloxy]-pyridin-2-yl}-carbamic acid methyl ester

To a mixture of 42 mg (0.1 mmol) 6-(2-amino-pyridin-4-yloxy)-naphthalene-1-carboxylic acid (3-trifluoromethyl-phenyl)-amide and 16 µL (0.12 mmol) triethylamine in 1 mL of THF are added 10 µL (0.12 mmol) of methyl chloroformate at it. After stirring for 1 h at rt the mixture is diluted with ethyl acetate. This is then washed with saturated sodium bicarbonate solution and brine, dried with sodium sulfate and evaporated. The residue is purified by flash-chromatography on a silica gel column eluting with ethyl acetate/hexanes 1:1. After a second chromatography of the enriched fractions, the pure title compound is obtained as a solid: m.p. 230-232 °C; MS: [M+1]⁺ = 482; TLC (ethyl acetate/hexanes 1:1): R_{f} = 0.45; HPLC: ^{E}t*_{Ret}* = 3.69.

### Example 70: 6-[2-(2-Amino-pyrimidin-4-yl)-ethyl]-naphthalene-1-carboxylic acid (3-trifluoromethyl-phenyl)-amide

6-(2-Amino-6-chloro-pyrimidin-4-ylethynyl)-naphthalene-1-carboxylic acid (3-trifluoromethyl-phenyl)-amide (0.069 g, 0.148 mmol) is hydrogenated in the presence of 12 mg of magnesium oxide and 20 mg 10% palladium on carbon in 5 mL of THF at rt. After 48 h the catalyst was filtered off and the filtrate evaporated. The residue is chromatographed on a 4 g silica gel column on a Combi-Flash Companion™ (Isco Inc.) apparatus using a gradient of 20% → 100% ethyl acetate in hexanes as solvent. The title compound is obtained as a colorless solid: m.p. 225-227 °C; MS: [M+1]⁺ = 435; TLC (ethyl acetate): R_{f} = 0.25; HPLC: ^{E}t*_{Ret}* = 3.43.

The starting material is prepared as follows:

### Step 70.1: 6-(2-Amino-6-chloro-pyrimidin-4-ylethynyl)-naphthalene-1-carboxylic acid (3-trifluoromethyl-phenyl)-amide

Nitrogen is passed through a solution of 130 mg (0.383 mmol) 6-ethynyl-naphthalene-1-carboxylic acid (3-trifluoromethyl-phenyl)-amide and 57 mg (0.348 mmol) 2-amino-4,6-dichloro-pyrimidine in 1.3 mL DMF. After 10 to 15 minutes, copper (I) iodide (3.5 mg (0.0184 mmol), bis-(triphenylphosphine)-palladium dichloride (17.4 mg, 0.0248 mmol) and triethylamine (52.2 µL, 0.375 mmol) is added and the mixture stirred at rt and under a nitrogen atmosphere for 16 h. The DMF is removed under reduced pressure and the residue is chromatographed on a 12 g silica gel column on a Combi-Flash Companion™ (Isco Inc.) apparatus using a gradient of 0% → 50% ethyl acetate in hexanes as solvent. The title compound is obtained as a colorless solid: m.p. 171-175 °C; MS: [M+1]⁺ = 465, 467; TLC (ethyl acetate/hexanes 1:1): R_{f} = 0.31; HPLC: ^{E}t*_{Ret}* = 4.74.

### Step 70.2: 6-Ethynyl-naphthalene-1-carboxylic acid (3-trifluoromethyl-phenyl)-amide

6-Trimethylsilanylethynyl-naphthalene-1-carboxylic acid (3-trifluoromethyl-phenyl)-amide (0.194 g, 0.47 mmol) is dissolved in 3.8 mL of methanol at rt. After the addition of potassium carbonate 0.1 g (0.724 mmol) the mixture is stirred for 16 at rt. The solvent was removed and the residue partitioned between 10 mL of ethyl acetate and 5 mL of H₂O. The organic phase is washed with brine, dried with sodium sulfate and evaporated. The title compound is obtained as a brown resin: MS: [M+1]⁺ = 340;HPLC: ^{E}t*_{Ret}* = 4.58.

### Step 70.3: 6-Trimethylsilanylethynyl-naphthalene-1-carboxylic acid (3-trifluoromethyl-phenyl)-amide

Trifluoro-methanesulfonic acid 5-(3-trifluoromethyl-phenylcarbamoyl)-naphthalen-2-yl ester (0.695 g (1.5 mmol), copper (I) iodide (0.015 g, 0.079 mmol) and bis-(triphenylphosphine)-palladium dichloride (17.4 mg, 0.0248 mmol) are mixed in a Schlenk apparatus under nitrogen and treated at rt with a carefully degassed solution of 0.233 mL (1.65 mmol) ethinyl-trimethylsilane and 0.225 mL (1.62 mmol) triethylamine in 5.6 mL dry DMF. The clear dark solution is kept at rt for 16 h and then the DMF is evaporated under reduced pressure. The residue is chromatographed on a 40 g silica gel column on a Combi-Flash Companion™ (Isco Inc.) apparatus using a gradient of 0% → 20% ethyl acetate in hexanes as solvent. The title compound is obtained as a tan solid: m.p. 134-136 °C; MS: [M+1]⁺ = 412; TLC (ethyl acetate/hexanes 1:4): R_{f} = 0.28; HPLC: ^{E}t*_{Ret}* = 5.40.

### Step 70.4: Trifluoro-methanesulfonic acid 5-(3-trifluoromethyl-phenylcarbamoyl)-naphthalen-2-yl ester

1.324 mg (4.0 mmol) 6-hydroxy-naphthalene-1-carboxylic acid (3-trifluoromethyl-phenyl)-amide are dissolved in 7.2 mL of pyridine and the solution collected to - 10 to -15°C. Trifluorosulfonic acid anhydride (0.824 mL, 5 mmol) is added dropwise at that temperature over 10 minutes. The mixture is then stirred at 0 °C for 10 minutes and then 2 h at rt. The reaction mixture is poured onto 25 mL of ice-H₂O, stirred efficiently for a few minutes and then extracted with tert.-butyl-methylether. The organic phases are combined and washed with 1N HCl and brine, dried with sodium sulfate and concentrated to about 15 to 20 mL. The suspension formed is cooled to 5 °C to complete he crystallization. The title compound is collected by filtration and dried: m.p. 177-178 °C; MS: [M+1]⁺ = 464; TLC (ethyl acetate/hexanes 3:7): R_{f} = 0.34; HPLC: ^{E}t*_{Ret}*=4.90.

### Example 71: 6-(6-Amino-pyrimidin-4-ylethynyl)-naphthalene-1-carboxylic acid (3-trifluoromethyl-phenyl)-amide

In a three-necked flask equipped with a reflux condenser, nitrogen inlet and magnetic stirrer are placed 12.8 mL of dimethoxy-ethane and 2.2 mL of H₂O. Nitrogen is bubbled through the solution for 5 minutes and then 33.3 mg (0.184 mmol) palladium chloride, 51.1 mg (0.263 mmol) copper (I) iodide and 191 mg (0.693 mmol) triphenylphosphine are added and the mixture is heated to 40 °C. Next, 6-trimethylsilanylethynyl-naphthalene-1-carboxylic acid (3-trifluoromethyl-phenyl)-amide (0.412 g, 1 mmol), 4-amino-6-chloropyrimidine (165 mg, 1.25 mmol) and potassium carbonate (629 mg, 4.5 mmol) are added and the mixture stirred at 75 °C for 15 h. After cooling the organic layer is separated and treated with 2.5 g of silica gel. The solvent is removed and the crude product coated on silica gel chromatographed on a 40 g silica gel column on a Combi-Flash Companion™ (Isco Inc.) apparatus using a gradient of 30% → 100% ethyl acetate in hexanes as solvent. The title compound is obtained as a tan solid: m.p. 217-220 °C; MS: [M+1]⁺ = 433; TLC (ethyl acetate/hexanes 1:4): R_{f} = 0.19; HPLC: ^{E}t*_{Ret}* = 3.36.

### Example 72: 6-(6-Amino-pyrimidin-4-yloxyl-1H-indole-3-carboxylic acid (3-trifluoromethyl-phenyl)-amide (Methode A).

In a sealed tube, a solution of 50 mg (0.19 mMol) 6-(6-amino-pyrimidin-4-yloxy)-1 H-indole-3-carboxylic acid, 69.1 □l (0.56 mMol) 3-aminobenzotrifluoride and 128.9 □l (0.93 mMol) Et₃N in 1 ml DMF was treated with 131 □l (0.22 mMol) of propylphosphonic anhydride (ca. 50% in DMF) and stirred at RT overnight. The reaction mixture was directly purified by reversed phase prep-HPLC (Waters system) to give the title compound as its TFA salt: MS: [M+1]⁺ = 414; HPLC: ^{F}t*_{Ret}* = 1.78.

The starting material is prepared as follows:

### Step 72.1: 6-Hydroxy-1H-indole-3-carboxylic acid methyl ester.

In a sealed flask, a mixture of 10.0 g (35.6 mmol) 6-benzyloxy-1H-indole-3-carboxylic acid methyl ester (synthesized according to a literature procedure : M. Fedouloff and al. Bioorg. Med. Chem. 9, 2001, 2119-2128), 2.26 g (35.6 mmol) ammonium formate and 3.78 g Pd/C 10% in 200 ml EtOH was stirred at RT for 1 h. The catalyst was filtered and washed with hot MeOH. The filtrate was evaporated to give the title compound: MS: [M+1]⁺ = 192; HPLC: ^{F}t*_{Ret}* = 1.13.

### Step 72.2: 6-(6-Chloro-pyrimidin-4-yloxy)-1H-indole-3-carboxylic acid methyl ester.

To a solution of 6.2 g (32.4 mMol) 6-hydroxy-1H-indole-3-carboxylic acid methyl ester in 50 ml acetone, 35.7 ml (35.7 mMol) 1 N NaOH and 4.8 g (32.4 mmol) 4,6-dichloropyrimidine were added. The reaction mixture was stirred at RT for 1 h during which time the product precipitate. The reaction mixture was filtered and the solid washed with cold acetone/H₂O (1:1) then Et₂O to give the title compound which was used without further purification: MS: [M+1]⁺ = 304; HPLC: ^{F}t*_{Ret}* = 1.94.

### Step 72.3: 6-(6-Azido-pyrimidin-4-yloxy)-1H-indole-3-carboxylic acid methyl ester.

A mixture of 7.0 g (23.0 mMol) 6-(6-chloro-pyrimidin-4-yloxy)-1H-indole-3-carboxylic acid methyl ester and 3.0 g (46.1 mMol) NaN₃ in 75 ml DMF was stirred at 60°C for 2.5 h. The reaction mixture was diluted in EtOAc then washed with H₂O and brine. The organic layer was dried over Na₂SO₄, filtered, and evaporated. Combi-Flash Companion™ (Isco Inc.) column chromatography (SiO₂; gradient elution, hexane/EtOAc 8:2 → 4:6) yielded the title compound: MS: [M+1]⁺ = 311; HPLC: ^{F}t*_{Ret}* = 2.07.

### Step 72.4: 6-(6-Amino-pyrimidin-4-yloxy)-1H-indole-3-carboxylic acid methyl ester.

In a sealed flask, a suspension of 3.4 g (11.0 mMol) 6-(6-azido-pyrimidin-4-yloxy)-1H-indole-3-carboxylic acid methyl ester, 1.4 g (22.1 mMol) ammonium formate and 1.2 g Pd/C 10% in 65 ml EtOH was stirred at RT for 1 h. The catalyst was filtered through a *Celite* pad and washed with MeOH. The filtrate was evaporated to give the title compound which was used without further purification: MS: [M+1]⁺ = 285; HPLC: ^{F}t_{Ret} = 1.08.

### Step 72.5: 6-(6-Amino-pyrimidin-4-yloxy)-1H-indole-3-carboxylic acid.

A suspension of 3.38 g (11.9 mMol) 6-(6-amino-pyrimidin-4-yloxy)-1H-indole-3-carboxylic acid methyl ester and 5.04 g (118.9 mMol) LiOH·H₂O in 136 ml MeOH / H₂O (5:3) was stirred at 80°C for 4 h. The clear solution was cooled to RT, acidified by the addition of 6.7 ml (178.0 mMol) formic acid, and concentrated under reduced pressure. The residue was diluted in H₂O and the formed solid was filtered and washed with H₂O to yield the title compound: MS: [M+1]⁺ = 271; HPLC: ^{F}t*_{Ret}* = 0.69.

### Example 73: 6-(6-Amino-pyrimidin-4-yloxy)-1H-indole-3-carboxylic acid (3-methoxy-phenyl)-amide (Methode B).

A solution of 50 mg (0.19 mMol) 6-(6-amino-pyrimidin-4-yloxy)-1H-indole-3-carboxylic acid, 68.0 mg (0.56 mmol) 3-methoxyphenylamine, 102.0 □l (0.93 mmol) NMM and 106 mg (0.22 mMol) HATU in 1 ml DMF was stirred at RT overnight. The reaction mixture was directly purified by reversed phase prep-HPLC (Waters system) to give the title compound as its TFA salt: MS: [M+1]⁺ = 376; HPLC: ^{F}t*_{Ret}* = 1.40.

### Example 74: 6-(6-Amino-pyrimidin-4-yloxy)-1H-indole-3-carboxylic acid (4-fluoro-3-trifluoromethyl-phenyl)-amide (Methode C).

A suspension of 50 mg (0.19 mmol) 6-(6-amino-pyrimidin-4-yloxy)-1H-indole-3-carboxylic acid in 2 ml dioxane was treated with 134.6 □l (1.85 mmol) SOCl₂ and stirred under reflux for 2 h. The reaction mixture was evaporated under reduced pressure leading to the crude acid chloride. To a solution of the crude acid chloride in 2 ml NMP, 35.6 □l (0-278 mmol) 4-fluoro-3-(trifluoromethyl)aniline and 306 □l (2.78 mmol) NMM were added. The reaction mixture was stirred at RT for 2 h then directly purified reversed phase prep-HPLC (Waters system). After lyophilization, the title compound was obtained as its TFA salt: MS: [M+1]⁺ = 432; HPLC: ^{G}t*_{Ret}* = 2.83.

### Example 75: The following compounds can be obtained analogously to Ex. 72, Ex. 73 or Ex. 74.

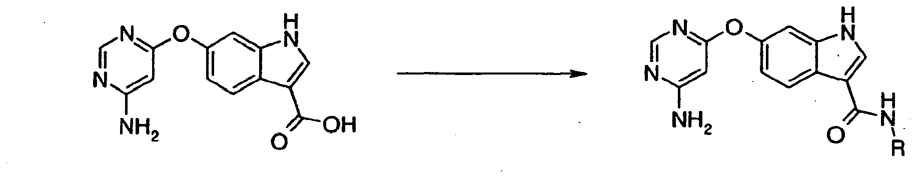

| | R | Methode | HPLC ^{F}t*_{Ret}* [min] | MS [M+1]⁺ |
|---|---|---|---|---|
| a) | 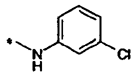 | A | 1.61 | 380 |
| b) | 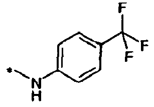 | A | 1.76 | 414 |
| c) | 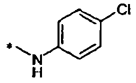 | A | 1.58 | 380 |
| d) | 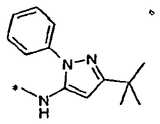 | A | 1.71 | 468 |
| e) | 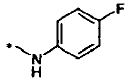 | B | 1.40 | 364 |
| f) | 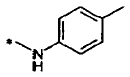 | B | 1.48 | 360 |
| g) | 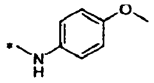 | B | 1.33 | 376 |
| h) | 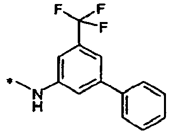 | C | 2.21 | 490 |

### Example 76: 6-(6-Amino-pyrimidin-4-yloxy)-1-methyl-1H-indole-3-carboxylic acid (3-trifluoromethyl-phenyl)-amide.

In a sealed flask, a solution of 16 mg (0.038 mmol) 6-(6-chloro-pyrimidin-4-yloxy)-1-methyl-1H-indole-3-carboxylic acid (3-trifluoromethyl-phenyl)-amide in 1 ml NMP and 1 ml NH₄OH 25% was stirred at 120°C for 2 h. The reaction mixture was concentrated under reduced pressure and the residue purified by prep-HPLC to give the title compound as its TFA salt: MS: [M+1]⁺ = 428; HPLC: ^{F}t*_{Ret}* = 1.87.

The starting material is prepared as follows:

### Step 76.1: 6-Benzyloxy-1-methyl-1H-indole-3-carboxylic acid methyl ester.

To a solution of 2.33 g (8.28 mmol) 6-benzyloxy-1H-indole-3-carboxylic acid methyl ester in 20 ml DMF, 4.05 g (12.42 mMol) Cs₂CO₃ and 1.03 ml (16.57 mmol) Mel were added and the mixture was stirred at 60°C for 3 h. The reaction middle was diluted in AcOEt and H₂O, and the aqueous phase was extracted with AcOEt (3 times). The combined organic fractions were washed with brine, then dried over Na₂SO₄, filtered, and evaporated. Purification via silica gel Combiflash chromatography (gradient elution, hexane / TBME 8:2 to 4:6) led to the title compound as a brownish solid: MS: [M+1]⁺ = 296; HPLC: ^{F}t*_{Ret}* = 2.58. R_{F} = 0.34 (TBME/ hexane 1:1).

### Step 76.2: 6-Benzyloxy-1-methyl-1H-indole-3-carboxylic acid.

A suspension of 2.06 g (6.98 mmol) 6-benzyloxy-1-methyl-1H-indole-3-carboxylic acid methyl ester and 2.63 g (62.78 mMol) LiOH·H₂O in 75 ml MeOH / H₂O (2:1) was stirred at 60°C overnight. The clear solution was cooled to RT and concentrated. The residue was diluted in H₂O and acidified by the addition of 2 M HCl. The formed white precipitate was collected by filtration, washed with H₂O and dried at 40°C under high vacuum to give the title compound as an off-white solid: MS: [M+1]⁺ = 282; HPLC: ^{F}t_{Ret} = 2.12.

### Step 76.3: 6-Hydroxy-1-methyl-1H-indole-3-carboxylic acid.

In a sealed flask, a suspension of 1.57 g (5.58 mmol) 6-benzyloxy-1-methyl-1H-indole-3-carboxylic acid, 528 mg (8.37 mmol) ammonium formate and 594 mg Pd/C 10% in 25 ml EtOH was stirred at RT for 2 h. The catalyst was filtered and washed with hot MeOH. The filtrate was evaporated to give the title compound as an off-white solid: MS: [M+1]⁺ = 192; HPLC: ^{F}t*_{Ret}* = 0.85.

### Step 76.4: 6-(6-Chloro-pyrimidin-4-yloxy)-1-methyl-1H-indole-3-carboxylic acid.

To a solution of 800 mg (4.18 mmol) 6-hydroxy-1-methyl-1H-indole-3-carboxylic acid in 20 ml acetone, 10 ml (10 mmol) 1 N NaOH and 935 mg (6.28 mMol) 4,6-dichloropyrimidine were added. The reaction mixture was stirred at RT for 2 h, then concentrated under reduced pressure. The residue was diluted in H₂O and extracted with CH₂Cl₂ (2 times). The aqueous phase was acidified by the addition of 2 N HCl (→ pH 3-4) and the resulting slurry was extracted with AcOEt (3 times). The combined organic fractions were dried over Na₂SO₄, filtered and evaporated to yield the title compound as a brown solid which was used in the next step without further purification: MS: [M+1]⁺ = 304; HPLC: ^{F}t*_{Ret}* = 1.68.

### Step 76.5: 6-(6-Chloro-pyrimidin-4-yloxy)-1-methyl-1H-indole-3-carboxylic acid (3-trifluoro-methylphenyl)-amide.

A suspension of 60 mg (0.20 mmol) 6-(6-chloro-pyrimidin-4-yloxy)-1-methyl-1H-indole-3-carboxylic acid in 2 ml dioxane was treated with 143.8 □l (1.98 mMol) SOCl₂ and stirred under reflux for 2 h. The reaction mixture was evaporated under reduced pressure leading to the crude acid chloride. To the crude acid chloride in 2 ml NMP, 36.9 □l (0.30 mMol) 3-(trifluoromethyl)aniline and 326.5 □l (2.96 mmol) NMM were added. The reaction mixture was stirred at RT for 2 h then directly purified by injection in prep-HPLC (Waters system). After lyophilization, the title compound was obtained: MS: [M+1]⁺ = 447; HPLC: ^{F}t*_{Ret}* = 2.76.

### Example 77: The following compounds can be obtained analogously to Ex. 76.

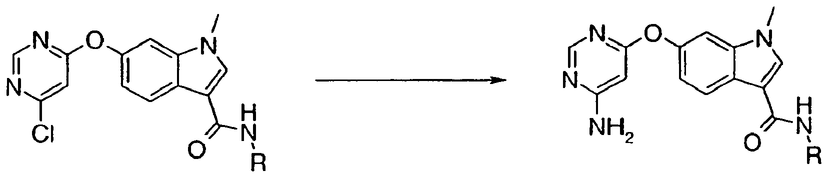

| | R | HPLC ^{F}t*_{Ret}* [min] | MS [M+1]⁺ |
|---|---|---|---|
| a) | 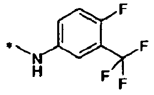 | 1.90 | 446 |
| b) | 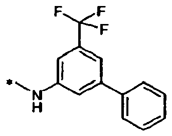 | 3.42 (^{G}t*_{Ret}*) | 504 |

### Example 78: 6-(6-Amino-pyrimidin-4-yloxy)-1-ethyl-1H-indole-3-carboxylic acid (3-trifluoromethyl-phenyl)-amide.

Prepared as described in Ex. 74 from 50 mg (0.17 mmol) 6-(6-amino-pyrimidin-4-yloxy)-1-ethyl-1H-indole-3-carboxylic acid and 31.3 □l (0.25 mMol) 3-aminobenzotrifluoride: [M+1]⁺ = 442; HPLC: ^{F}t*_{Ret}* = 1.93.

The starting material is prepared as follows:

### Step 78.1: 6-Benzyloxy-1-ethyl-1H-indole-3-carboxylic acid methyl ester.

Prepared as described in Ex. 76, Step 76.1 from 2.0 g (7.11 mMol) 6-benzyloxy-1 H-indole-3-carboxylic acid methyl ester and 1.06 ml (14.22 mmol) ethyl bromide: [M+1]⁺ = 310; HPLC: ^{G}t*_{Ret}* = 3.61.

### Step 78.2: 6-Benzyloxy-1-ethyl-1H-indole-3-carboxylic acid.

Prepared as described in Ex. 76, Step 76.2 from 1.8 g (7.76 mmol) 6-benzyloxy-1-ethyl-1H-indole-3-carboxylic acid methyl ester: [M+1]⁺ = 296; HPLC: ^{F}t*_{Ret}* = 2.27.

### Step 78.3: 6-Hydroxy-1-ethyl-1H-indole-3-carboxylic acid.

Prepared as described in Ex. 76, Step 76.3 from 1.64 g (5.55 mmol) 6-benzyloxy-1-ethyl-1 H-indole-3-carboxylic acid: [M+1]⁺ = 206; HPLC: ^{F}t*_{Ret}* = 1.05.

### Step 78.4: 6-(6-Chloro-pyrimidin-4-yloxy)-1-ethyl-1H-indole-3-carboxylic acid.

Prepared as described in Ex. 76, Step 76.4 from 1.21 g (6.33 mmol) 6-hydroxy-1-ethyl-1H-indole-3-carboxylic acid and 1.89 g (12.66 mmol) 4,6-dichloropyrimidine: [M+1]⁺ = 318; HPLC: ^{F}t*_{Ret}* = 1.85.

### Step 78.5: 6-(6-Azido-pyrimidin-4-yloxy)-1-ethyl-1H-indole-3-carboxylic acid.

A mixture of 1.5 g (4.72 mmol) 6-(6-chloro-pyrimidin-4-yloxy)-1-ethyl-1H-indole-3-carboxylic acid and 1.23 g (18.88 mmol) NaN₃ in 15 ml DMF was stirred at 60°C for 2.5 h. The reaction mixture was diluted in AcOEt then washed with 2 M HCl and brine. The organic layer was dried over Na₂SO₄, filtered, and evaporated. Combi-Flash Companion™ (Isco Inc.) column chromatography (SiO₂; hexane with 2% AcOH / EtOAc 8:2 → 2:8) yielded the title compound: MS: [M+1]⁺ = 325; HPLC: ^{F}t*_{Ret}* = 1.97.

### Step 78.6: 6-(6-Amino-pyrimidin-4-yloxy)-1-ethyl-1H-indole-3-carboxylic acid.

Prepared as described in Ex. 72, Step 72.4 from 1:06 g (3.28 mMol) 6-(6-azido-pyrimidin-4-yloxy)-1-ethyl-1H-indole-3-carboxylic acid, 418.0 mg (6.56 mMol) ammonium formate and 349.2 mg Pd/C 10%: [M+1]⁺ = 299; HPLC: ^{F}t*_{Ret}* = 1.00.

### Example 79: 6-(6-Amino-pyrimidin-4-yloxy)-1-ethyl-1H-indole-3-carboxylic acid (4-fluoro-3-trifluoromethyl-phenyl)-amide.

Prepared as described in Ex. 78 from 50 mg (0.17 mmol) 6-(6-amino-pyrimidin-4-yloxy)-1-ethyl-1H-indole-3-carboxylic acid and 32.3 □l (0.25 mMol) 4-fluoro-3-(trifluoromethyl)aniline: [M+1]⁺ = 460; HPLC: ^{F}t*_{Ret}* = 2.00.

### Example 80: 6-(6-Amino-pyrimidin-4-yloxy)-1-iso-propyl-1H-indole-3-carboxylic acid (3-trifluoromethyl-phenyl)-amide.

Prepared as described in Ex. 76 from 18 mg (0.038 mMol) 6-(6-chloro-pyrimidin-4-yloxy)-1-isopropyl-1H-indole-3-carboxylic acid (3-trifluoromethyl-phenyl)-amide: [M+1]⁺ = 456; HPLC: ^{G}t*_{Ret}* = 3.14.

The starting material is prepared as follows:

### Step 80.1: 6-Benzyloxy-1-iso-propyl-1H-indole-3-carboxylic acid methyl ester.

Prepared as described in Ex. 76, Step 76.1 from 1.0 g (3.56 mmol) 6-benzyloxy-1H-indole-3-carboxylic acid methyl ester and 667 □l (7.10 mMol) 2-bromopropane: [M+1]⁺ = 324; HPLC: ^{G}t*_{Ret}* = 3.72.

### Step 80.2: 6-Benzyloxy-1-iso-propyl-1H-indole-3-carboxylic acid.

Prepared as described in Ex. 76, Step 76.2 from 1.04 g (3.22 mMol) 6-benzyloxy-1-iso-propyl-1H-indole-3-carboxylic acid methyl ester: [M+1]⁺ = 310; HPLC: ^{F}t*_{Ret}* = 2.38.

### Step 80.3: 6-Benzyloxy-1-iso-propyl-1H-indole-3-carboxylic acid (3-trifluoromethyl-phenyl)-amide.

A suspension of 100 mg (0.32 mmol) 6-benzyloxy-1-iso-propyl-1H-indole-3-carboxylic acid in 2 ml dioxane was treated with 235.2 □l (3.23 mmol) SOCl₂ and stirred under reflux for 2 h. The reaction mixture was evaporated under reduced pressure leading to the crude acid chloride. To the crude acid chloride in 2 ml NMP, 60.3 □l (0.48 mmol) 3-(trifluoromethyl)aniline and 534.2 □l (4.85 mMol) NMM were added. The reaction mixture was stirred at RT for 2 h then diluted in H₂O and extracted with TBME. The organic fraction was successively washed with 2 M HCl, 2 M Na₂CO₃, and brine, then dried over Na₂SO₄, filtered and evaporated. Combi-Flash Companion™ (Isco Inc.) column chromatography (SiO₂; hexane / EtOAc 95:5 → 6:4) yielded the title compound: MS: [M+1]⁺ = 453; HPLC: ^{F}t*_{Ret}* = 3.25.

### Step 80.4: 6-Hydroxy-1-iso-propyl-1H-indole-3-carboxylic acid (3-trifluoromethyl-phenyl)-amide.

Prepared as described in Ex. 76, Step 76.3 from 44 mg (0.097 mmol) 6-benzyloxy-1-*iso*-propyl-1H-indole-3-carboxylic acid (3-trifluoromethyl-phenyl)-amide: [M+1]⁺ = 363; HPLC: ^{F}t*_{Ret}* = 2.43.

### Step 80.5: 6-(6-Chloro-pyrimidin-4-yloxy)-1-iso-propyl-1H-indole-3-carboxylic acid (3-trifluoromethyl-phenyl)-amide.

Prepared as described in Ex. 76, Step 76.4 from 34 mg (0.094 mmol) 6-hydroxy-1-*iso*-propyl-1H-indole-3-carboxylic acid (3-trifluoromethyl-phenyl)-amide and 14.0 mg (0.094 mmol) 4,6-dichloropyrimidine: [M+1]⁺ = 475; HPLC: ^{F}t*_{Ret}* = 3.00.

### Example 81: 6-(6-Acetylaminopyrimidin-4-yloxy)-naphthalene-1-carboxylic acid (4-piperazin-1-ylmethyl-3-trifluoromethylphenyl)amide

A solution of HCl (4.5 mL of 4M in dioxane) is added to a stirred solution of 6-(6-acetylamino-pyrimidin-4-yloxy)-naphthalene-1-carbonylamino (2-trifluoromethyl-benzyl)-1-1-piperazinecarboxylic acid, 1,1-dimethylethyl ester (step 81.3, 520 mg, 0.727 mmol) in dioxane (4.5 mL). After 2 hours, the mixture is neutralised with aqueous NaOH (2M). The precipitated product is filtered, washed with H₂O and dried. The solid obtained s dissolved in CH₂Cl₂-MeOH (5:1) and the CH₂Cl₂ is evaporated off under reduced pressure to afford a suspension which is filtered to give the title compound as a beige solid, m.p. 194-197°C.

The starting material is prepared as follows:

### Step 81.1: 6-(6-Chloropyrimidin-4-yloxy)-naphthalene-1-carbonyl chloride

A solution of oxalyl chloride (571 µL, 6.66 mmol) in CH₂Cl₂ (15 mL) is added to an ice-cooled solution of 6-(6-chloro-pyrimidin-4-yloxy)-naphthalene-1-carboxylic acid (Example 25, step 25.1; 1 g, 3.33 mmol) and DMF (10 µL) in CH₂Cl₂ (30 mL). The reaction mixture is stirred at room temperature for 1 h. The solvent is then evaporated off under reduced pressure to afford the title compound as a brown solid, which is used directly without further purification.

### Step 81.2: 6-(6-Chloropyrimidin-4-yloxy)-naphthalene-1-carbonylamino (2-trifluoromethyl-benzyl)-1-piperazinecarboxylic acid, 1,1-dimethylethyl ester

A solution of 6-(6-chloropyrimidin-4-yloxy)-naphthatene-1-carbonyl chloride (step 81. 1, 1.15 g, 2.89 mmol) in CH2Cl2 (20 mL) is added to a stirred solution of 4-(4-amino-2-trifluoromethylbenzyl)-1-piperazinecarboxylic acid, 1,1-dimethylethyl ester (922 mg, 2.51 mmol) and diisopropylethylamine (878 µL, 5.03 mmol) in CH₂Cl₂ (20 mL). After 30 min, the reaction mixture is poured into a mixture of H₂O and CH₂Cl₂. The aqueous phase is separated off and extracted with CH₂Cl₂. The combined organic layers are washed with H₂O and brine, dried (Na₂SO₄) and concentrated. Column chromatography (SiO₂; CH₂Cl₂ / EtOAc 1:1) gives the title compound as a brown solid.

### Step 81.3: 6-(6-Acetylamino-pyrimidin-4-yloxy)-naphthalene-1-carbonylamino (2-trifluoromethylbenzyl)-1-1-piperazinecarboxylic acid, 1,1-dimethylethyl ester

A mixture of 6-(6-chloro-pyrimidin-4-yloxy)-naphthalene-1-carbonylamino (2-trifluoromethylbenzyl)-1-piperazinecarboxylic acid, 1,1-dimethylethyl ester (step 81.2; 637 mg, 0.97 mmol), acetamide (86 mg, 1.46 mmol), (9,9-dimethyl-9H-xanthene-4,5-diyl)bis[diphenylphosphine] (34 mg, 0.058 mmol), tris(dibenzylideneacetone)dipalladium (18 mg, 0.019 mmol), cesium carbonate (448 mg, 1.36 mmol) in dry dioxan (5 mL) is heated under an argon atmosphere at 70°C for 3 h. The cooled suspension is diluted with H₂O, filtered (hyflo) and the residue is dissolved in EtOAc. The solvent is evaporated off under reduced pressure to afford the crude product which is used in the next step without further purification.

### Example 82: 6-(6-Amino-pyrimidin-4-yloxy)-naphthalene-1-carboxylic acid (4-piperaz in-1-ylmethyl-3-trifluoromethylphenyl)amide

A solution of 6-(6-acetylamino-pyrimidin-4-yloxy)-naphthalene-1-carboxylic acid (4-piperazin-1-ylmethyl-3-trifluoromethylphenyl)amide (example 81 ; 260 mg, 0.39 mmol) in HCl (2 mL of 4M) - MeOH (2 mL) is heated at 50°C for 16 hours. After cooling, the solution is neutralised with NaOH (4 mL of 2M) and the resulting suspension is filtered and washed with H₂O. The solid is then dried under high vacuum. Column chromatography (SiO₂; CH₂Cl₂/ EtOH/ NH₃ 90:9:1) gives the title compound as colourless solid: mp.: 123-128 °C.

### Example 83 : 6-(5-(4-Piperazin-1-ylmethyl-3-trifluoromethylphenylcarbamoyl)naphthalene-2-yloxy)pyrimidin-4-yl)carbamic acid methyl ester

This compound can be obtained analogously to example 81, utilising methylcarbamate in lieu of acetamide in the step 81.3. Beige solid, m.p. 138-148°C.

### Example 84 : 6-(6-Acetylamino-pyrimidin-4-yloxy)-naphthalene-1-carboxylic acid (4-(4-methyl-piperazin-1-ylmethyl-3-trifluoromethylphenyl)amide

This compound can be obtained analogously to example 81, utilising 4(-4-methylpiperazin-1-ylmethyl)-3-trifluoromethylphenylamine in lieu of 4-(4-amino-2-trifluoromethylbenzyl)-1-piperazinecarboxylic acid, 1,1-dimethylethyl ester in the step 81.2. Beige powder,

1H NMR (400 MHz, DMSO-D6) δ ppm 2.11 (3 H, s) 2.15 (3H, s) 2.26-2.44 (8 H, m) 3.57 (2 H, s) 7.45 (1 H, dd, *J*=9.2, 2.4 Hz) 7.61 (1 H, s) 7.64 (1 H, dd, *J*=8.0, 7.2 Hz) 7.70 (1 H, d, *J*=8.6 Hz) 7.78 (1 H, d*J*=7.0 Hz) 7.85 (1 H, d, *J*=2.4 Hz) 7.99 (1 H, d, *J*=8.8 Hz) 8.07 (1 H, d, *J*=8.5 Hz) 8.25 (1 H, s) 8.25 (1 H, dd, *J*=5.1, 4.1 Hz) 8.48 (1 H, s) 10.86 (1 H, s) 10.96 (1 H, s).

**Example 85** : 6-(6-Aminopyrimidin-4-yloxy)-naphthalene-1-carboxylic acid (4-(4-methyl-piperazin-1-ylmethyl-3-trifluoromethyl-phenyl)-amide

This compound can be obtained analogously to example 82, utilising 6-(6-acetylamino-pyrimidin-4-yloxy)-naphthalene-1-carboxylic acid (4-(4-methyl-piperazin-1-ylmethyl-3-trifluoromethylphenyl)amide (example 4) in lieu of 6-(6-Acetylamino-pyrimidin-4-yloxy)naphthalene-1-carboxylic acid (4-piperazin-1-ylmethyl-3-trifluoromethylphenyl)amide. Colourless powder, 1H NMR (400 MHz, DMSO-D6) δ ppm 2.15 (3 H, s) 2.29 - 2.35 (4 H, m) 2.36 - 2.43 (4 H, m) 3.57 (2 H, s) 5.79 (1 H, d, *J*=1.0 Hz) 6.86 (2 H, s) 7.39 (1 H, dd, *J*=9.2, 2.5 Hz) 7.62 (1 H, dd, *J*=8.1, 7.1 Hz) 7.70 (1 H, d, *J*=8.3 Hz) 7.76 (1 H, dd, *J*=7.3, 1.3 Hz) 7.77 (1 H, d, *J*=2.5 Hz) 7.99 (1 H, dd, *J*=8.6, 1.2 Hz) 8.06 (1 H, d, *J*=8.1 Hz) 8.06 (1 H, d, *J*=1.0 Hz) 8.23 (1 H, d, *J*=8.1 Hz) 8.24 (1 H, s) 10.83 (1 H, s)

### Example 86: 6-(5-(4-(4-methylpiperazin-1-ylmethyl-3-trifluoromethylphenylcarbamoyl)-naphthalene-2-yloxy)pyrimidin-4-yl)carbamic acid methyl ester

This compound can be obtained analogously to example 81, utilising 4(-4-methylpiperazin-1-ylmethyl)-3-trifluoromethyl-phenylamine in lieu of 4-(4-amino-2-trifluoromethyl-benzyl)-1-piperazinecarboxylic acid, 1,1-dimethylethyl ester in the step 81.2. and employing methylcarbamate in lieu of acetamide in the step 81.3. Colourless powder, 1 H NMR (400 MHz, DMSO-D6) δ ppm 2.15 (3 H, s) 2.24 - 2.47 (8 H, m) 3.57 (2 H, s) 3.68 (3 H, s) 7.38 (1 H,s) 7.47 (1 H, dd, *J*=9.2, 2.3 Hz,) 7.66 (1H, dd, *J*=8.2, 7.3 Hz) 7.72 (1 H, d, *J*=8.6 Hz) 7.80 (1 H, d, *J*=7.0 Hz) 7.88 (1 H, d, *J*=2.3 Hz) 8.01 (1 H, d, *J*=8.21 Hz) 8.09 (1 H, d, *J*=8.2 Hz) 8.22 - 8.31 (m,2 H) 8.46 (1 H, s) 10.86 (1 H, s) 10.90 (1 H, s)

### Example 87: 6-(6-Acetylamino-pyrimidin-4-yloxy)naphthalene-1-carboxylic acid (4-(4-isopropylpiperazin-1-ylmethyl-3-trifluoromethylphenyl)amide

This compound can be obtained analogously to example 81, utilising 4(-4-isopropylpiperazin-1-ylmethyl)-3-trifluoromethylphenylamine in lieu of 4-(4-amino-2-trifluoromethyl-benzyl)-1-piperazinecarboxylic acid, 1,1-dimethylethyl ester in step 81.2. pale yellow crystalline solid, m.p. = 210-213°C.

### Example 88: 6-(6-Aminopyrimidin-4-yloxy)-naphthalene-1-carboxylic acid (4-(4-isopropyl-piperazin-1-ylmethyl-3-trifluoromethylphenyl)amide

This compound can be obtained analogously to example 82, utilising 6-(6-acetylaminopyrimidin-4-yloxy)-naphthalene-1-carboxylic acid (4-(4-isopropylpiperazin-1-ylmethyl-3-trifluoromethylphenyl)amide (example 87) in lieu of 6-(6-Acetylamino-pyrimidin-4-yloxy)-naphthalene-1-carboxylic acid (4-piperazin-1-ylmethyl-3-trifluoromethylphenyl)amide. Colourless powder, m.p. 185-189°C.

### Example 89: 6-(5-(4-(4-Isopropylpiperazin-1-ylmethyl-3-trifluoromethyl-phenylcarbamoyl)-naphthalene-2-yloxy)-pyrimidin-4-yl)carbamic acid methyl ester

This compound can be obtained analogously to example 81, utilising 4-(4-isopropylpiperazin-1-ylmethyl)-3-trifluoromethylphenylamine in lieu of 4-(4-amino-2-trifluoromethylbenzyl)-1-piperazinecarboxylic acid, 1,1-dimethylethyl ester in step 81.2. and employing methylcarbamate in lieu of acetamide in the step 81.3. Colourless powder, m.p. = 175-178°C.

### Example 90: 7-(6-Acetylamino-pyrimidin-4-yloxy)-isoquinoline-4-carboxylic acid [4-(4-methyl-piperazin-1-ylmethyl)-3-trifluoromethyl-phenyl]-amide

A mixture of 7-(6-Chloro-pyrimidin-4-yloxy)-isoquinoline-4-carboxylic acid [4-(4-methyl-piperazin-1-ylmethyl)-3-trifluoromethyl-phenyl]-amide (step 90.1; 300 mg, 0.54 mmol), acetamide (47.7 mg, 0.81 mmol), (9,9-dimethyl-9H-xanthene-4,5-diyl)bis[diphenylphosphine] (18.7 mg, 0.032 mmol), tris(dibenzylideneacetone)dipalladium (10 mg, 0.0108 mmol), cesium carbonate (248 mg, 0.75 mmol) in dry dioxan (3 mL) is heated under an argon atmosphere at 70°C for 3 h. The cooled suspension is diluted with H₂O, filtered (hyflo) and the residue is dissolved in EtOAc. The solvent is evaporated off under reduced pressure to afford the crude product which is chromatographed by reversed phase MPLC (Büchi system), yielding, after neutralisation with saturated aqueous NaHCO₃, the title compound as a beige solid, 1 H NMR (400 MHz, DMSO-D6) □ppm 2.13 (3 H, s) 2.15 (3 H, s) 2.33 (4 H, s) 2.36 - 2.42 (4 H, m) 3.43 (1 H, ddd, *J*=13.93, 6.98, 5.05 Hz) 3.57 (2 H, s) 7.67 (1 H, d, *J*=0.76 Hz) 7.72 (1 H, d, *J*=9.16 Hz) 7.75 (1 H, dd, *J*=9.09, 2.27 Hz) 7.99 (1 H, dd, *J*=8.21, 1.52 Hz) 8.08 (1 H, d, *J*=2.46 Hz) 8.24 (1 H, d, *J*=1.71 Hz) 8.33 (1 H, d, *J*=9.16 Hz) 8.49 (2 H, d, *J*=0.82 Hz) 8.80 (1 H, s) 9.43 (1 H, s) 10.99 (1 H, s) 11.00 (1 H, s).

### The starting material is prepared as follows:

### Step 90.1: 7-(6-Chloro-pyrimidin-4-yloxy)-isoquinoline-4-carboxylic acid [4-(4-methyl-piperazin-1-ylmethyl)-3-trifluoromethyl-phenyl]-amide

A mixture of 7-(6-Chloro-pyrimidin-4-yloxy)-isoquinoline-4-carboxylic acid (1.95 g, 5.5 mmol), 4-(4-Methyl-piperazin-1-ylmethyl)-3-trifluoromethyl-phenylamine (1.5 g, 5.49 mmol) and triethylamine (6.42 mL, 46.2 mmol) in dry DMF (50 mL) is heated under an argon atmosphere at 50°C. A solution of propylphosphonic anhydride (5.4 mL, 8.2 mmol) 50% in DMF is then added. After 2 h, the reaction mixture is poured onto an aqueous solution of NaHCO₃ and stirred at 0°C for 1 h. The suspension is then filtered (hyflo) and the solid residue is dissolved in CH₂Cl₂ - MeOH (5:1). The solvent is evaporated off under reduced pressure to afford a crude product which is purified by reversed phase MPLC (Büchi system), yielding, after neutralisation with saturated aqueous NaHCO₃, the title compound as a orange solid.

### Example 91: (6-{4-[4-(4-Methyl-piperazin-1-ylmethyl)-3-trifluoromethyl-phenylcarbamoyl]-isoquinolin-7-yloxy)-pyrimidin-4-yl)-carbamic acid methyl ester

This compound can be obtained analogously to example 90, utilising methylcarbamate in lieu of acetamide. Beige solid, 1 H NMR (400 MHz, DMSO-D6) δ ppm 2.15 (3 H, s) 2.29 - 2.35 (4 H, m) 2.36 - 2.43 (4 H, m) 3.57 (2 H, s) 3.69 (3 H, s) 7.43 (1 H, d, *J*=1.0 Hz) 7.72 (1 H, d, *J*=8.5 Hz) 7.75 (1 H, dd, *J*=9.1, 2.4 Hz) 7.99 (1 H, dd, *J*=8.6, 1.1 Hz) 8.09 (1 H, d, *J*=2.5 Hz) 8.24 (1 H, d, *J*=2.1 Hz) 8.34 (1 H, d, *J*=9.2 Hz) 8.45 (1 H, d, *J*=1 Hz) 8.80 (1 H, s) 9.43 (1 H, d, *J*=0.5 Hz) 10.86 (1H, s) 10.99 (1 H, s).

### Example 92: Via analogous routes the following derivative can be obtained

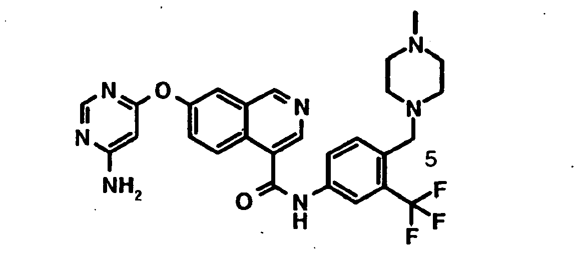

### Example 93: 7-(6-Amino-pyrimidin-4-vloxy)-isoquinoline-4-carboxylic acid [5-tert-butyl-2-(4-isopropyl-phenyl)-2H-pyrazol-3-yl]-amide

7-(6-Azido-pyrimidine-4-yloxy)-isoquinoline-4-carboxylic acid [5-tert-butyl -2-(4-isopropyl-phenyl)-2H-pyrazol-3-yl]-amide (49.0 mg, 0.089 Mmol) is dissolved in MeOH (5 ml) and submitted to hydrogenation over Raney-Nickel (12 mg) at atmospheric pressure at rt for 4 h. After completion the reaction mixture is filtered over a pad of celite and concentrated to give the title compound. MS: [M+1]⁺= 522. Mp 131-133°C.

The starting material is prepared as follows:

### Step 93.1: 7-Hydroxy-isoquinoline-4-carboxylic acid

7-Methoxy-isoquinoline-4-carboxylic acid (2.5 g, 12.3 mmol) is dissolved in HBr/HOAc (33% wt; 10 mL) and H₂O (0.5 mL) is added. The reaction mixture is warmed to 130°C in a sealed tube. After 3 h it is allowed to cool to ambient temperature. All volatiles are removed under reduced pressure and the remaining crude product is used without further purification for the next step.

### Step 93.2: 7-(6-Chloro-pyrimidine-4-yloxy)-isoquinoline-4-carboxylic acid

7-Hydroxy-isoquinoline-4-carboxylic acid (2.3 g, 12.3 Mmol) is dissolved acetone (60 mL) and an aqueous solution of NaOH (1M, 27 mL) is added, followed by 4,6-dicloropyrimidine (1.9 g, 13.2 mmol). The reaction is stirred at ambient temperature for 12h and acetone is removed under reduced pressure. The remaining aqueous solution is acidified with aqueous HCl (1 M). The resulting yellow precipitate of the product is isolated by filtration, washed repeatedly with cold H₂O and dried under high vacuum at 60°C.

### Step 93.3: 7-(6-Chloro-pyrimidine-4-vloxy)-isoquinoline-4-carboxyl choride

7-(6-Chloro-pyrimidine-4-yloxy)-isoquinoline-4-carboxylic acid (1.6 g, 5.5 Mmol) is suspended in CH₂Cl₂ and oxalylchloride (0.57 mL, 6.6 Mmol ) is added. The reaction is then stirred under reflux for 2 h, cooled to ambient temperature and concentrated under reduced pressure. The remaining crude product is used without further purification for the next step.

### Step 93.4: 7-(6-Chloro-pyrimidine-4-yloxy)-isoquinoline-4-carboxylic acid [5-tert-butyl -2-(4-isopropyl-phenyl)-2H-pyrazol-3-yl]-amide

7-(6-Chloro-pyrimidine-4-yloxy)-isoquinoline-4-carboxyl choride (0.5 g, 1.5 Mmol) is dissolved in CH₂Cl₂ (4 mL) and a solution of 5-tert-butyl-2-(4-isopropyl-phenyl)-2-H-pyrazol-3-ylamine (described for example in GB 0500435.3; 0.4 g, 1.5 Mmol) and pyridine (76 □L, 1.7 Mmol) dissolved in CH₂Cl₂ (4 mL) is added dropwise at rt. The reaction is stirred for 1.5 h at rt and then concentrated under reduced pressure. The residual crude product is purified by flash chromatography (SiO₂, CH₂Cl₂/MeOH, gradient 0-8 % MeOH) to give the title compound as a yellow solid.

### Step 93.5: 7-(6-Azido-pyrimidine-4-yloxy)-isoquinoline-4-carboxylic acid [5-tert-butyl -2-(4-isopropyl-phenyl)-2H-pyrazol-3-yl]-amide

7-(6-Chloro-pyrimidine-4-yloxy)-isoquinoline-4-carboxylic acid [5-tert-butyl -2-(4-isopropyl-phenyl)-2H-pyrazol-3-yl]-amide (128.o mg, 0.24 Mmol) is dissolved in DMF (5 mL) and NaN₃ (31.0 mg, 0.47 Mmol) is added in one portion at rt. The reaction mixture is stirred for 1.5 h at 70°C and then concentrated under reduced pressure. The residual crude product is submitted to flash chromatography (SiO₂; CH₂Cl₂/MeOH; gradient 0-5 % MeOH) to give the title compound as a yellow solid.

### Example 94: 7-(6-Amino-pyrimidin-4-yloxy)-isoquinoline-4-carboxylic acid [5-tert-butyl-2-(4-fluorophenyl)-2H-pyrazol-3-yl]-amide

The title compound is prepared in analogy to Ex. 93 using 5-tert-butyl-2-(4-fluoro-phenyl)-2H-pyrazol-3-ylamine MS: [M+1]⁺ = 498. Mp 124-126°C.

### Example 95: 7-(6-Methylamino-pyrimidin-4-yloxy)-isoquinoline-4-carboxylic acid (4-fluoro-3-trifluoromethyl-phenyl)-amide

7-(6-Chloro-pyrimidin-4-yloxy)-isoquinoline-4-carboxylic acid (4-fluoro-3-trifluoromethyl-phenyl)-amide (490 mg, 1.0 Mmol) is treat with a solution of methylamine in EtOH (33% wt) at rt. The reaction mixture is stirred for 1.5 h at ambient temperture. It is then concentrated under reduced pressure and the remaining crude product submitted to flash chromatography (SiO₂, CH₂Cl₂/MeOH; gradient 1-8 % MeOH) to give the title compound as a yellow solid. MS: [M+1]⁺ = 458. Mp 255-257 °C.

The starting material is prepared as follows:

### Step 95.1: 7-(6-Chloro-pyrimidin-4-yloxy)-isoquinoline-4-carboxylic acid (4-fluoro-3-trifluoromethyl-phenyl)-amide

The title compound is prepared in analogy to Step 93.4. from 7-(6-Chloro-pyrimidine-4-yloxy)-isoquinoline-4-carboxyl choride and 4-fluoro-3-triflourometyl-phenylamine.

### Example 96:7-(6-Methylamino-pyrimidin-4-yloxy)-isoquinoline-4-carboxylic acid (3-trifluoromethyl-phenyl)-amide

The title compound is prepared in analogy to Ex. 95 from 3-trifluoromethyl-phenylamine.
MS: [M+1]⁺= 440. Mp 205-208°C.

### Example 97: The following compounds can be obtained in analogy to Example 93 (Q = H) or Example 95 (R₄ = CH₃) starting from 6-(6-chloropyrimidin-4-yloxy)-naphthalene-1-carbonyl chloride (step 81.1) and appropriate 2-amino pyrazoles (described for example in GB 0500435.3).

| | MS [M+1]⁺ | Mp [°C] | Name |
|---|---|---|---|
| a) | 550 | 130-135 | 6-(6-Methylamino-pyrimidin-4-yloxy)-naphthalene-1-carboxylic acid 5-tert-butyl-2-(4-dimethylaminomethyl-phenyl)-2H-pyrazole-3-yl-amide |
| b) | 605 | | 6-(6-Methylamino-pyrimidin-4-yloxy)-naphthalene-1-carboxylic acid {5-tert-butyl-2-4-(4-methyl-piperazin-1-ylmethyl)-phenyl)-2H-pyrazol-3-yl}-amide |
| c) | 578 | | 6-(6-Amino-pyrimidin-4-yloxy)-naphthalene-1-carboxylic acid [5-tert-butyl-2-4-(4-morpholin-4-ylmethyl)-phenyl)-2H-pyrazol-3-yl]-amide |
| d) | 592 | 125-128 | 6-(6-Methylamino-pyrimidin-4-yloxy)-naphthalene-1-carboxylic acid [5-tert-butyl-2-4-(4-morpholin-4-ylmethyl)-phenyl)-2H-pyrazol-3-yl]-amide |
| e) | 507 | 105-110 | 6-(6-Methylamino-pyrimidin-4-yloxy)-naphthalene-1-carboxylic acid [5-tert-butyl-2-4-(3-methyl-phenyl)-2H-pyrazol-3-yl]-amide |
| f) | 592 | 105-110 | 6-(6-Methylamino-pyrimidin-4-yloxy)-naphthalene-1-carboxylic acid [5-tert-butyl-2-4-(4-morpholin-3-ylmethyl)-phenyl)-2H-pyrazol-3-yl]-amide |
| g) | 536 | 140-145 | 6-(6-Amino-pyrimidin-4-yloxy)-naphthalene-1-carboxylic acid [5-tert-butyl-2-(3-dimethylaminometylphenyl)-2H-pyrazol-3-yl]-amide |
| h) | 550 | 114-116 | 6-(6-Methylamino-pyrimidin-4-yloxy)-naphthalene-1- |
| | | | carboxylic acid [5-tert-butyl-2-(3-dimethylaminometylphenyl)-2H-pyrazol-3-yl]-amide |
| i) | 550 | 290-291 | 6-(6-Amino-pyrimidin-4-yloxy)-naphthalene-1-carboxylic acid [5-tert-butyl-2-(3-dimethylcarbamoyl-phenyl)-2H-pyrazol-3-yl]-amide |
| j) | 564 | 247-249 | 6-(6-Methylamino-pyrimidin-4-yloxy)-naphthalene-1-carboxylic acid [5-tert-butyl-2-(3-dimethylcarbamoyl-phenyl)-2H-pyrazol-3-yl]-amide |

### Reference Example 98: Dry-filled capsules

5000 capsules, each comprising as active ingredient 0.25 g of one of the compounds of formula I mentioned in the preceding Examples, are prepared as follows:

### Composition

| | |
|---|---|
| active ingredient | 1250 g |
| talcum | 180 g |
| wheat starch | 120 g |
| magnesium stearate | 80 g |
| lactose | 20 g |

Preparation process: The mentioned substances are pulverised and forced through a sieve of 0.6 mm mesh size. 0.33 g portions of the mixture are introduced into gelatin capsules using a capsule-filling machine.

### Reference Example 99: Soft capsules

5000 soft gelatin capsules, each comprising as active ingredient 0.05 g of one of the compounds of formula I mentioned in the preceding Examples, are prepared as follows:

### Composition

| | |
|---|---|
| active ingredient | 250 g |
| PEG 400 | 1 litre |
| Tween 80 | 1 litre |

Preparation process: The active ingredient is pulverised and suspended in PEG 400 (polyethylene glycol having an Mᵣ of from approx. 380 to approx. 420, Fluka, Switzerland) and Tween^{®}80 (polyoxyethylene sorbitan monolaurate, Atlas Chem. Ind. Inc., USA, supplied by Fluka, Switzerland) and ground in a wet pulveriser to a particle size of approx. from 1 to 3 µm. 0.43 g portions of the mixture are then introduced into soft gelatin capsules using a capsule-filling machine.

### Example 100: Inhibition of the tyrosine kinase activity of VEGF-R2 (KDR)

The inhibition test is carried out as described above. The IC₅₀ values for some of the compounds of formula I are given below:

| **Compound from Example No.** | **KDR IC₅₀ [µM]** |
|---|---|
| 8 | 0.046 |
| 9 | 0.029 |
| 10 | 0.037 |
| 12a) | 0.052 |
| 12b) | 0.010 |
| 12d) | 0.073 |
| 12f) | 0.017 |
| 15 | 0.020 |
| 17a) | 0.019 |
| 17b) | 0.054 |
| 17c) | 0.018 |
| 18 | 0.021 |
| 20 | 0.024 |
| 21 | 0.019 |
| 22 | 0.096 |
| 25 | 0.036 |
| 26a) | 0.021 |

### Example 101: Penetration / Permeation Studies

### a) Skin donors and preparation

Frozen human dorsal cadaver skin (Caucasian; back skin) was obtained from the National Disease Research Interchange, Philadelphia, USA. Skin from three different donors was used;

Thawed skin samples were dermatomed to 0.7 mm with an Aesculap dermatome to obtain "split-thickness" skin.

### b) Penetration assay

Percutaneous penetration was studied *in vitro* by means of static Franz-type diffusion cells with phosphate buffered saline/ fetal calf serum 2:1 as receptor fluid at 32° in triplicates for 48 hours. The compounds were applied as 1 % solutions (300 µl each) in propylene glycol (solution a) or in propylene glycol / oleyl alcohol 9:1 (solution b). Samples of 100 µl were taken 5 to 7 times and replaced by fresh receptor fluid as described previously (Schmook, et al., Skin Pharmacol. 1993, 6:116-124).

### c) Sample processing

Drug analysis was performed with specimens of the exposed skin (at the end of the 48 hours experiment), from which the stratum corneum had been removed by 20 strippings with an adhesive tape. The weighed skin samples were homogenized in 0.2 M ammonium phosphate buffer (pH 7.0). The homogenates were extracted with ethyl acetate and processed as described (Schmook, et al 1993).

Skin concentrations as a measurement for skin penetration for different compounds as given in the examples were found to be enhanced by a factor 5 to 500, e.g. 10 to 100, when given as solutions b as compared to those values when given as solutions a.

Some specific values for the compounds given and applied as solutions b are given in TABLE 1 and in TABLE 2 and applied as solutions a, c and d are given in TABLE 3. When these compounds were applied as solutions a) at least 10-fold lower skin concentrations and quasi undetectable (negligible) skin permeation was found.

**TABLE 1:**

| Compound | Batch of skin | Skin concentration [µg/g] | Permeation rate [ng/ml/hr] |
|---|---|---|---|
| 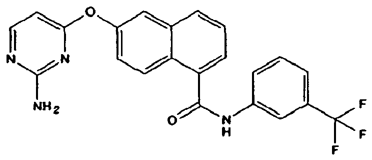 | 2 | 44 ± 17 | 13 ± 3 |
| | 3 | 24 ± 6 | 8 ± 2 |
| 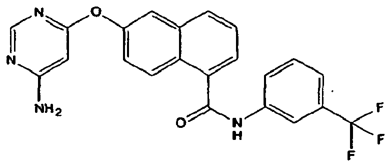 | 3 | 34 ± 3 | 12 ± 4 |
| 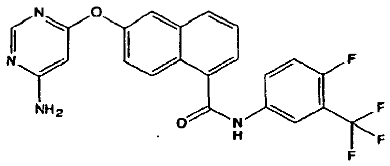 | 3 | 19 ± 7 | 22 ± 3 |
| 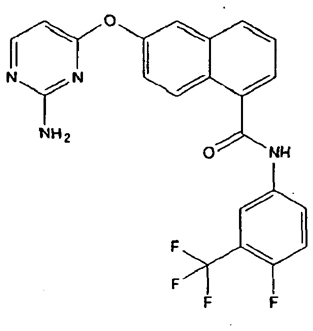 | 3 | 46 ± 13 | 19 ± 3 |

**TABLE 2:**

| Compound | Batch of skin | Skin concentration [µg/g] | Permeation rate [ng/ml/hr] |
|---|---|---|---|
| 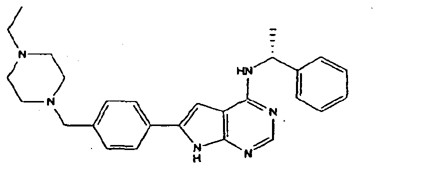 | 1 | 35 ± 12 | 182 ± 68 |
| | 2 | 72 ± 24 | 189 ± 43 |
| 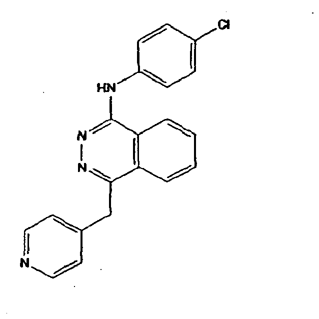 | 1 | 10.4 ± 2.0 | 286 ± 90 |

Table 3 shows corresponding specific using the skin model as described before of a compound of formula Iₚ

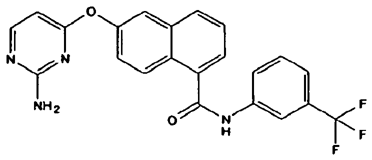

When applied as a solution in propylene glycol (= solution a) or in propylene glycol containing 0.1% DMSO (= solution c), or as a solution of propylene glycol containing 1 % DMSO (= solution d) equally low skin concentrations and quasi undetectable (negligible) permeation were found.

**TABLE 3:**

| Formulation of compound of formula Iₚ as | Batch of skin | Skin concentration [µg/g] | Permeation rate [ng/ml/hr] |
|---|---|---|---|
| solution a | 4 | 6.2 ± 1.5 | <2 |
| solution c | 4 | 4.4 ± 3.1 | <2 |
| solution d | 4 | 4.7 ± 2.9 | <2 |

## Claims

1. A composition for topical administration comprising a VEGF receptor inhibitor and a penetration enhancer, wherein the VEGF receptor inhibitor is a compound of formula 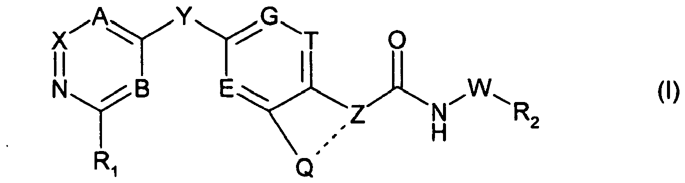 wherein
R, is H; halo; -(C₀₋₇)-R₃; -(C₀₋₇)-NR₄R₅; or -C(=O)-R₆;
R₂ is substituted (C₃₋₈)cycloalkyl; substituted aryl; or substituted heterocyclyl;
R₃ is H or unsubstituted or substituted (C₁₋₄)alkyl;
R₄ and R₅ are independently selected from the group consisting of H; unsubstituted or substituted (C₁₋₄)alkyl; (C₁₋₄)alkyl-carbonyl, wherein the (C₁₋₄)alkyl moiety is optionally substituted; and (C₁₋₄)alkoxy-carbonyl, wherein the (C₁₋₄)alkyl moiety is optionally substituted;
R₆ is H; unsubstituted or substituted (C₁₋₄)alkyl; (C₁₋₄)alkoxy, wherein the (C₁₋₄)alkyl moiety is optionally substituted; or unsubstituted, mono- or di-substituted amino;
A, B and X are independently selected from =C(R₇)- or N;
E, G and T are independently selected from =C(R₈)- or N;
R₇ and R₈ are independently selected from the group consisting of H, halo and unsubstituted or substituted (C₁₋₄)alkyl;
Y is -O-, -S-, -S(O)-, -S(O)₂-, -CH₂- or -CH₂-CH₂-;
Z is CH or N and Q is (C₁₋₄)alkylene or (C₂₋₄)alkenylene, wherein (C₁₋₄)alkylene or (C₂₋₄)alkenylene optionally may be substituted and wherein one or more of the carbon atoms of said (C₁₋₄)alkylene or (C₂₋₄)alkenylene chain optionally may be replaced by a heteroatom independently selected from nitrogen, oxygen and sulfur; and the bond between Q and Z **characterized by** a dotted line is a single bond; with the proviso that if Z is N, Q is not unsubstituted unbranched (C₁₋₄)alkylene; or
Z is C and Q is as defined above wherein the bond between Q and Z **characterized by** a dotted line is a double bond; and
W is either not present or (C₁₋₃)alkylene;
or a tautomer thereof, or in the form of a salt, a solvate or in the form of a salt and a solvate, and wherein the penetration enhancer is selected from oleyl alcohol.

2. A composition of claim 1, wherein the VEGF receptor inhibitor is a compound of formula (I),
wherein
R₁ is halo; -(C₀₋₇)-NR₄R₅; or -C(=O)-R₆;
R₂ is substituted (C₃₋₈)cycloalkyl; substituted aryl; or substituted heterocyclyl;
R₄ and R₅ are independently selected from the group consisting of H; (C₁₋₄)alkyl; (C₁₋₄)alkyl - carbonyl; and (C₁₋₄)alkoxy-carbonyl;
R₆ is (C₁₋₄)alkyl, (C₁₋₄)alkoxy, (C₁₋₄)alkyl-amino, di-(C₁₋₄)alkyl-amino-(C₁₋₄)alkyl-amino or di-(C₁₋₄)alkyl-amino;
A, B and X are independently selected from =C(R₇)- or N;
E, G and T are =C(R₈)-;
R₇ and R₈ are independently selected from H and halo;
Y is -O-, -S- or -CH₂-, especially -O-;
Z is N and Q is (C₁₋₄)alkylene or (C₂-₄)alkenylene, wherein one or more, especially one, of the carbon atoms of said (C₁₋₄)alkylene or (C₂₋₄)alkenylene chain optionally may be replaced by a heteroatom independently selected from N, O and S, especially from O, the N optionally substituted by (C₁₋₄)alkyl; and the bond between Q and Z **characterized by** a dotted line in formula I is a single bond; with the proviso that Q is not unsubstituted unbranched (C₁₋₄)alkylene;
or
Z is C and Q is as defined above wherein the bond between Q and Z **characterized by** a dotted line in formula I is a double bond; and
W is (C₁₋₃)alkylene or especially not present.

3. A composition of claims 1 or 2, wherein the VEGF receptor inhibitor is a compound of formula (I) wherein
Z is N and Q is (C₂₋₄)alkenylene, or (C₁₋₄)alkylene wherein one or more, especially one, of the carbon atoms of (C₁₋₄)alkylene is replaced by a heteroatom independently selected from N, O and S, especially from O; and the bond between Q and Z **characterized by** a dotted line in formula I is a single bond; or
Z is C and Q is as defined above wherein the bond between Q and Z **characterized by** a dotted line in formula I is a double bond.

4. A composition of any one of claims 1 to 3, wherein the VEGF receptor inhibitor is a compound of formula (I), wherein
A is N and B is =CH or B is N and A is =CH,
X is =CH,
Y is O,
E, G and T are =CH,
Z is C,
Q is -CH=CH-CH=,
W is not present,
R₁ is -(C₀₋₇)-NR₄R₅ and R₄ and R₅ are as defined above,
R₂ is substituted aryl.

5. A composition of any one of claims 1 to 4, wherein the VEGF receptor inhibitor is a compound of formula 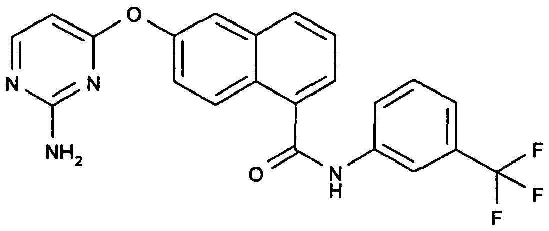 or a pharmaceutically acceptable salt thereof.

6. A composition of any one of claims 1 to 5 in the form of a cream.

7. A composition of any one of claims 1 to 5 for use as a topical pharmaceutical.

8. A composition of any one of claims 1 to 5 for use as a medicament.

9. A composition of any one of claims 1 to 5 for use in the method of treatment of a dermatological disease, selected from the group consisting of psoriasis, atopic dermatitis and acne.

10. A pharmaceutical composition comprising a composition of any one of claims 1 to 5 in association with at least one pharmaceutical excipient.

11. A pharmaceutical composition of claim 10, further comprising another pharmaceutically active agent.

12. A kit of parts for topical administration comprising
a) a VEGF receptor inhibitor of any one of claims 1 to 5, and
b) a penetration enhancer as described in claim 1.

## Patentansprüche

1. Zusammensetzung zur topischen Verabreichung, umfassend einen VEGF-Rezeptor-Inhibitor und einen Penetrationsverbesserer, wobei es sich bei dem VEGF-Rezeptor-Inhibitor um eine Verbindung der Formel 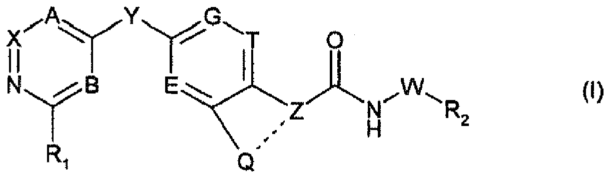 handelt, wobei
R₁ für H; Halogen; -(C₀₋₇)-R₃; -(C₀₋₇)-NR₄R₅ oder -C(=O)-R₆ steht;
R₂ für substituiertes (C₃₋₈)-Cycloalkyl; substituiertes Aryl oder substituiertes Heterocyclyl steht;
R₃ für H oder unsubstituiertes oder substituiertes (C₁₋₄) -Alkyl steht;
R₄ und R₅ unabhängig voneinander aus der aus H; unsubstituiertem oder substituiertem (C₁₋₄)-Alkyl; (C₁₋₄)-Alkyl-carbonyl, wobei die (C₁₋₄)-Alkylgruppe gegebenenfalls substituiert ist, und (C₁₋₄)-Alkoxycarbonyl, wobei die (C₁₋₄)-Alkylgruppe gegebenenfalls substituiert ist, bestehenden Gruppe ausgewählt sind;
R₆ für H; unsubstituiertes oder substituiertes (C₁₋₄) -Alkyl; (C₁₋₄)-Alkoxy, wobei die (C₁₋₄) - Alkylgruppe gegebenenfalls substituiert ist, oder unsubstituiertes, mono- oder disubstituiertes Amino steht;
A, B und X unabhängig voneinander aus =C(R₇)- oder N ausgewählt sind;
E, G und T unabhängig voneinander aus =C(R₈)- oder N ausgewählt sind;
R₇ und R₈ unabhängig voneinander aus der aus H, Halogen und unsubstituiertem oder substituiertem (C₁₋₄)-Alkyl bestehenden Gruppe ausgewählt sind;
Y für -O-, -S-, -S(O)-, -S(O)₂-, -CH₂- oder - CH₂-CH₂- steht;
Z für CH oder N steht und Q für (C₁₋₄)-Alkylen oder (C₂₋₄)-Alkenylen steht, wobei (C₁₋₄)-Alkylen oder (C₂₋₄)-Alkenylen gegebenenfalls substituiert sein kann und wobei eines oder mehrere der Kohlenstoffatome dieser (C₁₋₄)-Alkylen- oder (C₂₋₄)-Alkenylenkette gegebenenfalls durch ein unabhängig aus Stickstoff, Sauerstoff und Schwefel ausgewähltes Heteroatom ersetzt sein können; und die durch eine gestrichelte Linie gekennzeichnete Bindung zwischen Q und Z für eine Einfachbindung steht; mit der Maßgabe, dass, wenn Z für N steht, Q nicht für unsubstituiertes, unverzweigtes (C₁₋₄) - Alkylen steht; oder
Z für C steht und Q wie oben definiert ist, wobei die durch eine gestrichelte Linie gekennzeichnete Bindung zwischen Q und Z für eine Doppelbindung steht; und
W entweder fehlt oder für (C₁₋₃)-Alkylen steht;
oder ein Tautomer davon, oder in Form eines Salzes, eines Solvates oder in der Form eines Salzes und eines Solvates, und wobei der Penetrationsverbesserer aus Oleylalkohol ausgewählt ist.

2. Zusammensetzung nach Anspruch 1, wobei es sich bei dem VEGF-Rezeptor-Inhibitor um eine Verbindung der Formel (I) handelt, wobei
R₁ für Halogen; - (C₀₋₇) -NR₄R₅ oder -C(=O)-R₆ steht; R₂ für substituiertes (C₃₋₈)-Cycloalkyl; substituiertes Aryl oder substituiertes Heterocyclyl steht;
R₄ und R₅ unabhängig voneinander aus der aus H; (C₁₋₄) -Alkyl; (C₁₋₄)-Alkyl-carbonyl und (C₁₋₄)-Alkoxycarbonyl bestehenden Gruppe ausgewählt sind;
R₆ für (C₁₋₄)-Alkyl, (C₁₋₄)-Alkoxy, (C₁₋₄) -Alkyl-amino, Di-(C₁₋₄)-alkylamino-(C₁₋₄)-alkylamino oder Di-(C₁₋₄)-alkylamino steht;
A, B und X unabhängig voneinander aus =C(R₇)- oder N ausgewählt sind;
E, G und T für =C(R₈)- stehen;
R₇ und R₈ unabhängig voneinander aus H und Halogen ausgewählt sind;
Y für -O-, -S- oder -CH₂-, insbesondere -O-, steht;
Z für N steht und Q für (C₁₋₄)-Alkylen oder (C₂₋₄)-Alkenylen steht, wobei eines oder mehrere, insbesondere eines, der Kohlenstoffatome dieser (C₁₋₄) -Alkylen- oder (C₂₋₄)-Alkenylenkette gegebenenfalls durch ein aus N, 0 und S, insbesondere aus 0, ausgewähltes Heteroatom ersetzt sein können, wobei das N gegebenenfalls durch (C₁₋₄)-Alkyl substituiert ist; und die durch eine gestrichelte Linie in Formel I gekennzeichnete Bindung zwischen Q und Z für eine Einfachbindung steht; mit der Maßgabe, dass Q nicht für unsubstituiertes, unverzweigtes (C₁₋₄)-Alkylen steht;
oder
Z für C steht und Q wie oben definiert ist, wobei die durch eine gestrichelte Linie in Formel I gekennzeichnete Bindung zwischen Q und Z für eine Doppelbindung steht; und
W für (C₁₋₃)-Alkylen steht oder insbesondere fehlt.

3. Verbindung nach Anspruch 1 oder 2, wobei es sich bei dem VEGF-Rezeptor-Inhibitor um eine Verbindung der Formel (I) handelt, wobei
Z für N steht und Q für (C₂₋₄)-Alkenylen oder (C₁₋₄)-Alkylen steht, wobei eines oder mehrere, insbesondere eines, der Kohlenstoffatome von (C₁₋₄)-Alkylen durch ein unabhängig aus N, 0 und S, insbesondere aus 0, ausgewähltes Heteroatom ersetzt sind; und die durch eine gestrichelte Linie in Formel I gekennzeichnete Bindung zwischen Q und Z für eine Einfachbindung steht; oder
Z für C steht und Q wie oben definiert ist, wobei die durch eine gestrichelte Linie in Formel I gekennzeichnete Bindung zwischen Q und Z für eine Doppelbindung steht.

4. Zusammensetzung nach einem der Ansprüche 1 bis 3, wobei es sich bei dem VEGF-Rezeptor-Inhibitor um eine Verbindung der Formel (I) handelt, wobei
A für N steht und B für =CH steht oder B für N steht und A für =CH steht,
X für =CH steht,
Y für 0 steht,
E, G und T für =CH stehen,
Z für C steht,
Q für -CH=CH-CH= steht,
W fehlt,
R₁ für - (C₀₋₇)-NR₄R₅ steht und R₄ und R₅ wie oben definiert sind,
R₂ für substituiertes Aryl steht.

5. Zusammensetzung nach einem der Ansprüche 1 bis 4, wobei es sich bei dem VEGF-Rezeptor-Inhibitor um eine Verbindung der Formel 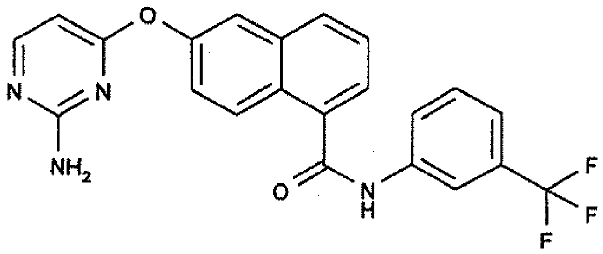 oder ein pharmazeutisch unbedenkliches Salz davon handelt.

6. Zusammensetzung nach einem der Ansprüche 1 bis 5 in Form einer Creme.

7. Zusammensetzung nach einem der Ansprüche 1 bis 5 zur Verwendung als topisches Pharmazeutikum.

8. Zusammensetzung nach einem der Ansprüche 1 bis 5 zur Verwendung als Medikament.

9. Zusammensetzung nach einem der Ansprüche 1 bis 5 zur Verwendung bei einem Verfahren zur Behandlung einer dermatologischen Krankheit ausgewählt aus der Gruppe aus Psoriasis, atopischer Dermatitis und Akne.

10. Pharmazeutische Zusammensetzung, umfassend eine Zusammensetzung nach einem der Ansprüche 1 bis 5 zusammen mit wenigstens einem pharmazeutischen Exzipienten.

11. Pharmazeutische Zusammensetzung nach Anspruch 10, weiterhin umfassend einen anderen pharmazeutischen Wirkstoff.

12. Kit von Teilen für die topische Verabreichung, umfassend
a) einen VEGF-Rezeptor-Inhibitor nach einem der Ansprüche 1 bis 5 und
b) einen wie in Anspruch 1 beschriebenen Penetrationsverbesserer.

## Revendications

1. Composition destinée à l'administration par voie topique comprenant un inhibiteur des récepteurs VEGF et un agent améliorant la pénétration, **caractérisée en ce que** l'inhibiteur des récepteurs VEGF est un composé de formule 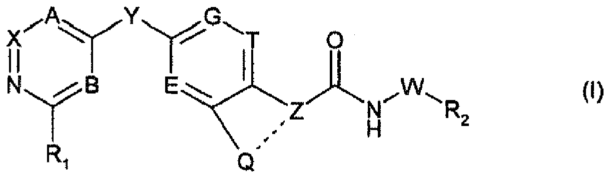 dans laquelle
R₁ est H ; halogène ; -(C₀₋₇)-R₃ ; -(C₀₋₇)-NR⁴R⁵ ; ou -C(=O)-R₆ ;
R₂ est (C₃₋₈)cycloalkyle substitué ; aryle substitué ; ou hétérocyclyle substitué ;
R₃ est H ou (C₁₋₄) alkyle non substitué ou substitué ;
R₄ et R₅ sont choisis indépendamment dans le groupe constitué par H ; (C₁₋₄)alkyle non substitué ou substitué ; (C₁₋₄) alkyl-carbonyle, où le motif (C₁₋₄)alkyle est éventuellement substitué ; et (C_{1- 4}) alcoxy-carbonyle, où le motif (C₁₋₄) alkyle est éventuellement substitué ;
R₆ est H ; (C₁₋₄) alkyle non substitué ou substitué ; (C₁₋₄) alcoxy, où le motif (C₁₋₄) alkyle est éventuellement substitué ; ou amino non substitué, mono- ou di-substitué ;
A, B et X sont choisis indépendamment parmi =C(R₇)-ou N ;
E, G et T sont choisis indépendamment parmi =C(R₈)-ou N ;
R₇ et R₈ sont choisis indépendamment dans le groupe constitué par H, halogéno et (C₁₋₄)alkyle non substitué ou substitué ;
Y est -O-, -S-, -S(O) -, -S(O)₂-, -CH₂- ou -CH₂-CH₂-;
Z est CH ou N et Q est (C₁₋₄) alkylène ou (C₂₋₄) alcénylène, où (C₁₋₄) alkylène ou (C₂₋₄)alcénylène peuvent être éventuellement substitués et où un ou plusieurs des atomes de carbone de ladite chaîne (C₁₋₄)alkylène ou (C₂₋₄)alcénylène peuvent être éventuellement remplacés par un hétéroatome choisi indépendamment parmi azote, oxygène et soufre ; et la liaison entre Q et Z **caractérisée par** une ligne en pointillé est une simple liaison ; à condition que si Z est N, Q ne soit pas (C₁₋₄)alkylène non substitué non ramifié ; ou
Z est C et Q est tel que défini ci-dessus où la liaison entre Q et Z **caractérisée par** une ligne en pointillé est une double liaison ; et
W soit n'est pas présent soit est (C₁₋₃)alkylène ;
ou un tautomère de celui-ci, ou sous forme d'un sel, d'un solvate ou sous forme d'un sel et d'un solvate et où l'agent améliorant la pénétration est choisi parmi l'alcool oléique.

2. Composition selon la revendication 1, **caractérisée en ce que** l'inhibiteur des récepteurs VEGF est un composé de formule (I), dans laquelle
R₁ est halogéno ; -(C₀₋₇)-NR₄R⁵ ; 1 ou -C(=O)-R₆ ;
R₂ est (C₃₋₈)cycloalkyle substitué ; aryle substitué ; ou hétérocyclyle substitué ;
R₄ et R₅ sont choisis indépendamment dans le groupe constitué par H ; (C₁₋₄)alkyle ; (C₁₋₄)alkyl-carbonyle ; et (C₁₋₄)alcoxy-carbonyle ;
R₆ est (C₁₋₄)alkyle, (C₁₋₄)alcoxy, (C₁₋₄)alkyl-amino, di-(C₁₋₄)alkyl-amino-(C₁₋₄)alkyl-amino ou di-(C₁₋₄)alkyl-amino ;
A, B et X sont choisis indépendamment parmi =C(R₇)-ou N ;
E, G et T sont =C(R₈)- ;
R₇ et R₈ sont choisis indépendamment parmi H et halogéno ;
Y est -O-, -S- ou -CH₂-, notamment -O- ;
Z est N et Q est (C₁₋₄)alkylène ou (C₂₋₄)alcénylène, où un ou plusieurs, notamment l'un, des atomes de carbone de ladite chaîne (C₁₋₄)alkylène ou (C₂₋₄)alcénylène peuvent éventuellement être remplacés par un hétéroatome choisi indépendamment parmi N, O et S, notamment parmi 0, le N étant éventuellement substitué par (C₁₋₄)alkyle ; et la liaison entre Q et Z **caractérisée par** une ligne en pointillé dans la formule I est une simple liaison ; à condition que Q ne soit pas (C₁₋₄)alkylène non substitué non ramifié ; ou
Z est C et Q est tel que défini ci-dessus où la liaison entre Q et Z **caractérisée par** une ligne en pointillé dans la formule I est une double liaison ; et
W est (C₁₋₃)alkylène ou notamment n'est pas présent.

3. Composition selon la revendication 1 ou 2, **caractérisée en ce que** l'inhibiteur des récepteurs VEGF est un composé de formule (I), dans laquelle
Z est N et Q est (C₂₋₄)alcénylène, ou (C₁₋₄)alkylène où un ou plusieurs, notamment l'un, des atomes de carbone de (C₁₋₄)alkylène sont remplacés par un hétéroatome choisi indépendamment parmi N, 0 et S, notamment parmi 0 ; et la liaison entre Q et Z **caractérisée par** une ligne en pointillé dans la formule I est une simple liaison ; ou
Z est C et Q est tel que défini ci-dessus où la liaison entre Q et Z **caractérisée par** une ligne en pointillé dans la formule I est une double liaison.

4. Composition selon l'une quelconque des revendications 1 à 3, **caractérisée en ce que** l'inhibiteur des récepteurs VEGF est un composé de formule (I), dans laquelle
A est N et B est =CH ou B est N et A est =CH,
X est =CH,
Y est 0,
E, G et T sont =CH,
Z est C,
Q est -CH=CH-CH=,
W n'est pas présent,
R₁ est - (C₀₋₇)-NR₄R₅ et R₄ et R₅ sont tels que définis ci-dessus,
R₂ est aryle substitué.

5. Composition selon l'une quelconque des revendications 1 à 4, **caractérisée en ce que** l'inhibiteur des récepteurs VEGF est un composé de formule 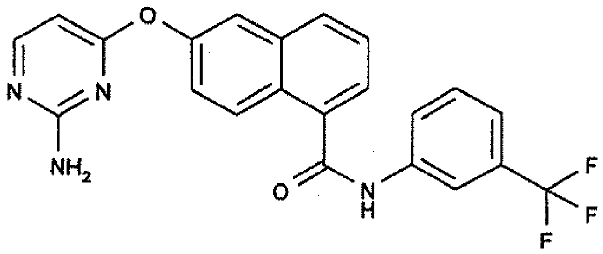 ou un sel pharmaceutiquement acceptable de celui-ci.

6. Composition selon l'une quelconque des revendications 1 à 5, sous forme de crème.

7. Composition selon l'une quelconque des revendications 1 à 5, destinée à être utilisée comme produit pharmaceutique à appliquer par voie topique.

8. Composition selon l'une quelconque des revendications 1 à 5, destinée à être utilisée comme médicament.

9. Composition selon l'une quelconque des revendications 1 à 5, destinée à être utilisée dans la méthode de traitement d'une maladie dermatologique, choisie dans le groupe constitué par le psoriasis, la dermatite atopique et l'acné.

10. Composition pharmaceutique comprenant une composition selon l'une quelconque des revendications 1 à 5, en association avec au moins un excipient pharmaceutique.

11. Composition pharmaceutique selon la revendication 10, comprenant en outre un autre agent pharmaceutiquement actif.

12. Kit de composants destinés à l'administration par voie topique, comprenant
a) un inhibiteur des récepteurs VEGF selon l'une quelconque des revendications 1 à 5, et
b) un agent améliorant la pénétration tel que décrit dans la revendication 1.
